# EUROPEAN PATENT APPLICATION

(11) **EP 2 767 594 A1**
(43) Date of publication of application: **20.08.2014**
(21) Application number: 13155742.3
(22) Date of filing: 19.02.2013
(51) Int. Cl.: C12Q 1/68

(54) **The signature of Wnt/ß-catenin signaling in Cancer**

(71) Applicant: Deutsches Krebsforschungszentrum, 69120 Heidelberg (DE); Ruprecht-Karls-Universität Heidelberg, 69117 Heidelberg (DE)
(72) Inventor: Erdmann, Gerrit, 69115 Heidelberg (DE); Boutros, Michael, 69117 Heidelberg (DE)
(74) Representative: Dick, Alexander

(57) **Abstract**

The present invention is concerned with compounds modulating Wnt/β-catenin signaling in cancer cells. Furthermore, contemplated by the present invention are therapeutic and diagnostic methods and means for identifying such a compound, as well as the use of said compound for the manufacture of a drug against cancer. The present invention also encompasses therapeutic and diagnostic methods and means for diagnosing cancer in the subject and for determining whether a subject is in need of an anti-cancer therapy, as well as uses thereof. It is also referred to a device and kit for carrying out the methods of the present invention.

SEQUENCE LISTING

<110>  Deutsches Krebsforschungszentrum
       Ruprecht-Karls-Universität Heidelberg

<120>  The signature of Wnt/ß-catenin signaling in Cancer

<130>  DK10065EP

<160>  24

<170>  PatentIn version 3.5

<210>  1
<211>  1413
<212>  DNA
<213>  Homo sapiens

<220>
<221>  CDS
<222>  (1)..(1413)

<400>  1
atg ggg aaa ctt cac tcc aag ccg gcc gcc gtg tgc aag cgc agg gag      48
Met Gly Lys Leu His Ser Lys Pro Ala Ala Val Cys Lys Arg Arg Glu
1               5                   10                  15

agc ccg gaa ggt gac agc ttc gcc gtg agc gct gcc tgg gct cgg aag      96
Ser Pro Glu Gly Asp Ser Phe Ala Val Ser Ala Ala Trp Ala Arg Lys
            20                  25                  30

ggc atc gag gag tgg atc ggg aga cag cgc tgc ccg ggc ggt gtc tcg      144
Gly Ile Glu Glu Trp Ile Gly Arg Gln Arg Cys Pro Gly Gly Val Ser
        35                  40                  45

gga ccc cga cag ctg cgg ttg gcg ggc acc ata ggc cga agc acc cgg      192
Gly Pro Arg Gln Leu Arg Leu Ala Gly Thr Ile Gly Arg Ser Thr Arg
    50                  55                  60

gag ctc gtg ggc gac gtg ttg aga gac acg ctc agc gag gaa gag gag      240
Glu Leu Val Gly Asp Val Leu Arg Asp Thr Leu Ser Glu Glu Glu Glu
65                  70                  75                  80

gac gac ttt cgg ctg gaa gtg gcc ctg cct cct gag aag act gac ggg      288
Asp Asp Phe Arg Leu Glu Val Ala Leu Pro Pro Glu Lys Thr Asp Gly
                85                  90                  95

ctg ggc agc gga gat gag aag aag atg gag aga gtg agc gaa ccc tgc      336
Leu Gly Ser Gly Asp Glu Lys Lys Met Glu Arg Val Ser Glu Pro Cys
            100                 105                 110

cca ggc tcc aag aag cag ctg aag ttt gaa gag ctc cag tgc gac gtg      384
Pro Gly Ser Lys Lys Gln Leu Lys Phe Glu Glu Leu Gln Cys Asp Val
        115                 120                 125

tcc atg gag gag gac agc cgg cag gag tgg acc ttc acc ctg tat gac      432
Ser Met Glu Glu Asp Ser Arg Gln Glu Trp Thr Phe Thr Leu Tyr Asp
    130                 135                 140

ttt gac aac aac ggc aag gtc acc cga gag gac atc acc agc ttg ctg      480
Phe Asp Asn Asn Gly Lys Val Thr Arg Glu Asp Ile Thr Ser Leu Leu
145                 150                 155                 160

cac acc atc tat gag gtg gtg gac tcc tct gtc aac cac tcc cca aca      528

His Thr Ile Tyr Glu Val Val Asp Ser Ser Val Asn His Ser Pro Thr
                165                 170                 175

tcc agc aag atg ctg cgg gta aag ctc acc gtg gcc ccc gat ggc agc      576
Ser Ser Lys Met Leu Arg Val Lys Leu Thr Val Ala Pro Asp Gly Ser
            180                 185                 190

cag agc aag agg agc gtc ctt gtc aat cag gct gac ctg cag agc gca      624
Gln Ser Lys Arg Ser Val Leu Val Asn Gln Ala Asp Leu Gln Ser Ala
        195                 200                 205

agg ccc cga gca gag acc aag ccc act gag gac ctg cgg agc tgg gag      672
Arg Pro Arg Ala Glu Thr Lys Pro Thr Glu Asp Leu Arg Ser Trp Glu
    210                 215                 220

aag aag cag cga gcc ccg ctc agg ttc cag ggt gac agc cgc ctg gag      720
Lys Lys Gln Arg Ala Pro Leu Arg Phe Gln Gly Asp Ser Arg Leu Glu
225                 230                 235                 240

cag tct ggc tgc tac cac cat tgc gta gat gag aac atc gag agg aga      768
Gln Ser Gly Cys Tyr His His Cys Val Asp Glu Asn Ile Glu Arg Arg
                245                 250                 255

aac cac tac tta gat ctc gcc ggg ata gaa aac tac acg tcc caa ttt      816
Asn His Tyr Leu Asp Leu Ala Gly Ile Glu Asn Tyr Thr Ser Gln Phe
            260                 265                 270

ggg cct ggc tcc cct tcc gtg gcc cag aag tca gaa ctg ccc ccc cgc      864
Gly Pro Gly Ser Pro Ser Val Ala Gln Lys Ser Glu Leu Pro Pro Arg
        275                 280                 285

acc tcc aat ccc act cga tct cgc tcc cat gag ccg gaa gcc atc cac      912
Thr Ser Asn Pro Thr Arg Ser Arg Ser His Glu Pro Glu Ala Ile His
    290                 295                 300

atc cca cac cga aag ccc caa ggc gtg gac ccg gcc tcc ttc cac ttc      960
Ile Pro His Arg Lys Pro Gln Gly Val Asp Pro Ala Ser Phe His Phe
305                 310                 315                 320

ctt gac acc cca atc gcc aag gtc tca gag ctc cag caa cgg ctc cgg     1008
Leu Asp Thr Pro Ile Ala Lys Val Ser Glu Leu Gln Gln Arg Leu Arg
                325                 330                 335

ggc acc cag gac ggg agc aag cac ttt gtg agg tcc ccc aag gcc cag     1056
Gly Thr Gln Asp Gly Ser Lys His Phe Val Arg Ser Pro Lys Ala Gln
            340                 345                 350

ggc aag agt gtg ggt gtg ggc cac gtg gcc aga ggg gca aga aac aag     1104
Gly Lys Ser Val Gly Val Gly His Val Ala Arg Gly Ala Arg Asn Lys
        355                 360                 365

ccc cct ctg gga ccc gcc atc cct gcg gtg tcc ccc tcc gcc cac ctg     1152
Pro Pro Leu Gly Pro Ala Ile Pro Ala Val Ser Pro Ser Ala His Leu
    370                 375                 380

gct gcc agc ccg gcc ctc ctc ccc tcc cta gcc ccc ctc ggg cac aag     1200
Ala Ala Ser Pro Ala Leu Leu Pro Ser Leu Ala Pro Leu Gly His Lys
385                 390                 395                 400

aag cac aag cac cga gcc aag gag agc cag cag ggc tgc cgg ggc ctg     1248
Lys His Lys His Arg Ala Lys Glu Ser Gln Gln Gly Cys Arg Gly Leu
                405                 410                 415

cag gca cca ctg gcc tca ggt ggc cct gtc ctg ggg cgg gag cac ctg     1296
Gln Ala Pro Leu Ala Ser Gly Gly Pro Val Leu Gly Arg Glu His Leu

420                 425                 430

cgg gag ctg ccc gcc ttg gtg gtg tat gag agc cag gcc ggg cag ccg        1344
Arg Glu Leu Pro Ala Leu Val Val Tyr Glu Ser Gln Ala Gly Gln Pro
        435                 440                 445

gtc cag aga cat gag cac cac cac cac cat gaa cat cac cac cat tac        1392
Val Gln Arg His Glu His His His His His Glu His His His His Tyr
    450                 455                 460

cac cac ttc tac cag aca tag                                            1413
His His Phe Tyr Gln Thr
465                 470


<210>  2
<211>  470
<212>  PRT
<213>  Homo sapiens

<400>  2

Met Gly Lys Leu His Ser Lys Pro Ala Ala Val Cys Lys Arg Arg Glu
1               5                   10                  15

Ser Pro Glu Gly Asp Ser Phe Ala Val Ser Ala Ala Trp Ala Arg Lys
            20                  25                  30

Gly Ile Glu Glu Trp Ile Gly Arg Gln Arg Cys Pro Gly Gly Val Ser
        35                  40                  45

Gly Pro Arg Gln Leu Arg Leu Ala Gly Thr Ile Gly Arg Ser Thr Arg
    50                  55                  60

Glu Leu Val Gly Asp Val Leu Arg Asp Thr Leu Ser Glu Glu Glu Glu
65                  70                  75                  80

Asp Asp Phe Arg Leu Glu Val Ala Leu Pro Pro Glu Lys Thr Asp Gly
                85                  90                  95

Leu Gly Ser Gly Asp Glu Lys Lys Met Glu Arg Val Ser Glu Pro Cys
            100                 105                 110

Pro Gly Ser Lys Lys Gln Leu Lys Phe Glu Glu Leu Gln Cys Asp Val
        115                 120                 125

Ser Met Glu Glu Asp Ser Arg Gln Glu Trp Thr Phe Thr Leu Tyr Asp
    130                 135                 140

Phe Asp Asn Asn Gly Lys Val Thr Arg Glu Asp Ile Thr Ser Leu Leu
145                 150                 155                 160

His Thr Ile Tyr Glu Val Val Asp Ser Ser Val Asn His Ser Pro Thr
                165                 170                 175

Ser Ser Lys Met Leu Arg Val Lys Leu Thr Val Ala Pro Asp Gly Ser
        180                 185                 190

Gln Ser Lys Arg Ser Val Leu Val Asn Gln Ala Asp Leu Gln Ser Ala
    195                 200                 205

Arg Pro Arg Ala Glu Thr Lys Pro Thr Glu Asp Leu Arg Ser Trp Glu
    210                 215                 220

Lys Lys Gln Arg Ala Pro Leu Arg Phe Gln Gly Asp Ser Arg Leu Glu
225                 230                 235                 240

Gln Ser Gly Cys Tyr His His Cys Val Asp Glu Asn Ile Glu Arg Arg
                245                 250                 255

Asn His Tyr Leu Asp Leu Ala Gly Ile Glu Asn Tyr Thr Ser Gln Phe
            260                 265                 270

Gly Pro Gly Ser Pro Ser Val Ala Gln Lys Ser Glu Leu Pro Pro Arg
        275                 280                 285

Thr Ser Asn Pro Thr Arg Ser Arg Ser His Glu Pro Glu Ala Ile His
    290                 295                 300

Ile Pro His Arg Lys Pro Gln Gly Val Asp Pro Ala Ser Phe His Phe
305                 310                 315                 320

Leu Asp Thr Pro Ile Ala Lys Val Ser Glu Leu Gln Gln Arg Leu Arg
                325                 330                 335

Gly Thr Gln Asp Gly Ser Lys His Phe Val Arg Ser Pro Lys Ala Gln
            340                 345                 350

Gly Lys Ser Val Gly Val Gly His Val Ala Arg Gly Ala Arg Asn Lys
        355                 360                 365

Pro Pro Leu Gly Pro Ala Ile Pro Ala Val Ser Pro Ser Ala His Leu
    370                 375                 380

Ala Ala Ser Pro Ala Leu Leu Pro Ser Leu Ala Pro Leu Gly His Lys
385                 390                 395                 400

Lys His Lys His Arg Ala Lys Glu Ser Gln Gln Gly Cys Arg Gly Leu
                405                 410                 415

Gln Ala Pro Leu Ala Ser Gly Gly Pro Val Leu Gly Arg Glu His Leu
            420                 425                 430

Arg Glu Leu Pro Ala Leu Val Val Tyr Glu Ser Gln Ala Gly Gln Pro
        435                 440                 445

Val Gln Arg His Glu His His His His His Glu His His His His Tyr
    450                 455                 460

His His Phe Tyr Gln Thr
465                 470

<210>  3
<211>  936
<212>  DNA
<213>  Homo sapiens

<220>
<221>  CDS
<222>  (1)..(936)

<400>  3
atg ggc gtc ccc acg gcc ctg gag gcc ggc agc tgg cgc tgg gga tcc      48
Met Gly Val Pro Thr Ala Leu Glu Ala Gly Ser Trp Arg Trp Gly Ser
1               5                   10                  15

ctg ctc ttc gct ctc ttc ctg gct gcg tcc cta ggt ccg gtg gca gcc      96
Leu Leu Phe Ala Leu Phe Leu Ala Ala Ser Leu Gly Pro Val Ala Ala
            20                  25                  30

ttc aag gtc gcc acg ccg tat tcc ctg tat gtc tgt ccc gag ggg cag      144
Phe Lys Val Ala Thr Pro Tyr Ser Leu Tyr Val Cys Pro Glu Gly Gln
        35                  40                  45

aac gtc acc ctc acc tgc agg ctc ttg ggc cct gtg gac aaa ggg cac      192
Asn Val Thr Leu Thr Cys Arg Leu Leu Gly Pro Val Asp Lys Gly His
    50                  55                  60

gat gtg acc ttc tac aag acg tgg tac cgc agc tcg agg ggc gag gtg      240
Asp Val Thr Phe Tyr Lys Thr Trp Tyr Arg Ser Ser Arg Gly Glu Val
65                  70                  75                  80

cag acc tgc tca gag cgc cgg ccc atc cgc aac ctc acg ttc cag gac      288
Gln Thr Cys Ser Glu Arg Arg Pro Ile Arg Asn Leu Thr Phe Gln Asp
                85                  90                  95

ctt cac ctg cac cat gga ggc cac cag gct gcc aac acc agc cac gac      336
Leu His Leu His His Gly Gly His Gln Ala Ala Asn Thr Ser His Asp
            100                 105                 110

ctg gct cag cgc cac ggg ctg gag tcg gcc tcc gac cac cat ggc aac      384
Leu Ala Gln Arg His Gly Leu Glu Ser Ala Ser Asp His His Gly Asn
        115                 120                 125

ttc tcc atc acc atg cgc aac ctg acc ctg ctg gat agc ggc ctc tac      432
Phe Ser Ile Thr Met Arg Asn Leu Thr Leu Leu Asp Ser Gly Leu Tyr
    130                 135                 140

tgc tgc ctg gtg gtg gag atc agg cac cac cac tcg gag cac agg gtc      480
Cys Cys Leu Val Val Glu Ile Arg His His His Ser Glu His Arg Val
145                 150                 155                 160

cat ggt gcc atg gag ctg cag gtg cag aca ggc aaa gat gca cca tcc      528
His Gly Ala Met Glu Leu Gln Val Gln Thr Gly Lys Asp Ala Pro Ser

                165                 170                 175

aac tgt gtg gtg tac cca tcc tcc tcc cag gat agt gaa aac atc acg 576
Asn Cys Val Val Tyr Pro Ser Ser Ser Gln Asp Ser Glu Asn Ile Thr
            180                 185                 190

gct gca gcc ctg gct acg ggt gcc tgc atc gta gga atc ctc tgc ctc 624
Ala Ala Ala Leu Ala Thr Gly Ala Cys Ile Val Gly Ile Leu Cys Leu
        195                 200                 205

ccc ctc atc ctg ctc ctg gtc tac aag caa agg cag gca gcc tcc aac 672
Pro Leu Ile Leu Leu Leu Val Tyr Lys Gln Arg Gln Ala Ala Ser Asn
    210                 215                 220

cgc cgt gcc cag gag ctg gtg cgg atg gac agc aac att caa ggg att 720
Arg Arg Ala Gln Glu Leu Val Arg Met Asp Ser Asn Ile Gln Gly Ile
225                 230                 235                 240

gaa aac ccc ggc ttt gaa gcc tca cca cct gcc cag ggg ata ccc gag 768
Glu Asn Pro Gly Phe Glu Ala Ser Pro Pro Ala Gln Gly Ile Pro Glu
                245                 250                 255

gcc aaa gtc agg cac ccc ctg tcc tat gtg gcc cag cgg cag cct tct 816
Ala Lys Val Arg His Pro Leu Ser Tyr Val Ala Gln Arg Gln Pro Ser
            260                 265                 270

gag tct ggg cgg cat ctg ctt tcg gag ccc agc acc ccc ctg tct cct 864
Glu Ser Gly Arg His Leu Leu Ser Glu Pro Ser Thr Pro Leu Ser Pro
        275                 280                 285

cca ggc ccc gga gac gtc ttc ttc cca tcc ctg gac cct gtc cct gac 912
Pro Gly Pro Gly Asp Val Phe Phe Pro Ser Leu Asp Pro Val Pro Asp
    290                 295                 300

tct cca aac ttt gag gtc atc tag 936
Ser Pro Asn Phe Glu Val Ile
305                 310

<210> 4
<211> 311
<212> PRT
<213> Homo sapiens

<400> 4

Met Gly Val Pro Thr Ala Leu Glu Ala Gly Ser Trp Arg Trp Gly Ser
1               5                   10                  15

Leu Leu Phe Ala Leu Phe Leu Ala Ala Ser Leu Gly Pro Val Ala Ala
            20                  25                  30

Phe Lys Val Ala Thr Pro Tyr Ser Leu Tyr Val Cys Pro Glu Gly Gln
        35                  40                  45

Asn Val Thr Leu Thr Cys Arg Leu Leu Gly Pro Val Asp Lys Gly His
    50                  55                  60

Asp Val Thr Phe Tyr Lys Thr Trp Tyr Arg Ser Ser Arg Gly Glu Val
65                  70                  75                  80

Gln Thr Cys Ser Glu Arg Arg Pro Ile Arg Asn Leu Thr Phe Gln Asp
                85                  90                  95

Leu His Leu His His Gly Gly His Gln Ala Ala Asn Thr Ser His Asp
            100                 105                 110

Leu Ala Gln Arg His Gly Leu Glu Ser Ala Ser Asp His His Gly Asn
        115                 120                 125

Phe Ser Ile Thr Met Arg Asn Leu Thr Leu Leu Asp Ser Gly Leu Tyr
    130                 135                 140

Cys Cys Leu Val Val Glu Ile Arg His His His Ser Glu His Arg Val
145                 150                 155                 160

His Gly Ala Met Glu Leu Gln Val Gln Thr Gly Lys Asp Ala Pro Ser
                165                 170                 175

Asn Cys Val Val Tyr Pro Ser Ser Ser Gln Asp Ser Glu Asn Ile Thr
            180                 185                 190

Ala Ala Ala Leu Ala Thr Gly Ala Cys Ile Val Gly Ile Leu Cys Leu
        195                 200                 205

Pro Leu Ile Leu Leu Leu Val Tyr Lys Gln Arg Gln Ala Ala Ser Asn
    210                 215                 220

Arg Arg Ala Gln Glu Leu Val Arg Met Asp Ser Asn Ile Gln Gly Ile
225                 230                 235                 240

Glu Asn Pro Gly Phe Glu Ala Ser Pro Pro Ala Gln Gly Ile Pro Glu
                245                 250                 255

Ala Lys Val Arg His Pro Leu Ser Tyr Val Ala Gln Arg Gln Pro Ser
            260                 265                 270

Glu Ser Gly Arg His Leu Leu Ser Glu Pro Ser Thr Pro Leu Ser Pro
        275                 280                 285

Pro Gly Pro Gly Asp Val Phe Phe Pro Ser Leu Asp Pro Val Pro Asp
    290                 295                 300

Ser Pro Asn Phe Glu Val Ile
305                 310

<210>  5
<211>  345
<212>  DNA
<213>  Homo sapiens

<220>
<221>  CDS
<222>  (1)..(345)

<400>  5
atg gca ggc ttg gcc ctg cag cca ggc act gcc ctg ctg tgc tac tcc      48
Met Ala Gly Leu Ala Leu Gln Pro Gly Thr Ala Leu Leu Cys Tyr Ser
1               5                   10                  15

tgc aaa gcc cag gtg agc aac gag gac tgc ctg cag gtg gag aac tgc      96
Cys Lys Ala Gln Val Ser Asn Glu Asp Cys Leu Gln Val Glu Asn Cys
            20                  25                  30

acc cag ctg ggg gag cag tgc tgg acc gcg cgc atc cgc gca gtt ggc      144
Thr Gln Leu Gly Glu Gln Cys Trp Thr Ala Arg Ile Arg Ala Val Gly
        35                  40                  45

ctc ctg acc gtc atc agc aaa ggc tgc agc ttg aac tgc gtg gat gac      192
Leu Leu Thr Val Ile Ser Lys Gly Cys Ser Leu Asn Cys Val Asp Asp
    50                  55                  60

tca cag gac tac tac gtg ggc aag aag aac atc acg tgc tgt gac acc      240
Ser Gln Asp Tyr Tyr Val Gly Lys Lys Asn Ile Thr Cys Cys Asp Thr
65                  70                  75                  80

gac ttg tgc aac gcc agc ggg gcc cat gcc ctg cag ccg gct gct gcc      288
Asp Leu Cys Asn Ala Ser Gly Ala His Ala Leu Gln Pro Ala Ala Ala
                85                  90                  95

atc ctt gcg ctg ctc cct gca ctc ggc ctg ctg ctc tgg gga ccc ggc      336
Ile Leu Ala Leu Leu Pro Ala Leu Gly Leu Leu Leu Trp Gly Pro Gly
            100                 105                 110

cag ctc tag                                                          345
Gln Leu

<210>  6
<211>  114
<212>  PRT
<213>  Homo sapiens

<400>  6

Met Ala Gly Leu Ala Leu Gln Pro Gly Thr Ala Leu Leu Cys Tyr Ser
1               5                   10                  15

Cys Lys Ala Gln Val Ser Asn Glu Asp Cys Leu Gln Val Glu Asn Cys
            20                  25                  30

Thr Gln Leu Gly Glu Gln Cys Trp Thr Ala Arg Ile Arg Ala Val Gly
        35                  40                  45

Leu Leu Thr Val Ile Ser Lys Gly Cys Ser Leu Asn Cys Val Asp Asp
    50                  55                  60

Ser Gln Asp Tyr Tyr Val Gly Lys Lys Asn Ile Thr Cys Cys Asp Thr
65                  70                  75                  80

Asp Leu Cys Asn Ala Ser Gly Ala His Ala Leu Gln Pro Ala Ala Ala
                85                  90                  95

Ile Leu Ala Leu Leu Pro Ala Leu Gly Leu Leu Leu Trp Gly Pro Gly
            100                 105                 110

Gln Leu

<210>  7
<211>  1782
<212>  DNA
<213>  Homo sapiens

<220>
<221>  CDS
<222>  (1)..(1782)

<400>  7
atg gag cag cct gag gac atg gcg tcg ctg agc gag ttc gac tcc ttg      48
Met Glu Gln Pro Glu Asp Met Ala Ser Leu Ser Glu Phe Asp Ser Leu
1               5                   10                  15

gcg ggc agc atc ccg gcc acc aag gtg gag atc acc gtg tcc tgc agg      96
Ala Gly Ser Ile Pro Ala Thr Lys Val Glu Ile Thr Val Ser Cys Arg
            20                  25                  30

aac ctc ctg gac aaa gac atg ttt tcc aag tcc gac cca ctg tgc gtc      144
Asn Leu Leu Asp Lys Asp Met Phe Ser Lys Ser Asp Pro Leu Cys Val
        35                  40                  45

atg tat acc caa ggg atg gag aac aag cag tgg cgg gag ttt ggg cgc      192
Met Tyr Thr Gln Gly Met Glu Asn Lys Gln Trp Arg Glu Phe Gly Arg
    50                  55                  60

acc gaa gtc atc gac aac acg ctc aat cct gac ttc gtg cgc aag ttc      240
Thr Glu Val Ile Asp Asn Thr Leu Asn Pro Asp Phe Val Arg Lys Phe
65                  70                  75                  80

att gtg gat tac ttt ttc gag gag aag cag aac ctc cgt ttt gat cta      288
Ile Val Asp Tyr Phe Phe Glu Glu Lys Gln Asn Leu Arg Phe Asp Leu
                85                  90                  95

tac gac gtt gac tct aag agt cct gat tta tcc aaa cac gat ttc ctg      336
Tyr Asp Val Asp Ser Lys Ser Pro Asp Leu Ser Lys His Asp Phe Leu
            100                 105                 110

ggc cag gcc ttc tgc acc ctt gga gag att gtg ggg tcc cct ggg agc      384
Gly Gln Ala Phe Cys Thr Leu Gly Glu Ile Val Gly Ser Pro Gly Ser
        115                 120                 125

cgc ctg gaa aag ccc ctc acg ata ggc gca ttc agc ctg aat tcc agg      432
Arg Leu Glu Lys Pro Leu Thr Ile Gly Ala Phe Ser Leu Asn Ser Arg
    130                 135                 140

acg ggc aaa ccc atg cca gct gtg tcc aac ggt ggt gtc cca gga aag      480
Thr Gly Lys Pro Met Pro Ala Val Ser Asn Gly Gly Val Pro Gly Lys
145                 150                 155                 160

aaa tgt ggc acc atc atc ctg tcc gct gag gag ctc agc aac tgt agg     528
Lys Cys Gly Thr Ile Ile Leu Ser Ala Glu Glu Leu Ser Asn Cys Arg
                165                 170                 175

gat gtc gcc acc atg cag ttc tgt gcc aac aag ctg gac aag aaa gat     576
Asp Val Ala Thr Met Gln Phe Cys Ala Asn Lys Leu Asp Lys Lys Asp
            180                 185                 190

ttc ttt ggg aaa tct gac ccc ttc ttg gta ttc tac aga agc aac gag     624
Phe Phe Gly Lys Ser Asp Pro Phe Leu Val Phe Tyr Arg Ser Asn Glu
        195                 200                 205

gat gga acg ttc acc att tgc cac aag acc gag gtc atg aag aac acc     672
Asp Gly Thr Phe Thr Ile Cys His Lys Thr Glu Val Met Lys Asn Thr
    210                 215                 220

cta aat cca gtc tgg caa act ttc tcc att ccc gtg aga gcc ctc tgc     720
Leu Asn Pro Val Trp Gln Thr Phe Ser Ile Pro Val Arg Ala Leu Cys
225                 230                 235                 240

aac ggc gac tac gat cgg acc atc aag gtg gag gtg tac gac tgg gat     768
Asn Gly Asp Tyr Asp Arg Thr Ile Lys Val Glu Val Tyr Asp Trp Asp
                245                 250                 255

cgg gac ggc agc cat gac ttc att ggg gag ttc acc acc agt tac cgg     816
Arg Asp Gly Ser His Asp Phe Ile Gly Glu Phe Thr Thr Ser Tyr Arg
            260                 265                 270

gag ctg gcc cgt ggg cag agc caa ttc aac atc tat gag gtg gta aac     864
Glu Leu Ala Arg Gly Gln Ser Gln Phe Asn Ile Tyr Glu Val Val Asn
        275                 280                 285

ccg aaa aag aaa atg aag aaa aag aaa tac gtg aat tct ggc aca gtc     912
Pro Lys Lys Lys Met Lys Lys Lys Lys Tyr Val Asn Ser Gly Thr Val
    290                 295                 300

acc ctg ctt tcc ttt gct gtg gag tca gag tgc acc ttc ctt gac tac     960
Thr Leu Leu Ser Phe Ala Val Glu Ser Glu Cys Thr Phe Leu Asp Tyr
305                 310                 315                 320

atc aaa gga ggg acc cag atc aac ttc act gtg gcc att gat ttc act     1008
Ile Lys Gly Gly Thr Gln Ile Asn Phe Thr Val Ala Ile Asp Phe Thr
                325                 330                 335

gcc tcc aat ggg aac ccc tca cag tcc aca tcc ctg cac tac atg agc     1056
Ala Ser Asn Gly Asn Pro Ser Gln Ser Thr Ser Leu His Tyr Met Ser
            340                 345                 350

ccc tac cag ctg aac gcc tac gcg ctg gcg ctg act gcc gtc gga gag     1104
Pro Tyr Gln Leu Asn Ala Tyr Ala Leu Ala Leu Thr Ala Val Gly Glu
        355                 360                 365

atc atc cag cac tac gac agt gac aag atg ttc cct gcc ctg ggc ttc     1152
Ile Ile Gln His Tyr Asp Ser Asp Lys Met Phe Pro Ala Leu Gly Phe
    370                 375                 380

ggg gcc aag ctg ccc ccg gat ggc aga gtg tcc cac gag ttc cca ctg     1200
Gly Ala Lys Leu Pro Pro Asp Gly Arg Val Ser His Glu Phe Pro Leu
385                 390                 395                 400

aat ggc aac cag gag aac ccc tca tgc tgt ggc atc gac ggc atc ctg     1248
Asn Gly Asn Gln Glu Asn Pro Ser Cys Cys Gly Ile Asp Gly Ile Leu
                405                 410                 415

gag gcc tac cac cgc agc ctg cgc act gtg cag ctg tac ggc ccc acc     1296

Glu Ala Tyr His Arg Ser Leu Arg Thr Val Gln Leu Tyr Gly Pro Thr
            420                 425                 430

aac ttt gcc ccc gtg gtc acc cac gtg gcc agg aat gca gcg gcc gtg 1344
Asn Phe Ala Pro Val Val Thr His Val Ala Arg Asn Ala Ala Ala Val
        435                 440                 445

cag gat ggc tcc cag tac tcg gtg ctg ctc atc att act gat ggg gtc 1392
Gln Asp Gly Ser Gln Tyr Ser Val Leu Leu Ile Ile Thr Asp Gly Val
    450                 455                 460

atc tcg gac atg gcg cag acc aag gag gcc att gtc aac gct gcc aag 1440
Ile Ser Asp Met Ala Gln Thr Lys Glu Ala Ile Val Asn Ala Ala Lys
465                 470                 475                 480

ctc ccc atg tcc atc att atc gtc ggc gtg ggc cag gca gag ttc gac 1488
Leu Pro Met Ser Ile Ile Ile Val Gly Val Gly Gln Ala Glu Phe Asp
                485                 490                 495

gcc atg gtg gag ctg gat ggc gac gac gtg cgg atc tcc tcc cgg ggg 1536
Ala Met Val Glu Leu Asp Gly Asp Asp Val Arg Ile Ser Ser Arg Gly
            500                 505                 510

aag ctg gct gaa cgc gac atc gtc cag ttt gta ccc ttc cgg gac tac 1584
Lys Leu Ala Glu Arg Asp Ile Val Gln Phe Val Pro Phe Arg Asp Tyr
        515                 520                 525

gtg gac cgc aca ggc aac cac gtg ctg agc atg gcc cgc ctg gcc cga 1632
Val Asp Arg Thr Gly Asn His Val Leu Ser Met Ala Arg Leu Ala Arg
    530                 535                 540

gac gtg ctg gca gag atc cct gac caa ctg gtg tcc tac atg aag gca 1680
Asp Val Leu Ala Glu Ile Pro Asp Gln Leu Val Ser Tyr Met Lys Ala
545                 550                 555                 560

cag ggc att cgc ccg cgt ccc cca ccc gca gca cca acc cac tcg ccc 1728
Gln Gly Ile Arg Pro Arg Pro Pro Pro Ala Ala Pro Thr His Ser Pro
                565                 570                 575

tcg cag tcc cca gcc cgc acg ccc cct gcg tcc ccc ctg cac acg cac 1776
Ser Gln Ser Pro Ala Arg Thr Pro Pro Ala Ser Pro Leu His Thr His
            580                 585                 590

atc tga 1782
Ile

<210> 8
<211> 593
<212> PRT
<213> Homo sapiens

<400> 8

Met Glu Gln Pro Glu Asp Met Ala Ser Leu Ser Glu Phe Asp Ser Leu
1               5                   10                  15

Ala Gly Ser Ile Pro Ala Thr Lys Val Glu Ile Thr Val Ser Cys Arg
            20                  25                  30

Asn Leu Leu Asp Lys Asp Met Phe Ser Lys Ser Asp Pro Leu Cys Val
        35                  40                  45

Met Tyr Thr Gln Gly Met Glu Asn Lys Gln Trp Arg Glu Phe Gly Arg
    50                  55                  60

Thr Glu Val Ile Asp Asn Thr Leu Asn Pro Asp Phe Val Arg Lys Phe
65                  70                  75                  80

Ile Val Asp Tyr Phe Phe Glu Glu Lys Gln Asn Leu Arg Phe Asp Leu
                85                  90                  95

Tyr Asp Val Asp Ser Lys Ser Pro Asp Leu Ser Lys His Asp Phe Leu
            100                 105                 110

Gly Gln Ala Phe Cys Thr Leu Gly Glu Ile Val Gly Ser Pro Gly Ser
        115                 120                 125

Arg Leu Glu Lys Pro Leu Thr Ile Gly Ala Phe Ser Leu Asn Ser Arg
    130                 135                 140

Thr Gly Lys Pro Met Pro Ala Val Ser Asn Gly Gly Val Pro Gly Lys
145                 150                 155                 160

Lys Cys Gly Thr Ile Ile Leu Ser Ala Glu Glu Leu Ser Asn Cys Arg
                165                 170                 175

Asp Val Ala Thr Met Gln Phe Cys Ala Asn Lys Leu Asp Lys Lys Asp
            180                 185                 190

Phe Phe Gly Lys Ser Asp Pro Phe Leu Val Phe Tyr Arg Ser Asn Glu
        195                 200                 205

Asp Gly Thr Phe Thr Ile Cys His Lys Thr Glu Val Met Lys Asn Thr
    210                 215                 220

Leu Asn Pro Val Trp Gln Thr Phe Ser Ile Pro Val Arg Ala Leu Cys
225                 230                 235                 240

Asn Gly Asp Tyr Asp Arg Thr Ile Lys Val Glu Val Tyr Asp Trp Asp
                245                 250                 255

Arg Asp Gly Ser His Asp Phe Ile Gly Glu Phe Thr Thr Ser Tyr Arg
            260                 265                 270

Glu Leu Ala Arg Gly Gln Ser Gln Phe Asn Ile Tyr Glu Val Val Asn
        275                 280                 285

Pro Lys Lys Lys Met Lys Lys Lys Lys Tyr Val Asn Ser Gly Thr Val
    290                 295                 300

Thr Leu Leu Ser Phe Ala Val Glu Ser Glu Cys Thr Phe Leu Asp Tyr
305                 310                 315                 320

Ile Lys Gly Gly Thr Gln Ile Asn Phe Thr Val Ala Ile Asp Phe Thr
                325                 330                 335

Ala Ser Asn Gly Asn Pro Ser Gln Ser Thr Ser Leu His Tyr Met Ser
            340                 345                 350

Pro Tyr Gln Leu Asn Ala Tyr Ala Leu Ala Leu Thr Ala Val Gly Glu
        355                 360                 365

Ile Ile Gln His Tyr Asp Ser Asp Lys Met Phe Pro Ala Leu Gly Phe
    370                 375                 380

Gly Ala Lys Leu Pro Pro Asp Gly Arg Val Ser His Glu Phe Pro Leu
385                 390                 395                 400

Asn Gly Asn Gln Glu Asn Pro Ser Cys Cys Gly Ile Asp Gly Ile Leu
                405                 410                 415

Glu Ala Tyr His Arg Ser Leu Arg Thr Val Gln Leu Tyr Gly Pro Thr
            420                 425                 430

Asn Phe Ala Pro Val Val Thr His Val Ala Arg Asn Ala Ala Ala Val
        435                 440                 445

Gln Asp Gly Ser Gln Tyr Ser Val Leu Leu Ile Ile Thr Asp Gly Val
    450                 455                 460

Ile Ser Asp Met Ala Gln Thr Lys Glu Ala Ile Val Asn Ala Ala Lys
465                 470                 475                 480

Leu Pro Met Ser Ile Ile Ile Val Gly Val Gly Gln Ala Glu Phe Asp
                485                 490                 495

Ala Met Val Glu Leu Asp Gly Asp Asp Val Arg Ile Ser Ser Arg Gly
            500                 505                 510

Lys Leu Ala Glu Arg Asp Ile Val Gln Phe Val Pro Phe Arg Asp Tyr
        515                 520                 525

Val Asp Arg Thr Gly Asn His Val Leu Ser Met Ala Arg Leu Ala Arg
    530                 535                 540

Asp Val Leu Ala Glu Ile Pro Asp Gln Leu Val Ser Tyr Met Lys Ala
545                 550                 555                 560

Gln Gly Ile Arg Pro Arg Pro Pro Pro Ala Ala Pro Thr His Ser Pro
                565                 570                 575

Ser Gln Ser Pro Ala Arg Thr Pro Pro Ala Ser Pro Leu His Thr His
            580                 585                 590

Ile

<210>  9
<211>  1005
<212>  DNA
<213>  Homo sapiens

<220>
<221>  CDS
<222>  (1)..(1005)

<400>  9
atg gca gcg gcc gcc agc ccc gcg ttc ctt cta cgc ctc ccg ctt ctg      48
Met Ala Ala Ala Ala Ser Pro Ala Phe Leu Leu Arg Leu Pro Leu Leu
1               5                   10                  15

ctc ctg ctg tcc agc tgg tgc agg acc ggg ctg gcc gac cct cac tct      96
Leu Leu Leu Ser Ser Trp Cys Arg Thr Gly Leu Ala Asp Pro His Ser
            20                  25                  30

ctt tgc tat gac atc acc gtc atc cct aag ttc aga cct gga cca cgg      144
Leu Cys Tyr Asp Ile Thr Val Ile Pro Lys Phe Arg Pro Gly Pro Arg
        35                  40                  45

tgg tgt gcg gtt caa ggc cag gtg gat gaa aag act ttt ctt cac tat      192
Trp Cys Ala Val Gln Gly Gln Val Asp Glu Lys Thr Phe Leu His Tyr
    50                  55                  60

gac tgt ggc agc aag aca gtc aca ccc gtc agt ccc ctg ggg aag aaa      240
Asp Cys Gly Ser Lys Thr Val Thr Pro Val Ser Pro Leu Gly Lys Lys
65                  70                  75                  80

cta aat gtc aca acg gcc tgg aaa gca cag aac cca gta ctg aga gag      288
Leu Asn Val Thr Thr Ala Trp Lys Ala Gln Asn Pro Val Leu Arg Glu
                85                  90                  95

gtg gtg gac ata ctt aca gag caa ctg ctt gac att cag ctg gag aat      336
Val Val Asp Ile Leu Thr Glu Gln Leu Leu Asp Ile Gln Leu Glu Asn
            100                 105                 110

tac ata ccc aag gaa ccc ctc acc ctg cag gcc agg atg tct tgt gag      384
Tyr Ile Pro Lys Glu Pro Leu Thr Leu Gln Ala Arg Met Ser Cys Glu
        115                 120                 125

cag aaa gcc gaa gga cac ggc agt gga tct tgg cag ctc agt ttc gat      432
Gln Lys Ala Glu Gly His Gly Ser Gly Ser Trp Gln Leu Ser Phe Asp
    130                 135                 140

gga cag atc ttc ctc ctc ttt gac tca gaa aac aga atg tgg aca acg      480
Gly Gln Ile Phe Leu Leu Phe Asp Ser Glu Asn Arg Met Trp Thr Thr
145                 150                 155                 160

gtt cat cct gga gcc aga aag atg aaa gaa aag tgg gag aat gac aag      528
Val His Pro Gly Ala Arg Lys Met Lys Glu Lys Trp Glu Asn Asp Lys

                165                 170                 175

gat atg acc atg tcc ttc cat tac atc tca atg gga gac tgc aca gga 576
Asp Met Thr Met Ser Phe His Tyr Ile Ser Met Gly Asp Cys Thr Gly
            180                 185                 190

tgg ctt gag gac ttc ttg atg ggc atg gac agc acc ctg gag cca agt 624
Trp Leu Glu Asp Phe Leu Met Gly Met Asp Ser Thr Leu Glu Pro Ser
        195                 200                 205

gca gga gca cca ccc acc atg tcc tca ggc aca gcc caa ccc agg gcc 672
Ala Gly Ala Pro Pro Thr Met Ser Ser Gly Thr Ala Gln Pro Arg Ala
    210                 215                 220

acg gcc acc acc ctc atc ctt tgc tgc ctc ctc atc atg tgt ctc ctc 720
Thr Ala Thr Thr Leu Ile Leu Cys Cys Leu Leu Ile Met Cys Leu Leu
225                 230                 235                 240

ata tgc tcc agg cac agt ctg acc caa agc cat ggc cac cac cct cag 768
Ile Cys Ser Arg His Ser Leu Thr Gln Ser His Gly His His Pro Gln
                245                 250                 255

tcc ctg cag cct cct cct cat cct ccc ctg ctt cat cct acc tgg ctg 816
Ser Leu Gln Pro Pro Pro His Pro Pro Leu Leu His Pro Thr Trp Leu
            260                 265                 270

ctg aga aga gtc ctt tgg agt gac agc tac caa ata gcg aag cgc ccc 864
Leu Arg Arg Val Leu Trp Ser Asp Ser Tyr Gln Ile Ala Lys Arg Pro
        275                 280                 285

ttg tct ggt gga cac gtg act cgc gtg act tta cct atc att gga gac 912
Leu Ser Gly Gly His Val Thr Arg Val Thr Leu Pro Ile Ile Gly Asp
    290                 295                 300

gac tca cac tcc tta ccc tgc cct ctt gcc ttg tat aca ata aat aac 960
Asp Ser His Ser Leu Pro Cys Pro Leu Ala Leu Tyr Thr Ile Asn Asn
305                 310                 315                 320

ggc gca gcc agg tat tcg gag cca cta cag gtc tcc ata tct tga 1005
Gly Ala Ala Arg Tyr Ser Glu Pro Leu Gln Val Ser Ile Ser
                325                 330

<210> 10
<211> 334
<212> PRT
<213> Homo sapiens

<400> 10

Met Ala Ala Ala Ala Ser Pro Ala Phe Leu Leu Arg Leu Pro Leu Leu
1               5                   10                  15

Leu Leu Leu Ser Ser Trp Cys Arg Thr Gly Leu Ala Asp Pro His Ser
            20                  25                  30

Leu Cys Tyr Asp Ile Thr Val Ile Pro Lys Phe Arg Pro Gly Pro Arg
        35                  40                  45

Trp Cys Ala Val Gln Gly Gln Val Asp Glu Lys Thr Phe Leu His Tyr
    50                  55                  60

Asp Cys Gly Ser Lys Thr Val Thr Pro Val Ser Pro Leu Gly Lys Lys
65                  70                  75                  80

Leu Asn Val Thr Thr Ala Trp Lys Ala Gln Asn Pro Val Leu Arg Glu
                85                  90                  95

Val Val Asp Ile Leu Thr Glu Gln Leu Leu Asp Ile Gln Leu Glu Asn
            100                 105                 110

Tyr Ile Pro Lys Glu Pro Leu Thr Leu Gln Ala Arg Met Ser Cys Glu
        115                 120                 125

Gln Lys Ala Glu Gly His Gly Ser Gly Ser Trp Gln Leu Ser Phe Asp
    130                 135                 140

Gly Gln Ile Phe Leu Leu Phe Asp Ser Glu Asn Arg Met Trp Thr Thr
145                 150                 155                 160

Val His Pro Gly Ala Arg Lys Met Lys Glu Lys Trp Glu Asn Asp Lys
                165                 170                 175

Asp Met Thr Met Ser Phe His Tyr Ile Ser Met Gly Asp Cys Thr Gly
            180                 185                 190

Trp Leu Glu Asp Phe Leu Met Gly Met Asp Ser Thr Leu Glu Pro Ser
        195                 200                 205

Ala Gly Ala Pro Pro Thr Met Ser Ser Gly Thr Ala Gln Pro Arg Ala
    210                 215                 220

Thr Ala Thr Thr Leu Ile Leu Cys Cys Leu Leu Ile Met Cys Leu Leu
225                 230                 235                 240

Ile Cys Ser Arg His Ser Leu Thr Gln Ser His Gly His His Pro Gln
                245                 250                 255

Ser Leu Gln Pro Pro Pro His Pro Pro Leu Leu His Pro Thr Trp Leu
            260                 265                 270

Leu Arg Arg Val Leu Trp Ser Asp Ser Tyr Gln Ile Ala Lys Arg Pro
        275                 280                 285

Leu Ser Gly Gly His Val Thr Arg Val Thr Leu Pro Ile Ile Gly Asp
    290                 295                 300

Asp Ser His Ser Leu Pro Cys Pro Leu Ala Leu Tyr Thr Ile Asn Asn
305                 310                 315                 320

Gly Ala Ala Arg Tyr Ser Glu Pro Leu Gln Val Ser Ile Ser
                325                 330

<210>  11
<211>  939
<212>  DNA
<213>  Homo sapiens

<220>
<221>  CDS
<222>  (1)..(939)

<400>  11
atg gtt gga ctg aag cct tca gac gtg cct ccc acc atg gct gtg aag      48
Met Val Gly Leu Lys Pro Ser Asp Val Pro Pro Thr Met Ala Val Lys
1               5                   10                  15

ttc ctg ggg gca ggc aca gca gcc tgt ttt gct gac ctc gtt acc ttt      96
Phe Leu Gly Ala Gly Thr Ala Ala Cys Phe Ala Asp Leu Val Thr Phe
            20                  25                  30

cca ctg gac aca gcc aag gtc cgc ctg cag atc cag ggg gag aac cag      144
Pro Leu Asp Thr Ala Lys Val Arg Leu Gln Ile Gln Gly Glu Asn Gln
        35                  40                  45

gcg gtc cag acg gcc cgg ctc gtg cag tac cgt ggc gtg ctg ggc acc      192
Ala Val Gln Thr Ala Arg Leu Val Gln Tyr Arg Gly Val Leu Gly Thr
    50                  55                  60

atc ctg acc atg gtg cgg act gag ggt ccc tgc agc ccc tac aat ggg      240
Ile Leu Thr Met Val Arg Thr Glu Gly Pro Cys Ser Pro Tyr Asn Gly
65                  70                  75                  80

ctg gtg gcc ggc ctg cag cgc cag atg agc ttc gcc tcc atc cgc atc      288
Leu Val Ala Gly Leu Gln Arg Gln Met Ser Phe Ala Ser Ile Arg Ile
                85                  90                  95

ggc ctc tat gac tcc gtc aag cag gtg tac acc ccc aaa ggc gcg gac      336
Gly Leu Tyr Asp Ser Val Lys Gln Val Tyr Thr Pro Lys Gly Ala Asp
            100                 105                 110

aac tcc agc ctc act acc cgg att ttg gcc ggc tgc acc aca gga gcc      384
Asn Ser Ser Leu Thr Thr Arg Ile Leu Ala Gly Cys Thr Thr Gly Ala
        115                 120                 125

atg gcg gtg acc tgt gcc cag ccc aca gat gtg gtg aag gtc cga ttt      432
Met Ala Val Thr Cys Ala Gln Pro Thr Asp Val Val Lys Val Arg Phe
    130                 135                 140

cag gcc agc ata cac ctc ggg cca tcc agg agc gac aga aaa tac agc      480
Gln Ala Ser Ile His Leu Gly Pro Ser Arg Ser Asp Arg Lys Tyr Ser
145                 150                 155                 160

ggg act atg gac gcc tac aga acc atc gcc agg gag gaa gga gtc agg      528
Gly Thr Met Asp Ala Tyr Arg Thr Ile Ala Arg Glu Glu Gly Val Arg
                165                 170                 175

ggc ctg tgg aaa gga act ttg ccc aac atc atg agg aat gct atc gtc      576
Gly Leu Trp Lys Gly Thr Leu Pro Asn Ile Met Arg Asn Ala Ile Val
            180                 185                 190

aac tgt gct gag gtg gtg acc tac gac atc ctc aag gag aag ctg ctg      624
Asn Cys Ala Glu Val Val Thr Tyr Asp Ile Leu Lys Glu Lys Leu Leu

195                 200                 205

gac tac cac ctg ctc act gac aac ttc ccc tgc cac ttt gtc tct gcc      672
Asp Tyr His Leu Leu Thr Asp Asn Phe Pro Cys His Phe Val Ser Ala
    210                 215                 220

ttt gga gcc ggc ttc tgt gcc aca gtg gtg gcc tcc ccg gtg gac gtg      720
Phe Gly Ala Gly Phe Cys Ala Thr Val Val Ala Ser Pro Val Asp Val
225                 230                 235                 240

gtg aag acc cgg tat atg aac tca cct cca ggc cag tac ttc agc ccc      768
Val Lys Thr Arg Tyr Met Asn Ser Pro Pro Gly Gln Tyr Phe Ser Pro
                245                 250                 255

ctc gac tgt atg ata aag atg gtg gcc cag gag ggc ccc aca gcc ttc      816
Leu Asp Cys Met Ile Lys Met Val Ala Gln Glu Gly Pro Thr Ala Phe
            260                 265                 270

tac aag gga ttt aca ccc tcc ttt ttg cgt ttg gga tcc tgg aac gtg      864
Tyr Lys Gly Phe Thr Pro Ser Phe Leu Arg Leu Gly Ser Trp Asn Val
        275                 280                 285

gtg atg ttc gta acc tat gag cag ctg aaa cgg gcc ctg atg aaa gtc      912
Val Met Phe Val Thr Tyr Glu Gln Leu Lys Arg Ala Leu Met Lys Val
    290                 295                 300

cag atg tta cgg gaa tca ccg ttt tga                                  939
Gln Met Leu Arg Glu Ser Pro Phe
305                 310

<210>  12
<211>  312
<212>  PRT
<213>  Homo sapiens

<400>  12

Met Val Gly Leu Lys Pro Ser Asp Val Pro Pro Thr Met Ala Val Lys
1               5                   10                  15

Phe Leu Gly Ala Gly Thr Ala Ala Cys Phe Ala Asp Leu Val Thr Phe
            20                  25                  30

Pro Leu Asp Thr Ala Lys Val Arg Leu Gln Ile Gln Gly Glu Asn Gln
        35                  40                  45

Ala Val Gln Thr Ala Arg Leu Val Gln Tyr Arg Gly Val Leu Gly Thr
    50                  55                  60

Ile Leu Thr Met Val Arg Thr Glu Gly Pro Cys Ser Pro Tyr Asn Gly
65                  70                  75                  80

Leu Val Ala Gly Leu Gln Arg Gln Met Ser Phe Ala Ser Ile Arg Ile
                85                  90                  95

Gly Leu Tyr Asp Ser Val Lys Gln Val Tyr Thr Pro Lys Gly Ala Asp
            100                 105                 110

Asn Ser Ser Leu Thr Thr Arg Ile Leu Ala Gly Cys Thr Thr Gly Ala
        115                 120                 125

Met Ala Val Thr Cys Ala Gln Pro Thr Asp Val Val Lys Val Arg Phe
    130                 135                 140

Gln Ala Ser Ile His Leu Gly Pro Ser Arg Ser Asp Arg Lys Tyr Ser
145                 150                 155                 160

Gly Thr Met Asp Ala Tyr Arg Thr Ile Ala Arg Glu Glu Gly Val Arg
                165                 170                 175

Gly Leu Trp Lys Gly Thr Leu Pro Asn Ile Met Arg Asn Ala Ile Val
            180                 185                 190

Asn Cys Ala Glu Val Val Thr Tyr Asp Ile Leu Lys Glu Lys Leu Leu
        195                 200                 205

Asp Tyr His Leu Leu Thr Asp Asn Phe Pro Cys His Phe Val Ser Ala
    210                 215                 220

Phe Gly Ala Gly Phe Cys Ala Thr Val Val Ala Ser Pro Val Asp Val
225                 230                 235                 240

Val Lys Thr Arg Tyr Met Asn Ser Pro Pro Gly Gln Tyr Phe Ser Pro
                245                 250                 255

Leu Asp Cys Met Ile Lys Met Val Ala Gln Glu Gly Pro Thr Ala Phe
            260                 265                 270

Tyr Lys Gly Phe Thr Pro Ser Phe Leu Arg Leu Gly Ser Trp Asn Val
        275                 280                 285

Val Met Phe Val Thr Tyr Glu Gln Leu Lys Arg Ala Leu Met Lys Val
    290                 295                 300

Gln Met Leu Arg Glu Ser Pro Phe
305                 310

<210>  13
<211>  828
<212>  DNA
<213>  Homo sapiens

<220>
<221>  CDS
<222>  (1)..(828)

<400>  13
atg gtt gga ctg aag cct tca gac gtg cct ccc acc atg gct gtg aag        48

Met Val Gly Leu Lys Pro Ser Asp Val Pro Pro Thr Met Ala Val Lys
1               5                   10                  15

ttc ctg ggg gca ggc aca gca gcc tgt ttt gct gac ctc gtt acc ttt      96
Phe Leu Gly Ala Gly Thr Ala Ala Cys Phe Ala Asp Leu Val Thr Phe
            20                  25                  30

cca ctg gac aca gcc aag gtc cgc ctg cag atc cag ggg gag aac cag      144
Pro Leu Asp Thr Ala Lys Val Arg Leu Gln Ile Gln Gly Glu Asn Gln
        35                  40                  45

gcg gtc cag acg gcc cgg ctc gtg cag tac cgt ggc gtg ctg ggc acc      192
Ala Val Gln Thr Ala Arg Leu Val Gln Tyr Arg Gly Val Leu Gly Thr
    50                  55                  60

atc ctg acc atg gtg cgg act gag ggt ccc tgc agc ccc tac aat ggg      240
Ile Leu Thr Met Val Arg Thr Glu Gly Pro Cys Ser Pro Tyr Asn Gly
65                  70                  75                  80

ctg gtg gcc ggc ctg cag cgc cag atg agc ttc gcc tcc atc cgc atc      288
Leu Val Ala Gly Leu Gln Arg Gln Met Ser Phe Ala Ser Ile Arg Ile
                85                  90                  95

ggc ctc tat gac tcc gtc aag cag gtg tac acc ccc aaa ggc gcg gac      336
Gly Leu Tyr Asp Ser Val Lys Gln Val Tyr Thr Pro Lys Gly Ala Asp
            100                 105                 110

aac tcc agc ctc act acc cgg att ttg gcc ggc tgc acc aca gga gcc      384
Asn Ser Ser Leu Thr Thr Arg Ile Leu Ala Gly Cys Thr Thr Gly Ala
        115                 120                 125

atg gcg gtg acc tgt gcc cag ccc aca gat gtg gtg aag gtc cga ttt      432
Met Ala Val Thr Cys Ala Gln Pro Thr Asp Val Val Lys Val Arg Phe
    130                 135                 140

cag gcc agc ata cac ctc ggg cca tcc agg agc gac aga aaa tac agc      480
Gln Ala Ser Ile His Leu Gly Pro Ser Arg Ser Asp Arg Lys Tyr Ser
145                 150                 155                 160

ggg act atg gac gcc tac aga acc atc gcc agg gag gaa gga gtc agg      528
Gly Thr Met Asp Ala Tyr Arg Thr Ile Ala Arg Glu Glu Gly Val Arg
                165                 170                 175

ggc ctg tgg aaa gga act ttg ccc aac atc atg agg aat gct atc gtc      576
Gly Leu Trp Lys Gly Thr Leu Pro Asn Ile Met Arg Asn Ala Ile Val
            180                 185                 190

aac tgt gct gag gtg gtg acc tac gac atc ctc aag gag aag ctg ctg      624
Asn Cys Ala Glu Val Val Thr Tyr Asp Ile Leu Lys Glu Lys Leu Leu
        195                 200                 205

gac tac cac ctg ctc act gac aac ttc ccc tgc cac ttt gtc tct gcc      672
Asp Tyr His Leu Leu Thr Asp Asn Phe Pro Cys His Phe Val Ser Ala
    210                 215                 220

ttt gga gcc ggc ttc tgt gcc aca gtg gtg gcc tcc ccg gtg gac gtg      720
Phe Gly Ala Gly Phe Cys Ala Thr Val Val Ala Ser Pro Val Asp Val
225                 230                 235                 240

gtg aag acc cgg tat atg aac tca cct cca ggc cag tac ttc agc ccc      768
Val Lys Thr Arg Tyr Met Asn Ser Pro Pro Gly Gln Tyr Phe Ser Pro
                245                 250                 255

ctc gac tgt atg ata aag atg gtg gcc cag gag ggc ccc aca gcc ttc      816
Leu Asp Cys Met Ile Lys Met Val Ala Gln Glu Gly Pro Thr Ala Phe

260                 265                 270

tac aag ggg tga                                                   828
Tyr Lys Gly
        275

<210>  14
<211>  275
<212>  PRT
<213>  Homo sapiens

<400>  14

Met Val Gly Leu Lys Pro Ser Asp Val Pro Pro Thr Met Ala Val Lys
1               5                   10                  15

Phe Leu Gly Ala Gly Thr Ala Ala Cys Phe Ala Asp Leu Val Thr Phe
            20                  25                  30

Pro Leu Asp Thr Ala Lys Val Arg Leu Gln Ile Gln Gly Glu Asn Gln
        35                  40                  45

Ala Val Gln Thr Ala Arg Leu Val Gln Tyr Arg Gly Val Leu Gly Thr
    50                  55                  60

Ile Leu Thr Met Val Arg Thr Glu Gly Pro Cys Ser Pro Tyr Asn Gly
65                  70                  75                  80

Leu Val Ala Gly Leu Gln Arg Gln Met Ser Phe Ala Ser Ile Arg Ile
                85                  90                  95

Gly Leu Tyr Asp Ser Val Lys Gln Val Tyr Thr Pro Lys Gly Ala Asp
            100                 105                 110

Asn Ser Ser Leu Thr Thr Arg Ile Leu Ala Gly Cys Thr Thr Gly Ala
        115                 120                 125

Met Ala Val Thr Cys Ala Gln Pro Thr Asp Val Val Lys Val Arg Phe
    130                 135                 140

Gln Ala Ser Ile His Leu Gly Pro Ser Arg Ser Asp Arg Lys Tyr Ser
145                 150                 155                 160

Gly Thr Met Asp Ala Tyr Arg Thr Ile Ala Arg Glu Glu Gly Val Arg
                165                 170                 175

Gly Leu Trp Lys Gly Thr Leu Pro Asn Ile Met Arg Asn Ala Ile Val
            180                 185                 190

Asn Cys Ala Glu Val Val Thr Tyr Asp Ile Leu Lys Glu Lys Leu Leu
        195                 200                 205

Asp Tyr His Leu Leu Thr Asp Asn Phe Pro Cys His Phe Val Ser Ala
    210                 215                 220

Phe Gly Ala Gly Phe Cys Ala Thr Val Val Ala Ser Pro Val Asp Val
225                 230                 235                 240

Val Lys Thr Arg Tyr Met Asn Ser Pro Pro Gly Gln Tyr Phe Ser Pro
                245                 250                 255

Leu Asp Cys Met Ile Lys Met Val Ala Gln Glu Gly Pro Thr Ala Phe
            260                 265                 270

Tyr Lys Gly
        275

<210>  15
<211>  22
<212>  DNA
<213>  Artificial Sequence

<220>
<223>  UBC forward primer

<400>  15
ggcaaagatc caagataagg aa                                                22

<210>  16
<211>  20
<212>  DNA
<213>  Artificial Sequence

<220>
<223>  UBC reverse primer

<400>  16
ggaccaagtg cagagtggac                                                   20

<210>  17
<211>  19
<212>  DNA
<213>  Artificial Sequence

<220>
<223>  GAPDH forward primer

<400>  17
agccacatcg ctcagacac                                                    19

<210>  18
<211>  19
<212>  DNA
<213>  Artificial Sequence

<220>
<223>  GAPDH reverse primer

<400>  18
gcccaatacg accaaatcc                                                    19


<210>  19
<211>  18
<212>  DNA
<213>  Artificial Sequence

<220>
<223>  ACTB forward primer

<400>  19
attggcaatg agcggttc                                                     18


<210>  20
<211>  21
<212>  DNA
<213>  Artificial Sequence

<220>
<223>  ACTB reverse primer

<400>  20
tgaaggtagt ttcgtggatg c                                                 21


<210>  21
<211>  19
<212>  DNA
<213>  Artificial Sequence

<220>
<223>  B2M forward primer

<400>  21
ttctggcctg gaggctatc                                                    19


<210>  22
<211>  23
<212>  DNA
<213>  Artificial Sequence

<220>
<223>  B2M reverse primer

<400>  22
tcaggaaatt tgactttcca ttc                                               23


<210>  23
<211>  20
<212>  DNA
<213>  Artificial Sequence

<220>
<223>  18s forward primer

<400>  23
ggagagggag cctgagaaac                                                   20


<210>  24

<211>  19
<212>  DNA
<213>  Artificial Sequence

<220>
<223>  18s reverse primer

<400>  24
tcgggagtgg gtaatttgc                                                    19

## Description

The present invention is concerned with compounds modulating Wnt/β-catenin signaling in cancer cells. Furthermore, contemplated by the present invention are therapeutic and diagnostic methods and means for identifying such a compound, as well as the use of said compound for the manufacture of a drug against cancer. The present invention also encompasses therapeutic and diagnostic methods and means for diagnosing cancer in the subject and for determining whether a subject is in need of an anti-cancer therapy, as well as uses thereof. It is also referred to a device and kit for carrying out the methods of the present invention.

The Wingless-type (Wnt)/β-catenin signaling plays important roles in multiple biological processes, including embryonic development, tissue homeostasis and carcinogenesis, and is conserved across metazoans (see Logan and Nusse 2004, Annu Rev Cell Dev Biol 20: 781-810). In model organisms and man, secretion of Wnts, a class of 19 hydrophobic proteins, requires the multi-pass transmembrane protein eveness-interupted/Wntless (Evi/Wls; see Bänziger et al. 2006, Cell 125: 509-522; Bartscherer et al. 2006, Cell 125: 523-533; Adell et al. 2009, Development 136: 905-910; Augustin et al. 2012, EMBO Mol Med 4: 38-51). Upon binding to receptors of the Frizzled (FZD) family and their co-receptors lipoprotein receptor related proteins 5 and 6 (Lrp5/6), Wnt proteins activate canonical and non-canonical signaling cascades. Canonical signaling prevents the constitutive degradation of cytosolic β-catenin, leading to its accumulation and translocation to the nucleus, where it acts as a cofactor for transcription factors of the T-cell specific (TCF) family. In the absence of signaling, degradation of β-catenin is initiated by adenomatous polyposis coli (APC), glycogen synthase kinase β (GSK3β) and axis inhibition protein-1 (Axin1), which form the 'destruction complex'. APC recruits casein kinase 1 (CK1α) and GSK3β which phosphorylate β-catenin and mark it for ubiquitination by the SKP1-Cullin 1-F-box (SCFβKP1-) E3 ubiquitin ligase complex, triggering its proteasomal degradation (see Klaus and Birchmeier 2008, Nat Rev Cancer 8: 387-398). Wnt signaling can be regulated by, in particular, secreted antagonists of Wnt signaling such as proteins of the Dickkopf family (DKK1-4) and secreted frizzled related proteins (sFRPs; see Buechling and Boutros 2011, Curr. Top. Dev. Biol. 97: 21-53). Thus, it is to be understood that said secreted Wnt antagonists may also modulate Wnt signaling, by, e.g. inhibition and/or activation of Wnt signaling. Examples for such modulating secreted Wnt antagonists are sFRPs. In presence of canonical Wnt ligands, phosphorylation of β-catenin by GSK3β is reduced and dephosphorylation by serine/threonine phosphatases PP1 and PP2a relieves β-catenin from constant degradation, allowing it to accumulate in the cytoplasm. β-catenin can now enter the nucleus where it displaces the transcriptional repressor transducin-like enhancer of split (TLE) (Groucho in drosophila) from T-cell specific transcription factors (TCF) such as TCF/LEF1 or TCF4 (TCF7L2). This turns TCFs into transcriptional activators resulting in the expression of target genes, for instance cyclin D1 (CCND1), Axin2 and cMyc. This process is aided by co-activators, e.g. Bcl9, and leads to recruitment of histone acetylases (HATs) such as CBP/P300. Hence, APC and β-catenin constitute opposing factors in there rgulation of canonical Wnt/β-catenin target genes: APC blocks Wnt target gene expression, β-catenin expression of these genes. Accordingly depletion of APC or β-catenin by e.g. RNAi causes the opposite effects. Knocksown of APC strongly promotes expression of Wnt/β-catenin target genes whereas knockdown of β-catenin strongly reduces their expression. In addition, numerous other signaling pathways can interact with the Wnt/β-catenin signaling cascade at different levels. Unexpectedly, secreted Wnt antagonists were found to attenuate Wnt/β-catenin signaling in colorectal cancer cell lines carrying APC mutations (see Suzuki et al. 2004, Nat. Genet. 36: 417-422; Baehs et al. 2009, Cancer Lett. 276: 152-159). The canonical Wnt signaling pathway is of particular importance for the development of colorectal cancer, which is one of the third most commonly diagnosed cancers worldwide with more than one million new cases each year. Colorectal cancer, also referred to as colon carcinoma or bowel cancer, is characterized by an uncontrolled cell growth in the colon, the rectum or in the appendix. Typically, colorectal cancer starts in the lining of the bowel, grows into the muscle layers underneath, and then through the bowel wall. Classic symptoms and signs of colorectal cancer are worsening constipation, blood in the stool, change in bowel habit, weight loss, fever, loss of appetite, and nausea or vomiting.

More than 90% of sporadic colorectal carcinomas are induced by loss-of function mutations in the adenomatosis polyposis coli (APC) gene, which encodes a scaffolding protein mediating constitutive destruction of the β-catenin transcriptional coactivator in the absence of Wnt ligand (see Tang et al. 2009, Proc Natl Acad Sci U S A. 105(28): 9697-9702). Approximately 10% of sporadic colorectal carcinomas harbor mutations in β-catenin (see Morin et al. 1997, Science 275: 1787-1790; Sparks et al. 1998, Cancer Res 58: 1130-1134). Thus, loss-of-function mutations in APC or activating mutations in β-catenin constitute the earliest events of colon cancer development. These mutations lead to excessive Wnt/β-catenin signaling, wherein said Wnt/β-catenin signaling regulates proliferation and differentiation of intestinal stem and progenitor cells, and induces hyperplasia in intestinal crypts (see Humphries and Wright 2008, Nat Rev Cancer 8: 415-424; Reya and Clevers 2005, Nature 434: 843-850). Furthermore, Wnt pathway activity is not only required in the initiating phase of colon tumor development, but also for the maintenance of colon cancer cells during tumor progression and the formation of metastases (see Scholer-Dahirel et al. 2011, Proc Natl Acad Sci U S A 108: 17135-17140). This makes the Wnt pathway an interesting target for therapeutic intervention in colon cancer patients (see Barker and Clevers 2006, Nat Rev Drug Discov 5, 997-1014).

As described above, the Wnt/β-catenin signaling pathway has been causally linked to onset and progression of colon cancer, however, targeted therapeutics are still missing.

Thus, the technical problem underlying the present invention can be seen as the provision of means and methods for reliably and efficiently identifying a compound modulating Wnt/β-catenin signaling to comply with the aforementioned needs. The technical problem is solved by the embodiments characterized in the claims and herein below.

Accordingly, the present invention relates to a method for identifying a compound modulating Wnt/β-catenin signaling comprising the steps of:
a) determining the amounts of at least the biomarkers NKD1 and C10orf54 in a sample comprising cells, wherein said cells have been brought into contact with a compound suspected to be a modulator of Wnt/β-catenin signaling; and
b) comparing the amount of the biomarker NKD1 determined in step a) to a reference for NKD1 and comparing the amount of the biomarker C10orf54 determined in step a) to a reference for C10orf54, whereby a compound modulating Wnt/β-catenin signaling is identified.

The method of the present invention, preferably, is an in vitro method. Moreover, it may comprise steps in addition to those explicitly mentioned above. For example, further steps may relate to sample pre-treatments or evaluation of the results obtained by the method. The method may be carried out manually or assisted by automation. Preferably, step a) and/or b) may in total or in part be assisted by automation, e.g., by a suitable robotic and sensory equipment for the determination in step a) or a computer-implemented comparison and/or diagnosis based on said comparison in step b).

The term "Wnt/β-catenin signaling" as used herein refers to signaling through the cellular signaling pathway well known to the skilled artisan (see, e.g., MacDonald 2009, Developmental Cell 17.1: 653-71). The term "Wnt/β-catenin signaling gene" as used herein, refers to a polynucleotide, preferably DNA or RNA, that encodes a polypeptide or RNA involved in the Wnt/β-catenin signaling pathway. Preferably, the term relates any gene affected, directly or indirectly, by a modulation of Wnt/β-catenin signaling, including genes mediating Wnt/β-catenin signaling and genes affected by Wnt/β-catenin signaling (Wnt/β-catenin target genes). More preferably, the term relates to genes mediating Wnt/β-catenin signaling. Preferably, the term "Wnt/β-catenin signaling protein", as used herein, refers to a polypeptide encoded by the aforementioned Wnt/β-catenin signaling gene. Preferably, said Wnt/β-catenin signaling protein is selected from a Wnt protein encoded by any one gene of WNT1, WNT2, WNT2B, WNT3, WNT3A, WNT4, WNT5A, WNT5B, WNT6, WNT7A, WNT7B, WNT8A, WNT8B, WNT9A, WNT9B, WNT10A, WNT10B, WNT11, or WNT16, or from a WNT receptor being any one of FZD1 to FZD10, Axin, APC, ß-catenin, GSK-3, and a TCF/LEF transcription factor such as LEF-2. Further known polypeptides and/or regulators of the Wnt/β-catenin signaling pathway in humans are shown in Table 9. Preferred compounds modulating the Wnt/β-catenin signaling pathway are described elsewhere herein.

The term "modulating" as used herein refers to blocking, de novo activating, enhancing or reducing Wnt/β-catenin signaling. It is to be understood that, preferably, said modulation refers to statistically significant changes in signaling activity. Whether modulation is statistically significant, or not, can be determined by the skilled artisan without further ado, preferably, by applying standard statistics such as, e.g., the determination of confidence intervals, p-value determination, Student's t-test, Mann-Whitney test etc. Preferred confidence intervals are at least 90%, at least 95%, at least 97%, at least 98% or at least 99%. The p-values are, preferably, 0.1, 0.05, 0.01, 0.005, or 0.0001. Modulation can, preferably, be conferred by alteration of protein-protein interactions, isomerization, proteolytic processing, intracellular translocation, ubiquitination, protein abundance or activity and/or RNA abundance.

The term "compound" as used herein encompasses any molecule, i.e. any organic or inorganic chemical substance. The organic molecule may belong to any known chemical class of molecules. Preferably, an organic molecule is a lipid, a fatty acid, a purine, a pyrimidine, an alkaloid, an amino acid, a peptide, an antibody, an aptamere, a protein, a biogenic amine, an isoprenoid, a steroid, or a single- or double- stranded nucleic acid. The compound may be a naturally occurring compound which can be obtained from a biological or environmental source or an artificially synthesized compound. In accordance with the present invention, a compound shall be used to modulate the Wnt/ß-catenin signaling. Modulation can be conferred by various effects elicited by a modulator compound as set forth above.

As used herein, a "compound suspected to be a modulator of Wnt/β-catenin signaling" is a compound according to the present invention for which it cannot be excluded from the state of the art that it has activity in modulating Wnt/β-catenin signaling. Preferably, the compound suspected to be a modulator of Wnt/β-catenin signaling belongs to the same chemical family, gene class, gene family, protein class, or protein family as a compound known as a modulator of Wnt/β-catenin signaling from the state of the art. More preferably, the compound suspected to be a modulator of Wnt/β-catenin signaling has a primary structure at least 80% identical, at least 85% identical, at least 90% identical, at least 91% identical, at least 92% identical, at least 93% identical, at least 94% identical, at least 95% identical, at least 96% identical, at least 97% identical, at least 98% identical, at least 99% identical, at least 99.5% identical, at least 99.75% identical, at least 99.9% identical, or at least 99.95% identical to a compound known as a modulator of Wnt/β-catenin signaling from the state of the art. Preferred examples of compounds known as a modulator of Wnt/β-catenin signaling from the state of the art are IWR1, IWR2, IWP1, IWP2, C59-Wnt, XAV 939, ICG-001, BIO, CHIR and LiCL (see Hernández et al. 2009, Mini Rev Med Chem 9: 1024-1029; Huang et al. 2009, Nature 461: 614-620; Lu et al. 2009, Bioorg. Med. Chem. Lett 19: 3825-3827; Dodge et al. 2012, J. Biol. Chem. 287: 23246-23254). Preferably, the compound suspected to be a modulator of Wnt/β-catenin signaling is a compound for which modulation of Wnt/β-catenin signaling can be predicted. More preferably, the compound suspected to be a modulator of Wnt/β-catenin signaling is selected from the list consisting of antisense RNA, siRNA, micro RNA, ribozymes, oligonucleotides, peptides, PNAs, proteins, antibodies, aptamers, and organic small molecule compounds.

Preferably, a modulating compound can elicit its modulating effect directly or indirectly. The directly modulating compound affects at least one Wnt/ß-catenin signaling molecule in the Wnt/ß-catenin signaling pathway. Direct modulation can, preferably, occur by modulating the biological activity of a Wnt/ß-catenin signaling protein, e.g. by binding to said protein. The indirectly modulating compounds affect regulators which govern the abundance of a Wnt/ß-catenin signaling protein. The abundance of a Wnt/ß-catenin signaling protein can, preferably, be modulated by altering the gene expression of the Wnt/ß-catenin signaling gene, e.g., preferably, the abundance of the RNA transcripts, the translation of said transcripts into protein, the stability of said Wnt/ß-catenin signaling protein, and the like. Suitable regulators governing the abundance of a Wnt/ß-catenin signaling protein which are preferably envisaged in accordance with the present invention are, e.g., transcription factors governing Wnt gene expression. The indirect modulation may also occur via modulating the biological activity or the abundance of a regulator of a Wnt/ß-catenin signaling protein and/or of a regulator of a gene encoding a Wnt/ß-catenin signaling protein.

Compounds according to the present invention which are preferably envisaged for the direct modulation of the biological activity of a Wnt/ß-catenin signaling protein are antibodies, aptamers, or small molecules that specifically bind to the Wnt/ß-catenin signaling protein and, preferably, thereby modulate the biological activity thereof.

Compounds according to the present invention which are preferably envisaged for the indirect modulation of the biological activity of a Wnt/ß-catenin signaling protein are single- or doublestranded nucleic acids and, in particular, antisense RNAs, inhibitory oligonucleotides, PNAs, micro RNAs, siRNA, ribozymes or triple-helix forming agents or small molecules that specifically bind to the Wnt/ß-catenin signaling gene encoding said protein or RNA transcripts thereof and, preferably, thereby modulate the biological activity thereof. Similarly, compounds according to the present invention which are preferably envisaged for the indirect modulation of the biological activity of a regulator of the Wnt/ß-catenin signaling protein are single- or doublestranded nucleic acids and, in particular, antisense RNAs, oligonucleotides, micro RNAs, siRNA, ribozymes or triple-helix forming agents or small molecules that specifically bind to the gene encoding the regulator or RNA transcripts thereof and, preferably, thereby modulate the biological activity thereof.

An antisense RNA as referred to herein means a single-stranded RNA that is complementary to a messenger RNA (mRNA) strand transcribed within a cell. Antisense RNA can be introduced into a cell to inhibit translation of a complementary mRNA by base pairing to it and physically obstructing the translation machinery. Preferably, the Wnt/β-catenin signaling pathway is inhibited, e.g. by degradation of RNA transcribed from a Wnt/β-catenin signaling gene (target RNA) or by inhibition of translation of said target RNA. It is to be understood that inhibiting as used herein does not necessarily mean the complete inhibition of translation in all cases. Inhibition, preferably, reduces translation by at least 50%, at least 60%, at least 70%, at least 80%, at least 90%, at least 95%, at least 98%, or at least 99% as compared to the uninhibited translation level in a reference. Methods relating to the use of antisense RNA to inhibit translation in animals, including mammals, are known in the art (see, e.g. Weiss 1999, Cell. Mol. Life Sci. 55: 334-358).

RNA interference (RNAi) as used herein refers to sequence-specific, post-transcriptional gene silencing of a selected target gene. The RNAi in the context of the present invention, preferably, specifically silences the expression of a Wnt/β-catenin signaling gene. It is to be understood that silencing as used herein does not necessarily mean the complete abolishment of gene expression in all cases. RNAi, preferably, silences gene expression by at least 50%, at least 60%, at least 70%, at least 80%, at least 90%, at least 95%, at least 98%, or at least 99% as compared to the expression level in a reference. RNAi requires in the cell the presence of a dsRNA, wherein said dsRNA is homologous in sequence to the target RNAs. The term double stranded RNA (dsRNA) as used herein refers to RNA having a duplex structure comprising two complementary and anti-parallel nucleic acid strands. Said strands have the same or a different number of nucleotides. It is, however, also contemplated by the present invention that the dsRNA is formed between two sequence stretches on the same RNA molecule. Also preferably, RNAi can be used for therapeutic approaches to treat several types of cancers as described elsewhere herein, wherein said cancer is accompanied with a modulated expression and/or activity of at least one of the Wnt/β-catenin signaling genes of the present invention. For such therapeutic approaches, an expression construct for small interfering RNA (siRNA) is introduced into target cells of the subject suffering from modulated expression of a Wnt/β-catenin signaling gene. Accordingly, siRNA can be combined efficiently with other therapy approaches. Methods relating to the use of RNAi to silence genes in animals, including mammals, are known in the art (see, e.g., Hammond et al. 2001, Nature Rev. Genet. 2: 110-119; Bernstein et al. 2001, Nature 409: 363-366; WO 9932619; and Elbashir et al. 2001, Nature 411: 494-498). As used herein, RNAi of the present invention is of sufficient length and complementarity to stably interact with the target RNA, i.e. it comprises at least 15, at least 17, at least 19, at least 21, at least 22 nucleotides complementary to the target RNA. By stably interact is meant an interaction of the RNAi or its products produced by the cell with a target RNA, e.g., by forming hydrogen bonds with complementary nucleotides in the target RNA under physiological conditions. Not all nucleotides of said RNAi necessarily exhibit complete Watson-Crick base pairs in the interaction with the target RNA; the two RNA strands may be substantially complementary. Preferably, complementarity between the RNAi agent and the RNA target is 100%, but can be less if desired, preferably, is 91 %, 92%, 93%, 94%, 95%, 96%, 97%, 98%, or 99%. For example, 19 bases out of 21 bases can be base-paired. In some instances, where distinction between various allelic variants is desired, 100% complementarity to the Wnt/β-catenin signaling gene is required in order to effectively discern the target sequence from the other allelic sequence. When selecting between allelic targets, the choice of length is also an important factor because it is the other factor involved in the percent complementary and the ability to differentiate between allelic differences. It is known to the skilled artisan that two types of small RNA molecules, small interfering RNA (siRNA) and microRNA (miRNA) are suitable forRNA interference.

The term "siRNA", as used herein, refers to: a) a dsRNA consisting of at least 15, at least 17, at least 19, at least 21 consecutive nucleotides base-paired, i.e. forming hydrogen bonds with a complementary nucleotide, b) a siRNA molecule or a molecule comprising an siRNA molecule, or c) a polynucleotide encoding a) or b), wherein, preferably, said polynucleotide is operatively linked to an expression control sequence. The siRNA in the context of the present invention, preferably, specifically silences the expression of a Wnt/β-catenin signaling gene. It is to be understood that silencing as used herein does not necessarily mean the complete abolishment of gene expression in all cases, as described elsewhere herein. The siRNA is a single-stranded RNA molecule with a length, preferably, greater than or equal to 15 nucleotides and, preferably, a length of 15 to 49 nucleotides, more preferably 17 to 30 nucleotides, and most preferably 17, 18, 19, 20, 21, 22, 23, 24, 25, 26, 27, 28, 29, and 30 nucleotides. The function of the siRNA to inhibit expression of the Wnt/β-catenin signaling gene is modulated by said expression control sequence. Preferred expression control sequences can be regulated by exogenous stimuli, e.g. the tet operator, whose activity can be regulated by tetracycline, or heat inducible promoters. Alternatively or in addition, one or more expression control sequences can be used which allow tissue-specific expression of the siRNA.

The term "micro RNA" or "miRNA" as used herein refers to a) a pre-microRNA, i.e. miRNA comprising at least 30, at least 40, at least 50, at least 60, at least 70 nucleotides base-paired to a complementary sequence on the same miRNA molecule (stem), i.e. as a dsRNA, separated by a stretch of non-base-paired nucleotides (loop), b) a pre-microRNA, i.e. a dsRNA molecule comprising a stretch of at least 19, at least 20, at least 21, at least 22, at least 23, at least24, at least 25 base-paired nucleotides formed by nucleotides of the same RNA molecule (stem), separated by a loop, c) a microRNA (miRNA), i.e. a dsRNA comprising at least 15, at least 17, at least 18, at least 19, at least 21 nucleotides on each of the two separate RNA strands, or d) a polynucleic acid encoding a) or b), wherein, preferably, said polynucleic acid is operatively linked to an expression control sequence as specified above. The miRNA in the context of the present invention, preferably, specifically silences the expression of a Wnt/β-catenin signaling gene. However, any type of RNA is encompassed by the present method. It is to be understood that silencing as used herein does not necessarily mean the complete abolishment of gene expression in all cases, as described elsewhere herein.

As used herein, the term "ribozyme" refers to an RNA molecule specifically hybridizing to a target RNA molecule (e.g. a RNA molecule transcribed from a Wnt/β-catenin signaling gene) and catalysing the hydrolysis of one or more phosphodiester bonds in said target RNA molecule, causing the target RNA to be degraded by cellular enzymes. The ribozyme in the context of the present invention, preferably, specifically hybridizes to an RNA molecule transcribed from a Wnt/β-catenin signaling gene and catalyses the hydrolysis of said RNA molecule. RNA sequences showing suitable catalytic properties, like hammerhead ribozyme, hairpin ribozyme, or RNase P are known in the art (see, e.g. Doherty and Doudna 2001, Annu. Rev. Biophys. Biomol. Struct. 30: 457-475). Sequence specificity and, thus, target RNA specificity is accomplished by specific binding of the ribozyme to the target RNA by means of Watson-Crick base pairing of complementary, anti-parallel RNA strands. Methods of generating ribozymes directed against RNA sequences of interest are known in the art (see, for example, Citti and Rainaldi 2005, Curr. Gene Ther. 5(1): 11-24).

The term "peptide nucleic acid" or "PNA", as used herein, refers to an artificially synthesized polymer similar to DNA or RNA (see, e.g. Nielsen 1991, Science 254(5037): 1497-1500). The PNA in the context of the present invention, preferably, modulates the Wnt/β-catenin signaling pathway, e.g. by modulating gene expression of a Wnt/β-catenin signaling gene, as antisense therapeutic agent, or anticancer agent. More preferably, said PNA is an inhibitor of the Wnt/β-catenin signaling pathway. The PNA of the present invention is of sufficient length and binds complementary DNA or RNA, e.g., by forming hydrogen bonds with complementary nucleotides in the target DNA or RNA under physiological conditions. Not all PNAs necessarily exhibit complete Watson-Crick base pairs in the interaction with the target DNA or RNA. However, the preferred complementarity between the PNA and the DNA or RNA target is 100%, but can be less if desired, preferably, is 91%, 92%, 93%, 94%, 95%, 96%, 97%, 98%, or 99%. The PNA can be prepared by well known methods in the art.

The term "antibody" as used herein refers to a molecule from the subgroup of gamma globulin proteins, also referred to as the immunoglobulins (Ig). The antibody is, preferably, of any subtype, i.e. IgA, IgD, IgE, IgM or, more preferably, IgG. The antibody in the context of the present invention is, preferably, an antibody against a gene product encoded by a Wnt/β-catenin signaling gene. More preferably, said antibody is an inhibitor of the Wnt/β-catenin signaling pathway. Such an antibody can be prepared by well-known methods using a purified polypeptide or a suitable fragment derived therefrom as an antigen. A fragment suitable as an antigen can be identified by antigenicity determining algorithms well known in the art. Such fragments can be obtained either by proteolytic digestion from a gene product encoded by a Wnt/β-catenin signaling gene or can be synthetic peptides. Preferably, the antibody of the present invention is a monoclonal antibody, a polyclonal antibody, a single chain antibody, a human or humanized antibody, or primatized, chimerized or fragment thereof. Also comprised as antibody of the present invention is a bispecific or a trispecific antibody, a synthetic antibody, an antibody fragment, such as Fab, Fv, or scFv fragments etc., or a chemically modified derivative of any of these. An antibody of the present invention preferably binds specifically (i.e. does not cross react with other polypeptides or peptides) to one of the polypeptides of the invention. Specific binding can be tested by various well known techniques. An antibody or fragments thereof can be obtained by using methods well known in the art, e.g., described in Harlow and Lane "Antibodies, A Laboratory Manual", CSH Press, Cold Spring Harbor, 1988. A monoclonal antibody can be prepared by the techniques originally described in Köhler and Milstein 1975, Nature 256: 495, and Galfré 1981, Meth. Enzymol. 73: 3, which comprise the fusion of mouse or other rodent myeloma cells to spleen cells derived from immunized mammals.

The term "aptamer" as used herein refers to an oligonucleotide or a peptide specifically binding to a gene product of one of the Wnt/β-catenin signaling genes of the present invention. The aptamer in the context of the present invention, preferably, modifies the activity and/or stability of said gene product, more preferably, is an inhibitor of the Wnt/β-catenin signaling pathway. Preferably, said aptamer is an RNA or a DNA aptamer comprising an RNA or DNA molecule that is able to specifically bind to the three-dimensional surface of a polypeptide and to inhibit the function of said polypeptide. Said RNA or DNA aptamer can be obtained, e.g. by in vitro selection, e.g. systematic evolution of ligands by exponential enrichment (SELEX). Methods relating to the development and use of RNA and DNA aptamers are known in the art (see, for example, Ulrich 2006, Handb. Exp. Pharmacol. 173: 305-326 and Ulrich 2005, Med. Chem. 1(2): 199-208). Preferably, said aptamer is a peptide aptamer comprising short variable peptide domains, wherein said domains are attached at both ends to a protein scaffold. Said peptide aptamer, preferably, comprises sequences of 8-80 amino acids, more preferably 10-50 amino acids, and most preferably 15-30 amino acids. Said amino acid sequences can, e.g. be isolated from randomized peptide expression libraries in a suitable host system like baker's yeast (see, for example, Klevenz et al. 2002, Cell. Mol. Life Sci. 59: 1993-1998). Said peptide aptamer, preferably, is used as free peptide. The peptide aptamer of this invention, preferably, comprises chemically modified amino acids, e.g., amino acids which are biotinylated, or are coupled to fluorophores, such as fluorescein, or Cy 3, are conformationally restricted, e.g. by disulfide bridging or by stapling (Walensky 2004, Science 305(5689): 1466-1470), or are linked to cell penetration peptides or protein transduction domains (Snyder 2004, Pharm Res 21(3): 389-393). Such modifications can improve the biological properties of the peptide aptamers, e.g., cell penetration, binding, stability, or may be used as detection labels. The peptide aptamer of the present invention can be recombinantly manufactured or chemically synthesized. The peptide aptamer comprises further amino acids, wherein said amino acids may serve as a tag for purification or detection. Moreover, the peptide aptamer of the present invention can be comprised by a fusion polypeptide, wherein the fusion partner, e.g., serves as a scaffold, fixing the peptide aptamer in a defined conformation. The variant or modified peptide aptamers, preferably, retain the biological activity of the peptide aptamer, i.e. they are capable of specifically binding to a gene product of one of the Wnt/β-catenin signaling genes of the present invention.

The term "biomarker" is well known to the skilled person and relates to a substance, preferably a cellular constituent, more preferably a cellular macromolecule, the concentration or chemical state of which is used or usable as an indicator of a biological state. Preferably, the biomarker is NKD1, C10orf54, PSCA, CPNE5, RAET1G, or UCP3. According to the method of the present invention, the amounts of at least the biomarkers NKD1 and C10orf54 are determined in a sample. More preferably, the amount of one biomarker selected from the group consisting of PSCA, CPNE5 and RAET1G is determined in a sample in addition to NKD1 and C10orf54. Even more preferably, the amounts of two biomarkers selected from the group consisting of PSCA, CPNE5 and RAET1G are determined in a sample in addition to NKD1 and C10orf54. Most preferably, the amounts of the biomarkers PSCA, CPNE5 and RAET1G are determined in a sample in addition to NKD1 and C10orf54. Preferably, the amount of the biomarker UCP3 is determined in a sample additionally to the biomarkers as described above.

The term "NKD1" as used herein refers to the Naked1 gene (see NCBI Gene ID: 85407, RefSeq ID: NM_033119, or NCBI Reference Sequence: NM_033119.4) and/or gene products encoded by said gene. The nucleic acid sequence, in particular the coding sequence (CDS), of NKD1 is shown in SEQ ID NO: 1, the encoded amino acid sequence is shown in SEQ ID NO: 2. NKD1 is known in the art to act as a negative regulator of the Wnt signaling pathway and interacts with the cytoplasmic disheveled protein 1 (DVL1; see Van Raay et al. 2007, Dev. Biol. 309, 151-168; Van Raay et al. 2011, PLoS ONE 6, e18650).

The term "C10orf54" as used herein refers to the chromosome 10 open reading frame 54 gene (see NCBI Gene ID: 64115, RefSeq ID: NM_022153, or NCBI Reference Sequence: NM_022153.1) and/or gene products encoded by said gene. The nucleic acid sequence, in particular the CDS, of C10orf54 is shown in SEQ ID NO: 3, the encoded amino acid sequence is shown in SEQ ID NO: 4. C10orf54 has been linked to the regulation of matrix metalloproteinases and invasiveness of tumours (see Sakr et al. 2010, Cancer Sci. 101, 2368-2374).

The term "PSCA" as used herein refers to the prostate stem cell antigen (see NCBI Gene ID: 8000, RefSeq ID: NM_005672, or NCBI Reference Sequence: NM_005672.4) and/or gene products encoded by said gene. The nucleic acid sequence, in particular the CDS, of PSCA is shown in SEQ ID NO: 5, the encoded amino acid sequence is shown in SEQ ID NO: 6. It is known from the said RefSeq entry, that PSCA encodes a glycosylphosphatidylinositol-anchored cell membrane glycoprotein. PSCA is highly expressed in the prostate and is also expressed in the bladder, the placenta, the colon, the kidney, and the stomach. PSCA is up-regulated in a large proportion of prostate cancers and is also detected in cancers of the bladder and pancreas. It includes a polymorphism that results in translation starting from an upstream start codon in some individuals; this polymorphism is thought to be associated with a risk for certain gastric and bladder cancers. Alternative splicing results in multiple transcript variants.

The term "CPNE5" as used herein refers to the copine V gene (see NCBI Gene ID: 57699, RefSeq ID: NM_020939, or NCBI Reference Sequence: NM_020939.1) and/or gene products encoded by said gene. The nucleic acid sequence, in particular the CDS, of CPNE5 is shown in SEQ ID NO: 7, the encoded amino acid sequence is shown in SEQ ID NO: 8. It is known from the said RefSeq entry, that CPNE5 is one of several genes that encode a calcium-dependent protein containing two N-terminal type II C2 domains and an integrin A domain-like sequence in the C-terminus. It is known that calcium-dependent membrane-binding proteins may regulate molecular events at the interface of the cell membrane and cytoplasm. Sequence analysis identified multiple alternatively spliced transcript variants but their full-length natures could not be determined.

The term "RAET1G" as used herein refers to the retinoic acid early transcript 1G gene (see NCBI Gene ID: 353091, RefSeq ID: NM_001001788, or NCBI Reference Sequence: NM_001001788.2) and/or gene products encoded by said gene. The nucleic acid sequence, in particular the CDS, of RAET1G is shown in SEQ ID NO: 9, the encoded amino acid sequence is shown in SEQ ID NO: 10. It is known from the said RefSeq entry, that members of the RAET1 family, such as RAET1G, are major histocompatibility complex (MHC) class I-related genes located within a 180-kb cluster on chromosome 6q24.2-q25.3. RAET1 proteins contain MHC class I-like alpha-1 and alpha-2 domains. RAET1E (MIM 609243) and RAET1G differ from the other RAET1 proteins (e.g., RAET1I or ULBP1; MIM 605697) in that they have type I membrane-spanning sequences at their C-termini rather than glycosylphosphatidylinositol anchor sequences (see Radosavljevic et al. 2002, Genomics 79(1): 114-23).

The term "UCP3" as used herein refers to the gene uncoupling protein 3 (mitochondrial, proton carrier; see NCBI Gene ID: 7352) and/or gene products encoded by said gene. In the context of the present invention two different isoforms of the gene UCP3 are used: the isoform UCP3L which is encoded by transcript variant long (see RefSeq ID: NM_003356 or NCBI Reference Sequence: NM_003356.3) and the isoform UCP3S which is encoded by transcript variant short (see RefSeq ID: NM_022803 or NCBI Reference Sequence: NM_022803.2). The nucleic acid sequence, in particular the CDS, of UCP3L is shown in SEQ ID NO: 11, the encoded amino acid sequence is shown in SEQ ID NO: 12. The nucleic acid sequence, in particular the CDS, of UCP3S is shown in SEQ ID NO: 13, the encoded translated amino acid sequence is shown in SEQ ID NO: 14. Mitochondrial uncoupling proteins (UCP) belong to the family of mitochondrial anion carrier proteins (MACP), acting by separating oxidative phosphorylation from ATP synthesis with energy dissipated as heat (mitochondrial proton leak). They facilitate the transfer of ions between inner to the outer mitochondrial membrane and can also reduce the mitochondrial membrane potential in mammalian cells. The different UCPs have tissue-specific expression; this gene is primarily expressed in skeletal muscle. This gene's protein product is postulated to protect mitochondria against lipid-induced oxidative stress. Expression levels of UCPs increase when fatty acid supplies to mitochondria exceed their oxidation capacity and the protein enables the export of fatty acids from mitochondria. UCPs contain the three solcar protein domains typically found in MACPs. Two splice variants have been found for this gene.

The term "determining" as used herein refers to measuring the amount or concentration of at least one biomarker in a sample, preferably semi-quantitatively or quantitatively. Such a biomarker is an indicator of a biological state. Preferred biomarkers are defined elsewhere herein. Measuring can be done directly or indirectly. Direct measuring relates to measuring the amount or concentration of said biomarker based on a signal, wherein said signal is obtained from said biomarker itself and wherein the intensity of the signal directly correlates with the number of molecules of said biomarker present in the sample. Such a signal can be obtained, e.g., by measuring an intensity value of a specific physical or chemical property of said biomarker. Indirect measuring includes measuring of a signal obtained from a secondary component, i.e., a component not being said biomarker itself, or a biological readout system, e.g., measurable cellular responses, ligands, labels, or enzymatic reaction products. Preferably, measuring is performed on a processed sample, said processing comprising extraction of polynucleotides or polypeptides from the sample. It is, however, also envisaged by the present invention that the gene product is determined by qRT-PCR, hybridization techniques and massive parallel sequencing. As used herein, a qRT-PCR is well known to the skilled artisan and refers to a method where messenger RNA is repeatedly amplified and detected via fluorescent signals. Thus, a qRT-PCR allows to relatively and absolutely quantify a gene product. Hybridization based techniques as used herein are well known to the skilled artisan and, preferably, comprise NanoString and Padlock systems that allow direct detection and quantification of mRNA (see Geiss et al. 2008, Nat. Biotechnol. 26: 317-325; Stougaard et al. 2011, Integr Biol (Camb) 3: 982-992). Massive parallel sequencing techniques are also well known to the skilled artisan and can be applied to detect and quantify gene products by sequencing of RNA.

The term "amount" as used herein refers to the absolute amount of at least one biomarker, the relative amount or concentration of the said biomarker as well as any value or parameter correlating thereto. Such values or parameters comprise intensity signal values from all specific physical and/or chemical properties including biochemical properties of said biomarker. Such properties include, e.g., molecular weight, viscosity, density, electrical charge, spin, optical activity, colour, fluorescence, chemoluminescence, elementary composition, chemical structure, capability to react with other compounds, capability to elicit a response in a biological read out system (e.g., induction of a reporter gene) and the like. Values for said properties may serve as characteristic features and can be determined by techniques well known in the art. Moreover, encompassed are all values or parameters obtained by direct measurements, e.g. intensity values in mass spectra or NMR spectra, and/or indirect measurements specified elsewhere in this specification, e.g., biological readout systems in response to said biomarker or intensity signals obtained from specifically bound ligands. Furthermore, the characteristic feature is any feature derived from the values of the physical and/or chemical properties of a biomarker by standard operations, e.g., mathematical calculations such as multiplication, division or logarithmic calculus. Most preferably, the at least one characteristic feature allows the determination and/or chemical identification of the said at least one biomarker and its amount. Accordingly, the characteristic value, preferably, also comprises information relating to the abundance of the biomarker from which the characteristic value is derived. For example, a characteristic value of a biomarker may be a peak in a mass spectrum. Such a peak contains characteristic information of the biomarker, i.e. the mass-to-charge ratio (m/z) information, as well as an intensity value being related to the abundance of the said biomarker (i.e. its amount) in the sample.

Moreover, the analysis step may, preferably, be preceded by a compound separation and / or enrichment step. Preferably, said compound separation and / or enrichment step yields a time resolved separation and / or enrichment of the compound comprised by the sample. Suitable techniques for separation and enrichment to be used preferably in accordance with the present invention, therefore, include all chromatographic separation techniques, i.e. liquid chromatography (LC), high performance liquid chromatography (HPLC), gas chromatography (GC), or thin layer chromatography. These techniques are well known in the art and can be applied by the person skilled in the art without further ado. The method of the present invention shall be, preferably, assisted by automation. For example, sample processing or pretreatment can be automated by robotics. Data processing and comparison is, preferably, assisted by suitable computer programs and databases. Automation as described herein before allows using the method of the present invention in high-throughput approaches.

Preferably, the biomarker is determined by a specific chemical or biological assay. Said assay shall comprise means allowing to specifically detect at least one biomarker in the sample. Preferably, said means are capable of specifically recognizing the chemical structure of the biomarker or are capable of specifically identifying the biomarker based on its capability to react with other compounds or its capability to elicit a response in a biological read out system (e.g., induction of a reporter gene). Means capable of specifically recognizing the chemical structure of a biomarker are, preferably, diagnostic antibodies or other proteins, wherein said proteins specifically interact with chemical structures, such as receptors or enzymes. Specific diagnostic antibodies, for instance, may be obtained using the biomarker as antigen by methods well known in the art. Diagnostic antibodies as referred to herein include both polyclonal and monoclonal antibodies, as well as fragments thereof, such as Fv, Fab and F(ab)2 fragments that are capable of binding the antigen or hapten. The term diagnostic antibody according to the present invention also includes humanized hybrid antibodies wherein amino acid sequences of a non-human donor antibody exhibiting a desired antigen-specificity are combined with sequences of a human acceptor antibody. The sequences of the donor antibody used will usually include at least the antigen-binding amino acid residues of the donor but may comprise other structurally and/or functionally relevant amino acid residues of the donor antibody as well. Such hybrids can be prepared by several methods well known in the art. Moreover, encompassed are single chain antibodies, which are well known to the skilled artisan. Moreover, said antibodies may be used as a basis to generate oligopeptides specifically recognizing the biomarker. These oligopeptides shall, for example, comprise the enzyme's binding domains or pockets for the said biomarker. Suitable antibody and/or enzyme based assays may be RIA (radioimmunoassay), ELSA (enzyme-linked immunosorbent assay), sandwich enzyme immune tests, electro-chemiluminescence sandwich immunoassays (ECLIA), dissociation-enhanced lanthanide fluoro immuno assay (DELFIA) or solid phase immune tests. Moreover, the biomarker may also be identified based on its capability to react with other compounds, i.e. by a specific chemical reaction. Further, the biomarker may be determined in a sample due to its capability to elicit a response in a biological read out system. The biological response shall be detected as read out indicating the presence and/or the amount of the biomarker comprised by the sample. The biological response may be, e.g., the induction of gene expression or a phenotypic response of a cell or a subject. Preferably, the biomarker is determined by qRT-PCR, hybridization techniques and massive parallel sequencing as described above herein.

Moreover, depending on the technique used for determining the amount of a biomarker, the analyte determined by the determination technique may differ with respect to its chemical nature from the biomarker found in the sample. Such analytes include derivatives of the biomarkers, wherein said derivatives of biomarkers are generated by the pretreatment processes used for the sample or by the determination technique as such. However, it is to be understood that the said derivative of the biomarkerrepresents the biomarker qualitatively or, preferably, quantitatively.

Preferably, the amount of a polynucleotide according to the present invention is determined by one of several methods well-known in the art. Quantification preferably is absolute, i.e. relating to a specific number of polynucleotides or, more preferably, relative, i.e. measured in arbitrary normalized units. Methods allowing for absolute or relative quantification are well known in the art, e.g., quantitative PCR methods are methods for relative quantification; if a calibration curve is incorporated in such an assay, the relative quantification can be used to obtain an absolute quantification. Other methods known are, e.g. nucleic acid sequence-based amplification (NASBA) or the Branched DNA Signal Amplification Assay method, preferably in combination with dot blot or luminex detection of amplified polynucleotides. Preferably, the polynucleotide amounts are normalized polynucleotide amounts, i.e. the polynucleotide amounts obtained are set into relation to at least one reference amplification product, thereby, preferably, setting the polynucleotide amounts into relation to the number of cells in the sample and/or the efficiency of polynucleotide amplification. Thus, preferably, the reference amplification product is a product obtained from a polynucleotide known to have a constant abundancy in each cell, i.e. a polynucleotide comprised in most, preferably all, cells of a sample in approximately the same amount. More preferably, the reference amplification product is amplified from a chromosomal or mitochondrial gene or from the mRNA of a housekeeping gene. A preferred housekeeping gene is selected from the group consisting of: UBC, GAPDH, ACTB, B2M, and 18s. The skilled artisan knows said housekeeping genes. It is to be understood that said housekeeping genes are used alone or in combination with each other.

In a preferred embodiment of the present invention the forward and reverse primer pairs specific for housekeeping genes are used as shown in Table 1. It is to be understood that also other primer pairs for the said housekeeping genes can be used. The skilled artisan known how to generate suitable forward and primer pair sequenes for housekeeping genes, e.g. by using different software tools. Table 2 shows the gene name and the NCBI gene ID of the said housekeeping genes.

**Table 1: Forward and reverse primer pairs of the housekeeping genes used:**

| **Gene** | **Forward Primer** | | **Reverse Primer** | |
|---|---|---|---|---|
| | **Sequence** | **SEQ ID NO:** | **Sequence** | **SEQ ID NO:** |
| UBC | GGCAAAGATCCAAGATAAGGAA | 15 | GGACCAAGTGCAGAGTGGAC | 16 |
| GAPDH | AGCCACATCGCTCAGACAC | 17 | GCCCAATACGACCAAATCC | 18 |
| ACTB | ATTGGCAATGAGCGGTTC | 19 | TGAAGGTAGTTTCGTGGATGC | 20 |
| B2M | TTCTGGCCTGGAGGCTATC | 21 | TCAGGAAATTTGACTTTCCATTC | 22 |
| 18s | GGAGAGGGAGCCTGAGAAAC | 23 | TCGGGAGTGGGTAATTTGC | 24 |

**Table 2: Gene name and NCBI gene ID of the housekeeping genes used:**

| Gene Symbol | Gene Name | NCBI Gene ID |
|---|---|---|
| UBC | Ubiquitin C | 7316 |
| GAPDH | glyceraldehyde-3-phosphate dehydrogenase | 2597 |
| ACTB | actin, beta | 60 |
| 18s (RNA18S5) | RNA, 18S ribosomal 5 | 100008588 |

The term "subject" as used herein encompasses animals, preferably mammals, amphibians, and ray-finned fishes, and, more preferably, mouse, rat, cat, dog, cattle, rabbit, Guinean pig, pig, Chinese hamster, monkey, xenopus, zebrafish, and humans. Preferably, xenopus and/or zebrafish are used for initial in vivo testing of compounds. The subject, preferably, is apparently healthy, e.g. not suffering from cancer. Typically, such a subject is apparently healthy with respect to cancer prior to the onset of signs or symptoms of cancer or any cancer-related medical event. It is understood that in case the cells of the present invention are intentionally brought into contact with a compound suspected to be a modulator of Wnt/β-catenin signaling, the subject, preferably, is not a human. It is, however, also envisaged that the method of the present invention is employed for identifying compounds present in the environment (e.g. pollutants) being modulators of Wnt/β-catenin signaling using e.g. an epidemiologic approach. In such cases, the subject may be a human.

The term "sample", as used herein, refers to a sample of cells obtained from cultured cells or from a subject. Preferably, the sample comprises isolated and/or separated cells or a cell lysate. Preferably, said cells are metazoan cells, more preferably mammalian cells; most preferably, the cells are selected from the group consisting of mouse, rat, feline, canine, bovine, rabbit, Guinea pig, pig, Chinese hamster, monkey, and human cells. Preferably, the sample is obtained from cultivated cells, more preferably from a cell line; even more preferably cells are cultivated cells of a cancer cell line. Most preferably, the cells are cells from a cell line selected from the group consisting of HCT116, DLD1, CACO-2, SW480, RKO, HT29, COLO320, HT55, SW48, and LoVo. Further preferred cells are cells from a cancer cell line found in Rowan et al. 2000, PNAS 97: 3352-3357.

It is, however, also envisaged by the present invention that the sample comprises cells obtained from a subject, e.g., from a body fluid, a tissue or an organ of said subject. Said cells obtained from a tissue or organ of a subject are, preferably, obtained from any tissue or organ by well known techniques, e.g., by biopsy or other surgical procedures. Said cells obtained from a body fluid of a subject are obtained by well-known techniques and include, preferably, blood. The sample is obtained by well-known techniques and includes, preferably, use of brushes, (cotton) swabs, spatula, rinse/wash fluids, punch biopsy devices, puncture of cavities with needles or surgical instrumentation. A tissue or organ sample is obtained from any tissue or organ by, e.g., biopsy or other surgical procedures. A sample of a body fluid is obtained by well-known techniques and includes, preferably, a sample of blood. Preferably, said sample of a subject is obtained from any tissue or organ of a subject.

The term "HCT116 cell line" as used herein refers to a continuous line of human colorectal carcinoma cells. Culturing said cell line is well known in the art (see, e.g., Rowan et al. 2000, PNAS 97: 3352-3357 and Chan et al. 2002, Proc Natl Acad Sci U S A 99: 8265-8270).

The term "DLD1 cell line" as used herein refers to a continuous line of human epithelial colorectal adenocarcinoma cells. Culturing said cell line is well known in the art (see, e.g., Rowan et al. 2000, PNAS 97: 3352-3357).

The term "CACO-2 cell line" as used herein refers to a continuous line of heterogeneous human epithelial colorectal adenocarcinoma cells. Culturing said cell line is well known in the art (see, e.g., Rowan et al. 2000, PNAS 97: 3352-3357).

The term "SW480 cell line" as used herein refers to a continuous line of human colorectal adenocarcinoma cells. Culturing said cell line is well known in the art (see, e.g., Rowan et al. 2000, PNAS 97: 3352-3357).

The term "RKO cell line" as used herein refers to a continuous line of human epithelial colon carcinoma cells. Culturing said cell line is well known in the art (see, e.g., Zhang et al. 2010, Chem. Biol. Interact. 185: 174-181).

The term "HT29 cell line" as used herein refers to a continuous line of human colorectal adenocarcinoma cells. Culturing said cell line is well known in the art (see, e.g., Rowan et al. 2000, PNAS 97: 3352-3357).

The term "COLO320 cell line" as used herein refers to a continuous line of human colorectal adenocarcinoma cells. Culturing said cell line is well known in the art (see, e.g., Rowan et al. 2000, PNAS 97: 3352-3357).

The term "LoVo cell line" as used herein refers to a continuous line of human colorectal adenocarcinoma cells. Culturing said cell line is well known in the art (see, e.g., Rowan et al. 2000, PNAS 97: 3352-3357).

The term "HT55 cell line" as used herein refers to a continuous line of human colorectal adenocarcinoma cells. Culturing said cell line is well known in the art (see, e.g., Rowan et al. 2000, PNAS 97: 3352-3357).

The term "SW48 cell line" as used herein refers to a continuous line of human colorectal adenocarcinoma cells. Culturing said cell line is well known in the art (see, e.g., Rowan et al. 2000, PNAS 97: 3352-3357).

The term "comparing" as used herein refers to comparing the determined amount for at least one biomarker as referred to herein to a corresponding reference. It is to be understood that comparing as used herein refers to any kind of comparison made between the amounts of the biomarker with that of the reference. The comparison referred to in step b) of the method of the present invention may be carried out manually or computer assisted. The amounts of the biomarker and the reference can be, e.g., compared to each other and the said comparison can be automatically carried out by a computer program executing an algorithm for the comparison. The computer program carrying out the said evaluation will provide the desired assessment in a suitable output format. For a computer assisted comparison, the determined amount of the biomarker is compared to the corresponding amount of a suitable reference stored in a database by a computer program. The computer program may further evaluate the result of the comparison, i.e. automatically provide the desired assessment in a suitable output format. Based on the comparison of the amount determined in step a) and the reference, it is possible to assess whether a compound is capable of modulating Wnt/β-catenin signaling, or not. Therefore, the reference is to be chosen so that either a difference or a similarity in the amounts of the biomarker and the reference allows identifying such a compound. Preferably, comparing refers to assessing whether the results of the determination described hereinabove in detail, i.e. the results of the qualitative or quantitative determination of the amount of a biomarker, are identical or similar to a reference or differ therefrom.

The term "reference", relates to an amount obtained, preferably, from a sample of cells, wherein said cells (i) have not been brought into contact with a compound suspected to modulate Wnt/β-catenin signaling, or (ii) have been brought into contact with a compound known to modulate Wnt/β-catenin signaling. Cells having not been brought into contact with a compound suspected to modulate Wnt/β-catenin signaling, are, e.g., cells with an unchanged Wnt/β-catenin signaling and, more preferably, apparently healthy cells. Cells having been brought into contact with a compound known to modulate Wnt/β-catenin signaling, are, e.g., cells with modulated Wnt/β-catenin signaling. The reference amount is determined as described herein above for the amount of the at least one biomarker. It is understood that, according to the present invention, the value determined for a given biomarker is compared to a corresponding reference. The term "corresponding reference" is understood by the skilled person and relates to an amount obtained for the same biomarker in a different, preferably control, sample, more preferably comprising cells of the same origin and/or kind as the cells in the sample. Preferably, the reference is calculated from the average or median for the relative or absolute amount for the at least one biomarker (average reference). How to calculate a suitable reference value, preferably, the average or median, is well known in the art.

Preferably, the reference is obtained from a sample of cells corresponding to the cells of step a) and wherein said cells have not been brought into contact with a compound suspected to modulate Wnt/β-catenin signaling. In accordance with the present invention said cells corresponding to the cells of step a), preferably, are cells of the same origin, more preferably, the same kind of cells or cells of the same physiological state, or, most preferably, the same cells as the cells in the sample. Thus, a compound modulating Wnt/β-catenin signaling can be identified based on the differences between the amounts of the at least one biomarker in a sample and the aforementioned reference, i.e. differences in the qualitative or quantitative composition with respect to the aforementioned biomarkers. The same applies if an average reference as specified above is used. The difference can be an increase in the absolute or relative amount of the biomarker (sometimes referred to as up-regulation of the biomarker; see also Examples) or a decrease in either of said amounts or the absence of a detectable amount of the biomarker (sometimes referred to as down-regulation of the biomarker; see also Examples). Preferably, the difference in the relative or absolute amount is significant, i.e. outside of the interval between 45th and 55th percentile, 40th and 60th percentile, 30th and 70th percentile, 20th and 80th percentile, 10th and 90th percentile, 5th and 95th percentile, 1st and 99th percentile of the reference amount. Preferably, an increased or decreased amount of the at least one biomarker in comparison to the reference obtained from a sample of cells having not been brought into contact with a compound suspected to modulate Wnt/β-catenin signaling or an average reference, is indicative for a compound modulating Wnt/β-catenin signaling.

Also preferably, the reference is obtained from a sample of cells, wherein said cells are corresponding to the cells of step a) and wherein said cells have been brought into contact with a compound known to modulate Wnt/β-catenin signaling. In accordance with the present invention said cells corresponding to the cells of step a), preferably, are cells of the same origin, more preferably, the same kind of cells or cells of the same physiological state, or, most preferably, the same cells as the cells in the sample.Thus, a compound modulating Wnt/β-catenin signaling can be identified based on the degree of identity or similarity between the amounts of the at least one biomarker in a sample and the aforementioned reference, i.e. based on a similar, preferably essentially identical, or, more preferably identical qualitative or quantitative composition with respect to the aforementioned biomarkers. The amounts of the biomarker and the reference are essentially identical, if the values for the characteristic features and, in the case of quantitative determination, the intensity values are essentially identical. Said amounts are similar, if the values of the characteristic features are identical but the intensity values are different. Such a difference is, preferably, not significant and shall be characterized in that the values for the intensity are within at least the interval between 1st and 99th percentile, 5th and 95th percentile, 10th and 90th percentile, 20th and 80th percentile, 30th and 70th percentile, or 40th and 60th percentile of the reference amount. Preferably, an identical amount of the biomarker and the reference obtained from a sample of cells having been brought into contact with a compound known to modulate Wnt/β**-**catenin signaling or an average reference, is indicative for a compound modulating Wnt/β-catenin signaling.

Advantageously, it has been found in the context of the present invention that the biomarkers identified across a panel of cell lines ensuring greatest possible independence from genetic background offer reliable identification of differential gene expression then microarrays. Specifically, the markers allow for directly measuring the activity of a cancer signaling pathway, i.e. the wnt-pathway. Using the method of the present invention, candidate compounds can be screened for their specificity and for their impact on said cancer signaling pathway.

The definitions made above apply mutatis mutandis to the following.

Moreover, the present invention relates to a method for identifying a drug, comprising the steps of the method for identifying a compound modulating Wnt/β-catenin signaling of the present invention and, further, the step of identifying a compound modulating Wnt/β-catenin signaling as a drug.

The term "drug", as used herein, relates to a chemical substance used in the treatment, cure, prevention, or diagnosis of disease in a subject. Preferably, the term relates to a chemical substance used in the treatment, cure, prevention, or diagnosis of cancer in a subject. Said drug, preferably, comprises the compound modulating Wnt/β-catenin signaling of the present invention and, more preferably, one or more pharmaceutically acceptable carrier. Preferably, said compound is a drug suspected to be a drug against cancer, more preferably the drug is a drug against cancer. The compound modulating Wnt/β-catenin signaling of the present invention can be formulated as a pharmaceutically acceptable salt. Acceptable salts comprise acetate, methylester, HCl, sulfate, chloride and the like. The drug is, preferably, administered topically or systemically. Suitable routes of administration conventionally used for drug administration are intratumoral, peritumoral, oral, intravenous, or parenteral administration as well as application as suppository. Preferably, oral and intravenous drug administration is used. However, depending on the nature and mode of action of the compound, the drug can be administered by other routes as well. For example, a polynucleotide compound is administered in a gene therapy approach by using viral vectors, viruses or liposomes. Moreover, the compound is administered in combination with other drugs either in a common drug or as separated drugs wherein said separated drugs can be provided in the form of a kit of parts. The compound is, preferably, administered in conventional dosage forms prepared by combining the drugs with standard pharmaceutical carriers according to conventional procedures. The procedure comprises mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

The carrier(s) must be acceptable in the sense of being compatible with the other ingredients of the formulation and being not deleterious to the recipient thereof. The pharmaceutical carrier employed can be, for example, either a solid, a gel or a liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania. The diluent(s) is/are selected so as not to affect the biological activity of the combination. Examples of such diluents are distilled water, physiological saline, Ringer's solutions, dextrose solution, and Hank's solution. In addition, the drug also include other carriers, adjuvants, or nontoxic, nontherapeutic, non-immunogenic stabilizers and the like.

A therapeutically effective dose relates to an amount of the compound to be used as a drug of the present invention, wherein said compound prevents or treats the symptoms accompanying cancer. Therapeutic efficacy and toxicity of such a compound can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. A typical dose can be, for example, in the range of 1 to 1000 µg; however, doses below or above this exemplary range are envisioned, especially considering the aforementioned factors. Generally, the regimen as a regular administration of the drug should be in the range of 1 µg to 10 mg units per day. If the regimen is a continuous infusion, it should also be in the range of 1 µg to 10 mg units per kilogram of body weight per minute, respectively. Progress can be monitored by periodic assessment. However, depending on the subject and the mode of administration, the quantity of substance administration may vary over a wide range to provide from about 0.01 mg per kg body mass to about 10 mg per kg body mass. The drugs referred to herein is administered at least once in order to treat or ameliorate or prevent cancer. However, the said drug can be administered more than one time, for example from one to four times daily up to a non-limited number of days. A specific drug is prepared in a manner well known in the pharmaceutical art and comprises at least one active compound referred to herein above in admixture or otherwise associated with a pharmaceutically acceptable carrier or diluent. For making those specific drug, the active compound will usually be mixed with a carrier or the diluent, or enclosed or encapsulated in a capsule, sachet, cachet, paper or other suitable containers or vehicles. The resulting formulations are to be adopted to the mode of administration, i.e. in the forms of tablets, capsules, suppositories, solutions, suspensions or the like. Dosage recommendation is indicated in the prescribers or users instructions in order to anticipate dose adjustments depending on the considered recipient.

The term "treatment", as used herein, refers to treatment of the diseases according to the present invention, preferably cancer, or of the symptoms accompanied therewith to a significant extent. The skilled artisan knows suitable therapies. Said therapy is, preferably, an anti-cancer therapy, more preferably, a therapy using a compound, wherein said compound is an inhibitor of the Wnt/β-catenin signaling pathway. Said therapy as used herein also aims to entirely restore the health with respect to the cancer referred to herein. It is to be understood that the therapy as used in accordance with the present invention may not be effective in all subjects to be treated. However, the term shall require that a statistically significant portion of subjects suffering from cancer can be successfully treated. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 60%, at least 70%, at least 80%, or at least 90% of the subjects of a given cohort or population.

The tem "prevention" refers to retainment of health with respect to the diseases according to the present invention, preferably cancer, or the symptoms referred to herein for a certain period of time in a subject. It will be understood that the said period of time is dependent on the amount of the drug compound administered and individual factors of the subject. It is to be understood that prevention may not be effective in all subjects treated with the compound according to the present invention. However, the term requires that a statistically significant portion of subjects of a cohort or population are effectively prevented from suffering from cancer or the symptoms referred to herein. Preferably, a cohort or population of subjects is envisaged in this context, wherein said cohort or population normally, i.e. without preventive measures according to the present invention, would develop cancer or symptoms as referred to herein. Whether a portion is statistically significant can be determined without further ado by the person skilled in the art using various well known statistic evaluation tools discussed above. Preferably, prevention shall be effective for at least 60%, at least 70%, at least 80%, or at least 90% of the subjects of a given cohort or population. Preferably, prevention is prevention of tissue invasion and metastasis formation by cancer cells.

The term "cancer", as used herein, relates to a disease of a subj ect characterized by uncontrolled growth by a group of body cells (cancer cells). This uncontrolled growth may be accompanied by intrusion into and destruction of surrounding tissue and possibly spread of cancer cells to other locations in the body. Thus, the term, preferably, encompasses pre-malignant lesions, malignant lesions as well as solid tumors and metastatic cancer (both local metastasis and distant metastasis). The symptoms accompanying cancer are well known from standard text books of medicine. Preferably, cancer is selected from the group consisting of colorectal cancer, breast cancer, esophageal cancer, melanoma, hepatocellular carcinoma, desmoid tumor, pancreatic cancer, gastric cancer, ovarian cancer, and prostate cancer.

In a further embodiment, the present invention relates to the use of at least the biomarkers NKD1 and C10orf54 in a sample comprising cells for determining whether said cells have been brought into contact with a compound modulating Wnt/β**-**catenin signaling. Preferably, said compound modulating Wnt/β-catenin signaling is an inhibitor of Wnt/β-catenin signaling. Preferably, said compound is a drug or suspected to be a drug against cancer. Preferably, said cancer is selected from the group consisting of: colorectal cancer, breast cancer, esophageal cancer, melanoma, hepatocellular carcinoma, desmoid tumor, pancreatic cancer, gastric cancer, ovarian cancer, and prostate cancer.

Moreover, the present invention relates to a method for diagnosing cancer in a subject suspected to suffer therefrom, comprising the steps of:
a) determining the amounts of at least the biomarkers NKD1 and C10orf54 in a sample of the said subject; and
b) comparing the amount of the biomarker NKD1 determined in step a) to a reference for NKD1 and comparing the amount of the biomarker C10orf54 determined in step a) to a reference for C10orf54, whereby cancer in the subject is to be diagnosed.

The term "diagnosing" as used herein refers to assessing whether a subject suffers from cancer, or not. As will be understood by those skilled in the art, such an assessment is usually not intended to be correct for 100% of the subjects to be diagxnosed. The term, however, requires that assessment of the presence or absence of cancer is correct for a statistically significant portion of the subjects (e.g. a cohort in a cohort study). Whether a portion is statistically significant can be determined without further ado by the person skilled in the art using various well known statistic evaluation tools as described herein above.

In the context of the method for diagnosing cancer of the present invention, the term "reference" relates to an amount, preferably, determined in a sample from a subject, wherein said subject (i) is apparently healthy or (ii) suffers from cancer. Also preferably, such reference amount is determined in a sample obtained from cells, wherein said cells (i) have not been brought into contact with a compound known to modulate Wnt/β-catenin signaling or (ii) have been brought into contact with a compound known to modulate Wnt/β-catenin signaling. Cells having not been brought into contact with a compound known to modulate Wnt/β-catenin signaling, are, e.g., cells with an unchanged Wnt/β-catenin signaling and, more preferably, apparently healthy cells. Cells having been brought into contact with a compound known to modulate Wnt/β-catenin signaling, are, e.g., cells with modulated Wnt/β-catenin signaling. The reference amount is determined as described herein above for the amount of the biomarker. Moreover, the reference, also preferably, is an average reference as described above.

Preferably, a subject suffering from cancer can be diagnosed based on the differences between the amounts of the at least one biomarker in a sample and the aforementioned reference, i.e. differences in the qualitative or quantitative composition with respect to the aforementioned biomarkers. The same applies if an average reference as specified above is used. The difference can be an increase in the absolute or relative amount of the biomarker (sometimes referred to as up-regulation of the biomarker; see also Examples) or a decrease in either of said amounts or the absence of a detectable amount of the biomarker (sometimes referred to as down-regulation of the biomarker; see also Examples). Preferably, the difference in the relative or absolute amount is significant, i.e. outside of the interval between 45th and 55th percentile, 40th and 60th percentile, 30th and 70th percentile, 20th and 80th percentile, 10th and 90th percentile, 5th and 95th percentile, 1st and 99th percentile of the reference amount. Preferably, an increased or decreased amount of the at least one biomarker in comparison to the reference obtained in a sample from an apparently healthy subject, a sample obtained from cells having not brought into contact with a compound known to modulate Wnt/β-catenin signaling, or an average reference is indicative for a subject suffering from cancer. Also preferably, such an increased or decreased amount of the at least one biomarker in comparison to the reference is indicative for a subject in need of an anti-cancer therapy.

Also preferably, a subject suffering from cancer can be identified based on the degree of identity or similarity between the amounts of the at least one biomarker in a sample and the aforementioned reference, i.e. based on an identical or similar qualitative or quantitative composition with respect to the aforementioned biomarkers. The amounts of the biomarker and the reference are identical, if the values for the characteristic features and, in the case of quantitative determination, the intensity values are identical. Said amounts are similar, if the values of the characteristic features are identical but the intensity values are different. Such a difference is, preferably, not significant and shall be characterized in that the values for the intensity are within at least the interval between 1st and 99th percentile, 5th and 95th percentile, 10th and 90th percentile, 20th and 80th percentile, 30th and 70th percentile, 40th and 60th percentile of the reference amount. Preferably, an identical amount of the biomarker and the reference obtained in a sample from a subject suffering from cancer, a sample obtained from cells having brought into contact with a compound known to modulate Wnt/β-catenin signaling, or an average reference is indicative for a subject suffering from cancer. Also preferably, such an identical amount of the biomarker and the reference is indicative for a subject in need of an anti-cancer therapy.

Further, the present invention relates to a method for determining whether a subject is in need of an anti-cancer therapy comprising the steps of:
a) determining the amounts of at least the biomarkers NKD1 and C10orf54 in a sample of the said subject; and
b) comparing the amount of the biomarker NKD1 determined in step a) to a reference for NKD1 and comparing the amount of the biomarker C10orf54 determined in step a) to a reference for C10orf54, whereby it is determined whether the subject is in need of the anti-cancer therapy.

Furthermore, the term "subject in need of an anti-cancer therapy" as used herein relates to a subject as described above suffering from cancer. In order to treat said cancer or the symptoms accompanied therewith to a significant extent, said subject is in need of an anti-cancer therapy. Preferably, said therapy uses an inhibitor of the Wnt/β-catenin signaling pathway.

The present invention also relates to a device adapted for carrying out a method of the invention comprising:
a) an analyzing unit comprising a detection agent, wherein said detection agent specifically determines the amounts of at least the biomarkers NKD1 and C10orf54 in a sample obtained from cells; and
b) an evaluation unit for comparing the determined amounts with reference amounts, said unit comprising a database with reference amount values and a computer-implemented algorithm for carrying out the comparison.

The term "device" as used herein relates to a system of means comprising at least the aforementioned means operatively linked to each other as to allow the identification. Preferred means for determining the amount of the said biomarkers and means for carrying out the comparison are disclosed above in connection with the methods of the invention. How to link the means in an operating manner will depend on the type of means included into the device. For example, where means for automatically determining the amount of the biomarkers are applied, the data obtained by said automatically operating means can be processed by, e.g., a computer program in order to establish an identification (i.e. of a compound modulating Wnt/β-catenin signaling), a diagnosis (i.e. a subject suffering from cancer), and/or a determination (i.e. whether a subject in in need of an anti-therapy). Preferably, said device allows the identification of a compound modulating Wnt/β-catenin signaling, the diagnosis a subject suffering from cancer, and/or the determination whether a subject in in need of an anti-cancer therapy. More preferably, the means are comprised by a single device in such a case. Said device may accordingly include an analyzing unit for the measurement of the amount of the biomarker in a sample and an evaluation unit for processing the resulting data for the diagnosis. Alternatively, where means such as test stripes are used for determining the amount of the gene products, the means for diagnosing may comprise control stripes or tables allocating the determined amount to an amount known to modulate Wnt/β-catenin signaling. Preferred means for detection are disclosed in connection with embodiments relating to the methods of the invention above. In such a case, the means are operatively linked in that the user of the system brings together the result of the determination of the amount and the identification and/or diagnostic value thereof due to the instructions and interpretations given in a manual. The means may appear as separate devices in such an embodiment and are, preferably, packaged together as a kit. The person skilled in the art will realize how to link the means without further inventive skills. Preferred devices are those which can be applied without the particular knowledge of a specialized clinician, e.g., test stripes or electronic devices which merely require loading with a sample. The results may be given as output of parametric diagnostic raw data, preferably, as absolute or relative amounts. It is to be understood that these data will need interpretation by the clinician. However, also envisaged are expert system devices wherein the output comprises processed diagnostic raw data the interpretation of which does not require a specialized clinician. Further preferred devices comprise the analyzing units/devices (e.g., biosensors, arrays, solid supports coupled to ligands specifically recognizing the polypeptides, Plasmon surface resonance devices, NMR spectrometers, mass- spectrometers etc.) or evaluation units/devices referred to above in accordance with the methods of the invention.

As used herein, the term "detection agent" relates to an agent specifically interacting with, and thus recognizing, the biomarkers defined above. Preferably, said detection agent is labeled in a way allowing detection of said detection agent inside the human body. Preferably, said detection agent is a polypeptide, e.g. an anticalin, a designed ankyrin repeat protein (DARPin), a single-chain T-cell receptor, or an antibody. Preferably, the detection agent is water soluble and can be transported to the cell via the bloodstream. Also preferably, the detection agent recognizes the biomarkers defined above by the presence of said biomarkers or a fragment thereof on the surface of a cell, like e.g. a single-chain T-cell receptor or an specifically recognizing antibody. Most preferably, the detection agent enters the cell, e.g. by endocytosis, by receptor mediated endocytosis or mediated by a protein transduction domain or the like. Preferably, the label allowing detection of the detection reagent inside the human body is a label as described herein above. More preferably, said label is detectable by computer tomography (CT, e.g. Iodine), by magnet resonance tomography (MRT, e.g. gadolinium), or by positron emission tomography (PET, e.g. 18F, 99mTc, 111In, 131I, or 186Re; see van Dongen et al. 2007, The Oncologist 12(12): 1379-1389) and non-invasive tomography methods well known to the skilled artisan.

The present invention further relates to a kit comprising instructions to carry out one of the methods of the present invention, and means for determining the amounts of at least the biomarkers NKD1 and C10orf54 in a sample comprising cells.

The term "kit" as used herein refers to a collection of the aforementioned components, preferably, provided separately or within a single container. The container, also preferably, comprises instructions for carrying out the method of the present invention. The kit, preferably, contains the aforementioned components in a ready-to-use formulation. Preferably, the kit may additionally comprise instructions, e.g., a user's manual for interpreting the results of any identification, diagnosis, and/or determination with respect to the methods of the present invention. Particularly, such manual may include information for allocating the amounts of the determined biomarkers to the kind of identification, diagnosis, and/or determination. Details are to be found elsewhere in this specification. Additionally, such user's manual may provide instructions about correctly using the components of the kit for determining the amount(s) of the respective biomarker. A user's manual may be provided in paper or electronic form, e.g., stored on CD or CD ROM, or downloadable via a web-interface from an online repository. The present invention also relates to the use of said kit in any of the methods according to the present invention.

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

### FIGURES

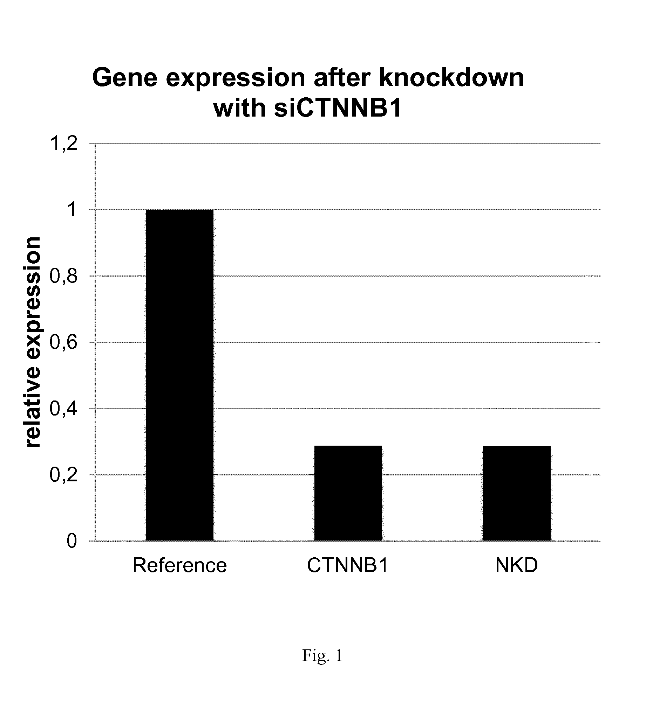

**Figure 1****:** Relative gene expression after knockdown with siCTNNB1 are shown for CTNNB1 and NKD. Gene expression was measured using quantitative Real-Time PCR.

### EXAMPLES

### Example 1: Identification of biomarkers modulating Wnt/β-catenin signaling

### RNAi Experiments

siRNAs against CTNNB1/β-catenin, APC and a control were obtained from Ambion (Applied Biosystems, Foster City, CA). Cells were transfected in 6-well plates with 10 nM siRNA using a total of 0.1% DharmaFECT 1 (Thermo Fischer Scientific, Waltham, MA) or RNAImax (Invitrogen, Carlsbad, CA). Cells were incubated for 72 hrs at 37°C and 5% CO₂ and two wells per knockdown were pooled for total RNA extraction using the RNeasy kit (Qiagen, Hilden).

### Expression profiling by RNA sequencing (RNAseq)

Total RNA was extracted using the RNeasy Mini kit (Qiagen, Hilden). RNA integrity was verified on an Agilent 2100 Bioanalyzer. mRNA was isolated with the Dynabeads mRNA Purification Kit (Invitrogen). For each sample, a library for SOLiD sequencing was prepared using the "Whole transcriptome analysis kit" (4409491 Rev. D, Applied Biosystems). In brief, polyA RNA was fragmented using RNAseIII, purified, hybridized and ligated to RNA adapters. Ligation products were reverse transcribed, purified and approx. 200 nt cDNA fragments were isolated after electrophoresis on a 6% TBE-Urea gel. cDNA was PCR amplified with SOLiD sequencing primers and purified to produce a library suitable as a template for emulsion PCR. DNA concentrations were determined using qPCR. A barcoded pooled library was generated by combining equimolar amounts from all samples. Emulsion PCR and sequencing with the SOLiD system was performed according to the manufacturers' protocol (Applied Biosystems).

### Bioinformatic analysis

Mapping of SOLiD reads was performed using the mapReads algorithm, supplied by Applied Biosystems as part of the whole transcriptome analysis pipeline (v1.2). Two mismatches were allowed in both seed regions (bases 1-25, and bases 20-45) of the aligned read. Reads that mapped uniquely to the Human Genome (Hg18) were retained for further analysis. Reads were also mapped to a filter reference (Applied Biosystems). The filter library removed from further analysis reads that aligned to sequences from ribosomal RNA, tRNAs, single base repeats, adapter sequences. The python package HTSeq (v0.4.7), was used to count the number of reads mapping to each gene. Expression values (read counts) were obtained by summing the number of reads that mapped uniquely to annotated regions of the Human Genome (Hg18). Reads that mapped to a locus that had more than one annotated gene were considered ambiguous and not used. Bioconductor package, DESeq (v1.3) (Anders and Huber, 2010, Genome Biol. 11(10): R106), was used to identify differentially expressed genes between siRNA knockdown samples and control siRNA. Differential expression was determined from read count data scaled for different sequencing depth. Reads per kilo base per million reads mapped (RPKM) values were calculated to estimate and compare mRNA expression levels across samples. Gene length was defined as the region that encompasses the union of all transcript variants of a gene that do not overlap other genes.

Using the above mentioned bioinformatics analysis, biomarkers, which are modulated in 3 or more cell lines could be identified (see Tables 3 and 4).

**Table 3: Shown are biomarkers being modulated in 3 or more cell lines according to the following conditions: opposite regulation in APC vs. CTNNB1 RNAi, AND (abs. log₂ FC APC > 1 AND abs. log₂ FC CTNNB1 > 1.5). Due to the aforementioned opposing roles of APC and β-catenin (CTNNB1) in regulating the expression of Wnt/β-catenin target genes, opposite regulation after knockdown of either genes is central to ensure the selection of genes that are targets of the Wnt/β-catenin signaling pathway. Logarithmic conversion is a frequently used method to present fold changes (FC). Thresholds for FC changes were used to remove genes which do not respond strongly to the Wnt/β-catenin signaling pathway and, thus, would not be reliable to detect changes in the said Wnt/β-catenin signaling pathway. As β-catenin is the most central component in Wnt/β-catenin signaling pathway, a more stringent cutoff was placed on this parameter as opposed to APC, which is one of several factors blocking Wnt/β-catenin signaling pathway. Hence, APC knockdown phenotypes may be less pronounced than β-catenin knockdown phenotypes, especially, when comparing several cell lines. The efficiency of knockdown may vary between cell lines: while most CTNNB1 siRNAs are good knockdowns, APC knockdown efficiency shows more variation. The term "n.d." (not detectable) applies to down-regulated genes only, where no reads were found after knockdown.**

| Gene Symbol | ENSBL_ID; ENTREZ_ID | Caco2; siCTNN | Colo320; siCTNN | DLD1; siCTNN | HCT116; siCTNN | HT29; siCTNN | RKO; siCTNN | SW480; siCTNN |
|---|---|---|---|---|---|---|---|---|
| | | B1 knock-down | B1 knock-down | B1 knock-down | B1 knock-down | B1 knock-down | B1 knock-down | B1 knockdown |
| NKD1 | ENSG00000140 807; 85407 | -2.104 | n.d. | -2.159 | -2.093 | n.d. | n.d. | -1.528 |
| C10orf 54 | ENSG00000107 738; 64115 | 1.367 | 2.027 | 2.496 | n.d. | 1.592 | 1.55 | n.d. |
| PSCA | ENSG00000167 653; 8000 | n.d. | 2.101 | 4.110 | n.d. | 1.687 | n.d. | 2.157 |
| CPNE5 | ENSG00000124 772; 57699 | 2.053 | 2.632 | 2.497 | n.d. | n.d. | n.d. | n.d. |

**Table 4: Shown are biomarkers being modulated in 4 or more cell lines according to the following conditions opposite regulation in APC vs. CTNNB1 RNAi, AND (abs. log₂ FC APC > 1 AND abs. log₂ FC CTNNB1 > 1.5). Due to the aforementioned opposing roles of APC and β-catenin (CTNNB1) in regulating the expression of Wnt/β-catenin target genes, opposite regulation after knockdown of either genes is central to ensure the selection of genes that are targets of the Wnt/β-catenin signaling pathway. Logarithmic conversion is a frequently used method to present fold changes (FC). Thresholds for FC changes were used to remove genes which do not respond strongly to the Wnt/β-catenin signaling pathway and, thus, would not be reliable to detect changes in the said Wnt/β-catenin signaling pathway. As β-catenin is the most central component in Wnt/β-catenin signaling pathway, a more stringent cutoff was placed on this parameter as opposed to APC which is one of several factors blocking Wnt/β-catenin signaling pathway. Hence, APC knockdown phenotypes may be less pronounced than β-catenin knockdown phenotypes, especially when comparing several cell lines. The efficiency of knockdown may vary between cell lines: while most CTNNB1 siRNAs are good knockdowns, APC knockdown efficiency shows more variation. The term "n.d." (not detectable) applies to down-regulated genes only, where no reads were found after knockdown.**

| Gene Symbol | ENSBL_ID; ENTREZ_ID | Caco2; siCTNN | Colo320; siCTNN | DLD1; siCTNN | HCT116; siCTNN | HT29; siCTNN | RKO; siCTNN | SW480; siCTNN |
|---|---|---|---|---|---|---|---|---|
| | | B1 knock-down | B1 knock-down | B1 knock-down | B1 knock-down | B1 knock-down | B1 knock-down | B1 knockdown |
| NKD1 | ENSG00000140 807; 85407 | -2.104 | n.d. | -2.159 | -2.093 | n.d. | n.d. | -1.528 |
| C10orf 54 | ENSG00000107 738; 64115 | 1.367 | 2.027 | 2.496 | n.d. | 1.592 | 1.55 | n.d. |
| PSCA | ENSG00000167 653; 8000 | n.d. | 2.101 | 4.110 | n.d. | 1.687 | n.d. | 2.157 |
| CPNE5 | ENSG00000124 772; 57699 | 2.053 | 2.632 | 2.497 | n.d. | n.d. | n.d. | n.d. |
| RAET1 G | ENSG00000203 722; 353091 | n.d. | 1.516 | 1.513 | n.d. | 2.691 | n.d. | 1.741 |

The p-values of the biomarkers displayed in Tables 3 and 4 reflect significance of the differential expression for each individual biomarker (see Tables 5 and 6 below). However, said p-values do not reflect significance over the whole set of cell lines and they do not reflect any additional parameter, e.g. that APC and CTNNB1 knockdown would cause opposite phenotypes. Due to differences in sequencing depth of the samples (i.e. lower sequencing depth), some of the p-values displayed in Tables 5 and/or 6 seem to be not significant.

**Table 5: P-values of the biomarkers displayed in Tables 3 and 4.**

| Gene Symbol | ENSBL_ID; ENTREZ_ID | Caco2; siCTNN | Colo320; siCTNN | DLD1; siCTNN | HCT116; siCTNN | HT29; siCTNN | RKO; siCTNN | SW480; siCTNN |
|---|---|---|---|---|---|---|---|---|
| | | B1 knock-down | B1 knock-down | B1 knock-down | B1 knock-down | B1 knock-down | B1 knock-down | B1 knock-down |
| NKD1 | ENSG00000140 807; 85407 | 0.085 | | 0.011 | 0.005 | | 0.540 | 1.950E-05 |
| C10orf 54 | ENSG00000107 738; 64115 | 0.047 | 0.097 | 4.430E-06 | | 0.000 | | |
| PSCA | ENSG00000167 653; 8000 | | 0.844 | 0.844 | | 0.035 | | 0.608 |
| CPNE5 | ENSG00000124 772; 57699 | 0.791 | 0.344 | 0.344 | | | | |
| RAET1 G | ENSG00000203 722; 353091 | | 0.805 | 0.560 | | 0.918 | | 0.765 |
| UCP3 | ENSG00000175 564; 7352 | 1.000 | 0.705 | | 0.968 | | 0.599 | 0.637 |

**Table 6: Benjamini corrected p-values of the biomarkers displayed in Tables 3 and 4.**

| Gene Symbol | ENSBL_ID; ENTREZ_ID | Caco2; siCTNN | Colo320; siCTNN | DLD1; siCTNN | HCT116; siCTNN | HT29; siCTNN | RKO; siCTNN | SW480; siCTNN |
|---|---|---|---|---|---|---|---|---|
| | | B1 knock-down | B1 knock-down | B1 knock-down | B1 knock-down | B1 knock-down | B1 knock-down | B1 knockdown |
| NKD1 | ENSG00000140 807; 85407 | 1.000 | | 0.265 | 0.589 | | 1.000 | 0.009 |
| C10orf 54 | ENSG00000107 738; 64115 | 1.000 | 1.000 | 0.001 | | 0.062 | | |
| PSCA | ENSG00000167 653; 8000 | | 1.000 | 1.000 | | 1.000 | | 1.000 |
| CPNE5 | ENSG00000124 772; 57699 | 1.000 | 1.000 | 1.000 | | | | |
| RAET1 G | ENSG00000203 722; 353091 | | 1.000 | 0.989 | | 1.000 | | 1.000 |
| UCP3 | ENSG00000175 564; 7352 | 1.000 | 1.000 | | 1.000 | | 1.000 | 1.000 |

### Example 2: Identification of individual cell line response genes

### siRNA Transfection

siRNAs against CTNNB1/β-catenin, Evi, APC and the control were obtained from Ambion (Applied Biosystems, Foster City, CA). Cells were transfected in 12- or 6-well plates with 10 nM siRNA using a total of 0.1% DharmaFECT 1 (Thermo Fischer Scientific, Waltham, MA) or RNAImax (Invitrogen, Carlsbad, CA).

### Expression profiling by RNA sequencing (RNAseq)

Total RNA was extracted as described below and RNA integrity was verified on an Agilent 2100 Bioanalyzer. mRNA was isolated with the Dynabeads mRNA Purification Kit (Invitrogen). For each sample, a library for SOLiD sequencing was prepared using the 'Whole transcriptome analysis kit' (4409491 Rev. D, Applied Biosystems). In brief, polyA RNA was fragmented using RNAseIII, purified, hybridized and ligated to RNA adapters. Ligation products were reverse transcribed, purified and approx. 200 nt cDNA fragments were isolated after electrophoresis on a 6% TBE-Urea gel. cDNA was PCR amplified with SOLiD sequencing primers and purified to produce a library suitable as a template for emulsion PCR. DNA concentrations were determined using qPCR. A barcoded pooled library was generated by combining equimolar amounts from all samples. Emulsion PCR and sequencing with the SOLiD system was performed according to the manufacturers' protocol (Applied Biosystems).

### Bioinformatic analysis

Mapping of SOLiD reads was performed using the mapReads algorithm, supplied by Applied Biosystems as part of the whole transcriptome analysis pipeline (v1.2). Two mismatches were allowed in both seed regions (bases 1-25, and bases 20-45) of the aligned read. Reads that mapped uniquely to the Human Genome (Hg18) were retained for further analysis. Reads were also mapped to a filter reference (Applied Biosystems). The filter library removed from further analysis reads that aligned to sequences from ribosomal RNA, tRNAs, single base repeats, adapter sequences. The python package HTSeq (v0.4.7), was used to count the number of reads mapping to each gene (http://www-huber.embl.de/users/anders/HTSeq/doc/overview.html). Expression values (read counts) were obtained by summing the number of reads that mapped uniquely to annotated regions of the Human Genome (Hg18). Reads that mapped to a locus that had more than one annotated gene were considered ambiguous and not used. Bioconductor package, DESeq (v1.3; Anders and Huber, 2010, Genome Biol. 11(10): R106), was used to identify differentially expressed genes between siRNA knockdown samples and control siRNA. Differential expression was determined from read count data scaled for different sequencing depth. Reads per kilo base per million reads mapped (RPKM) values were calculated to estimate and compare mRNA expression levels across samples. Gene length was defined as the region that encompasses the union of all transcript variants of a gene that do not overlap other genes.

Table 7 shows the five most significant up- and down-regulated biomarkers determined in 7 individual cell lines according to the following conditions: opposite regulation in APC vs. CTNNB1 RNAi, AND (abs. log₂ FC APC > 1 AND abs. log₂ FC CTNNB1 > 1.5).

**Table 7: The five most significant up- and down-regulated biomarkers were determined in several individual cell lines according to the following conditions: opposite regulation in APC vs. CTNNB1 RNAi, AND (abs. log₂ FC APC > 1 AND abs. log₂ FC CTNNB1 > 1.5). Due to the aforementioned opposing roles of APC and β-catenin (CTNNB1) in regulating the expression of Wnt/β-catenin target genes, opposite regulation after knockdown of either genes is central to ensure the selection of genes that are targets of the Wnt/β-catenin signaling pathway. Logarithmic conversion is a frequently used method to present fold changes (FC). Thresholds for FC changes were used to remove genes which do not respond strongly to the Wnt/β-catenin signaling pathway and, thus, would not be reliable to detect changes in the said Wnt/β-catenin signaling pathway. As β-catenin is the most central component in Wnt/β-catenin signaling pathway, a more stringent cutoff was placed on this parameter as opposed to APC which is one of several factors blocking Wnt/β-catenin signaling pathway. Hence, APC knockdown phenotypes may be less pronounced than β-catenin knockdown phenotypes, especially, when comparing several cell lines. The efficiency of knockdown may vary between cell lines: while most CTNNB1 siRNAs are good knockdowns, APC knockdown efficiency shows more variation.**

| **Caco2** | | | | |
|---|---|---|---|---|
| Gene Symbol | ENSBL_ID; ENTREZ_ID | Regulation by CTNNB1 knockdown | CTNNB1 knockdown; log 2 fold change | APC; log 2 fold change |
| LGR5 | ENSG00000139292; 8549 | down | -3,328 | 1,392 |
| FAM46C | ENSG00000183508; 54855 | down | -1,791 | 1,032 |
| NKD1 | ENSG00000140807; 85407 | down | -2,104 | 1,217 |
| ITGA9 | ENSG00000144668; 3680 | down | -2,502 | 1,04 |
| WNT5B | ENSG00000111186; 81029 | down | -2,629 | 1,394 |
| SYTL5 | ENSG00000147041; 94122 | up | 1,877 | -1,647 |
| PTPRR | ENSG00000153233; 5801 | up | 1,875 | -1,135 |
| PHEX | ENSG00000102174; 5251 | up | 1,574 | -1,323 |
| PIK3CD | ENSG00000171608; 5293 | up | 2,316 | -1,945 |
| PIK3IP1 | ENSG00000100100; 113791 | up | 2,178 | -1,024 |

| **Colo320** | | | | |
|---|---|---|---|---|
| Gene Symbol | ENSBL_ID; ENTREZ_ID | Regulation by CTNNB1 knockdown | CTNNB1 knockdown; log 2 fold change | APC; log 2 fold change |
| MANF | ENSG00000145050; 7873 | down | -1,584 | 1,509 |
| TMEM200A | ENSG00000164484; 114801 | down | -1,83 | 1,409 |
| FABP2 | ENSG00000145384; 2169 | down | -2,291 | 2,388 |
| ST8SIA4 | ENSG00000113532; 7903 | down | -1,806 | 1,241 |
| LACE1 | ENSG00000135537; 246269 | down | -2,654 | 1,239 |
| IGFBP5 | ENSG00000115461; 3488 | up | 5,866 | -1,297 |
| EPAS1 | ENSG00000116016; 2034 | up | 3,708 | -1,469 |
| CLUL1 | ENSG00000079101; 27098 | up | 5,612 | -1,771 |
| PLCE1 | ENSG00000138193; 51196 | up | 5,217 | -1,449 |
| SHISA3 | ENSG00000178343; 152573 | up | 5,47 | n.d. |

| **DLD1** | | | | |
|---|---|---|---|---|
| Gene Symbol | ENSBL_ID; ENTREZ_ID | Regulation by CTNNB1 knockdown | CTNNB1 knockdown; log 2 fold change | APC; log 2 fold change |
| PRR9 | ENSG00000203783; 574414 | down | -2,403 | 2,08 |
| CADPS | ENSG00000163618; 8618 | down | -1,773 | 2,082 |
| NKD1 | ENSG00000140807; 85407 | down | -2,159 | 2,396 |
| BCL11A | ENSG00000119866; 53335 | down | -4,501 | 2,007 |
| PTGS2 | ENSG00000073756; 5743 | down | -2,627 | 2,278 |
| REG4 | ENSG00000134193; 83998 | up | 5,173 | -1,058 |
| PSCA | ENSG00000167653; 8000 | up | 4,11 | -1,313 |
| KRT4 | ENSG00000170477; 3851 | up | 5,577 | -1,682 |
| CD177 | ENSG00000204936; 57126 | up | 4,038 | -1,421 |
| RBP4 | ENSG00000138207; 5950 | up | 3,754 | -2,042 |

| **HCT116** | | | | |
|---|---|---|---|---|
| Gene Symbol | ENSBL_ID; ENTREZ_ID | Regulation by CTNNB1 knockdown | CTNNB1 knockdown; log 2 fold change | APC; log 2 fold change |
| ADAM19 | ENSG00000135074; 8728 | down | -1,922 | 1,156 |
| ADAMTS14 | ENSG00000138316; 140766 | down | -2,699 | 1,157 |
| AXIN2 | ENSG00000168646; 8313 | down | -2,277 | 3,272 |
| DUSP6 | ENSG00000139318; 1848 | down | -1,525 | 1,175 |
| KIAA1199 | ENSG00000103888; 57214 | down | -1,825 | 1,865 |
| RGS2 | ENSG00000116741; 5997 | up | 1,672 | -2,435 |
| GPR87 | ENSG00000138271; 53836 | up | 2,532 | -1,009 |
| DPP4 | ENSG00000197635; 1803 | up | 1,521 | -1,822 |
| THSD7A | ENSG00000005108; 221981 | up | 2,884 | n.d. |
| LAMA4 | ENSG00000112769; 3910 | up | 1,545 | -2,556 |
| RUNX2 | ENSG00000124813; 860 | down | -2,486 | 1,953 |
| NKD1 | ENSG00000140807; 85407 | down | -2,093 | 2,396 |

| **HT29** | | | | |
|---|---|---|---|---|
| Gene Symbol | ENSBL_ID; ENTREZ_ID | Regulation by CTNNB1 knockdown | CTNNB1 knockdown; log 2 fold change | APC; log 2 fold change |
| WNT11 | ENSG00000085741; 7481 | down | -2,023 | 2,582 |
| PTPRO | ENSG00000151490; 5800 | down | -1,674 | 1,168 |
| CD83 | ENSG00000112149; 9308 | down | -2,279 | 1,035 |
| LRRC2 | ENSG00000163827; 79442 | down | -4,253 | 1,115 |
| ATOH8 | ENSG00000168874; 84913 | down | -3,62 | 1,403 |
| CEACAM5 | ENSG00000105388; 1048 | up | 4,842 | -3,45 |
| SDCBP2 | ENSG00000125775; 27111 | up | 2,365 | -1,338 |
| VSIG1 | ENSG00000101842; 340547 | up | 5,042 | -1,865 |
| TFF3 | ENSG00000160180; 7033 | up | 2,023 | -1,523 |
| C10orf54 | ENSG00000107738; 64115 | up | 1,592 | -1,114 |

| **RKO** | | | | |
|---|---|---|---|---|
| Gene Symbol | ENSBL_ID; ENTREZ_ID | Regulation by CTNNB1 knockdown | CTNNB1 knockdown; log 2 fold change | APC; log 2 fold change |
| SNAR-F | Not available; 100126781 | down | n.d. | 1,55 |
| SNORD31 | ENSG00000201669, ENSG00000201847; 9298 | down | n.d. | 1,55 |
| GC | ENSG00000145321; 2638 | down | n.d. | 1,064 |
| IMMP1L | ENSG00000148950; 196294 | down | n.d. | 1,479 |
| UCP3 | ENSG00000175564; 7352 | down | n.d. | 1,479 |
| CREB3L1 | ENSG00000157613; 90993 | up | 3,496 | n.d. |
| MACC1 | ENSG00000183742; 346389 | up | 3,904 | n.d. |
| KRT20 | ENSG00000171431; 54474 | up | 1,965 | -2,164 |
| ARL9 | ENSG00000196503; 132946 | up | 1,782 | -1,48 |
| DISP2 | ENSG00000140323; 85455 | up | 2,319 | n.d. |

| **SW480** | | | | |
|---|---|---|---|---|
| Gene Symbol | ENSBL_ID; ENTREZ_ID | Regulation by CTNNB1 knockdown | CTNNB1 knockdown; log 2 fold change | APC; log 2 fold change |
| CPXM1 | ENSG00000088882; 56265 | up | n.d. | 1,236 |
| MSI1 | ENSG00000135097; 4440 | up | -2,428 | 1,099 |
| CHRNB4 | ENSG00000117971; 1143 | up | -2,165 | 2,262 |
| SLC19A3 | ENSG00000135917; 80704 | up | n.d. | 1,999 |
| DKK4 | ENSG00000104371; 27121 | up | n.d. | 1,777 |
| GPR39 | ENSG00000183840; 2863 | down | 2,819 | -1,808 |
| CCDC159 | ENSG00000183401; 126075 | down | 2,741 | n.d. |
| TIMP4 | ENSG00000157150; 7079 | down | 2,741 | n.d. |
| APOL3 | ENSG00000128284; 80833 | down | 1,519 | -2,03 |
| MEGF10 | ENSG00000145794; 84466 | down | 2,031 | n.d. |

### Example 3: Gene expression after knockdown with siCTNNB1

Relative gene expression after knockdown with **siCTNNB1** is shown in Figure 1. Gene expression was measured using quantitative Real-Time PCR. siRNAs against CTNNB1/β-catenin, Evi, APC and the control were obtained from Ambion (Applied Biosystems, Foster City, CA). Cells were transfected in 12- or 6-well plates with 10 nM siRNA using a total of 0.1 % DharmaFECT 1 (Thermo Fischer Scientific, Waltham, MA) or RNAImax (Invitrogen, Carlsbad, CA). cDNA was prepared from 0.5 or 2 µg total RNA using the RevertAid H Minus First Strand cDNA Synthesis Kit (Thermo Fischer Scientific). Oligo-dT primers were used to prime the reaction. Subsequently the cDNA was diluted to 5ng/µl and used for qPCR on the Lightcylcer480 (Roche) using the universal probe library system (Roche) in a 384-well format. Primer probe combinations for each quarry gene were constructed using the Roche ProbeFinder V2.45, automatically selecting intron-spanning primers. All samples were amplified as triplicates and differential expression was analyzed using the LightCycler®480 Software. The reference refers to UBC and GAPDH combined to serve as a housekeeping gene. Corresponding relative gene expression amounts are shown in Table 8.

**Table 8: Relative gene expression amounts of CTNNB1 and NKD.**

| | **Replicate 1** | **Replicate 2** | **Replicate Average** | |
|---|---|---|---|---|
| **Reference** | 1.000 | 1.000 | 1.000 | 0.000 |
| **CTNNB1** | 0.250 | 0.325 | 0.288 | -1.797 |
| **NKD** | 0.225 | 0.350 | 0.287 | -1.801 |
| | | | | |

| | Error rate Replicate 1 | Error rate Replicate 2 | Standarddeviation | |
|---|---|---|---|---|
| **Reference** | 0.000 | 0.000 | 0.000 | |
| **CTNNB1** | 0.047 | 0.254 | 0.053 | |
| **NKD** | 0.069 | 0.067 | 0.088 | |

### Example 4: Known polypeptides and/or regulators of the Wnt/β-catenin signaling pathway in humans

About 150 known polypeptides and/or regulators of the Wnt/β-catenin signaling pathway in humans are shown in Table 9.

**Table 9: Known polypeptides and/or regulators of the Wnt/β-catenin signaling pathway in humans.**

| **ENSEMBL GENE_ID** | **ENTREZ GENE_ID** | **Description** |
|---|---|---|
| ENSG00000159692 | 1487 | hsa04310:Wnt signaling pathway, hsa04330:Notch signaling pathway, hsa05200:Pathways in cancer, hsa05220:Chronic myeloid leukemia, |
| ENSG00000175029 | 1488 | hsa04310:Wnt signaling pathway, hsa04330:Notch signaling pathway, hsa05200:Pathways in cancer, hsa05220:Chronic myeloid leukemia, |
| ENSG00000005339 | 1387 | hsa04110:Cell cycle, hsa04310:Wnt signaling pathway, hsa04330:Notch signaling pathway, hsa04350:TGF-beta signaling pathway, hsa04520:Adherens junction, hsa04630:Jak-STAT signaling pathway, hsa04720:Long-term potentiation, hsa04916:Melanogenesis, hsa05016:Huntington's disease, hsa05200:Pathways in cancer, hsa05211 :Renal cell carcinoma, hsa05215:Prostate cancer, |
| ENSG00000168772 | 80319 | hsa04310:Wnt signaling pathway, |
| ENSG00000100393 | 2033 | hsa04110:Cell cycle, hsa04310:Wnt signaling pathway, hsa04330:Notch signaling pathway, hsa04350:TGF-beta signaling pathway, hsa04520:Adherens junction, hsa04630:Jak-STAT signaling pathway, hsa04720:Long-term potentiation, hsa04916:Melanogenesis, hsa05016:Huntington's disease, hsa05200:Pathways in cancer, hsa05211 :Renal cell carcinoma, hsa05215:Prostate cancer, |
| ENSG00000072803 | 23291 | hsa04114:Oocyte meiosis, hsa04120:Ubiquitin mediated proteolysis, hsa04310:Wnt signaling pathway, hsa04340:Hedgehog signaling pathway, |
| ENSG00000175592 | 8061 | hsa04310:Wnt signaling pathway, |
| ENSG00000134318 | 9475 | hsa04062:Chemokine signaling pathway, hsa04270:Vascular smooth muscle contraction, hsa04310:Wnt signaling pathway, hsa04350:TGF-beta signaling pathway, hsa04360:Axon guidance, hsa04510:Focal adhesion, hsa04670:Leukocyte transendothelial migration, hsa04810:Regulation of actin cytoskeleton, hsa05130:Pathogenic Escherichia coli infection, |
| ENSG00000175792 | 8607 | hsa04310:Wnt signaling pathway, |
| ENSG00000113558 | 6500 | hsa04110:Cell cycle, hsa04114:0ocyte meiosis, hsa04120:Ubiquitin mediated proteolysis, hsa04310:Wnt signaling pathway, hsa04350:TGF-beta signaling pathway, |
| ENSG00000175387 | 4087 | hsa04110:Cell cycle, hsa04310:Wnt signaling pathway, hsa04350:TGF-beta signaling pathway, hsa04520:Adherens junction, hsa05200:Pathways in cancer, hsa05210:Colorectal cancer, hsa05212:Pancreatic cancer, |
| ENSG00000166949 | 4088 | hsa04110:Cell cycle, hsa04310:Wnt signaling pathway, hsa04350:TGF-beta signaling pathway, hsa04520:Adherens junction, hsa05200:Pathways in cancer, hsa05210:Colorectal cancer, hsa05212:Pancreatic cancer, hsa05220:Chronic myeloid leukemia, |
| ENSG00000141646 | 4089 | hsa04110:Cell cycle, hsa04310:Wnt signaling pathway, hsa04350:TGF-beta signaling pathway, hsa04520:Adherens junction, hsa05200:Pathways in cancer, hsa05210:Colorectal cancer, hsa05212:Pancreatic cancer, hsa05220:Chronic myeloid leukemia, |
| ENSG00000164736 | 64321 | hsa04310:Wnt signaling pathway, |
| ENSG00000163904 | 59343 | hsa04310:Wnt signaling pathway, |
| ENSG00000156076 | 11197 | hsa04310:Wnt signaling pathway, |
| ENSG00000115266 | 10297 | hsa04310:Wnt signaling pathway, hsa04810:Regulation of actin cytoskeleton, hsa05200:Pathways in cancer, hsa05210:Colorectal cancer, hsa05213:Endometrial cancer, hsa05217:Basal cell carcinoma, |
| ENSG00000134982 | 324 | hsa04310:Wnt signaling pathway, hsa04810:Regulation of actin cytoskeleton, hsa05200:Pathways in cancer, hsa05210:Colorectal cancer, hsa05213:Endometrial cancer, hsa05217:Basa1 cell carcinoma, |
| ENSG00000103126 | 8312 | hsa04310:Wnt signaling pathway, hsa05200:Pathways in cancer, hsa05210:Colorectal cancer, hsa05213:Endometrial cancer, hsa05217:Basal cell carcinoma, |
| ENSG00000168646 | 8313 | hsa04310:Wnt signaling pathway, hsa05200:Pathways in cancer, hsa05210:Colorectal cancer, hsa05213:Endometrial cancer, hsa05217:Basal cell carcinoma, |
| ENSG00000166167 | 8945 | hsa04114:Oocyte meiosis, hsa04120:Ubiquitin mediated proteolysis, hsa04310:Wnt signaling pathway, hsa04340:Hedgehog signaling pathway, |
| ENSG00000166869 | 63928 | hsa04010:MAPK signaling pathway, hsa04020:Calcium signaling pathway, hsa04114:Oocyte meiosis, hsa04210:Apoptosis, hsa04310:Wnt signaling pathway, hsa04360:Axon guidance, hsa04370:VEGF signaling pathway, hsa04650:Natural killer cell mediated cytotoxicity, hsa04660:T cell receptor signaling pathway, hsa04662:B cell receptor signaling pathway, hsa04720:Long-term potentiation, hsa05010:Alzheimer's disease, hsa05014:Amyotrophic lateral sclerosis (ALS), |
| ENSG00000187446 | 11261 | hsa04010:MAPK signaling pathway, hsa04020:Calcium signaling pathway, hsa04114:Oocyte meiosis, hsa04210:Apoptosis, hsa04310:Wnt signaling pathway, hsa04360:Axon guidance, hsa04370:VEGF signaling pathway, hsa04650:Natural killer cell mediated cytotoxicity, hsa04660:T cell receptor signaling pathway, hsa04662:B cell receptor signaling pathway, hsa04720:Long-term potentiation, hsa05010:Alzheimer's disease, hsa05014:Amyotrophic lateral sclerosis (ALS), |
| ENSG00000070808 | 815 | hsa04012:ErbB signaling pathway, hsa04020:Calcium signaling pathway, hsa04114:0ocyte meiosis, hsa04310:Wnt signaling pathway, hsa04720:Long-term potentiation, hsa04722:Neurotrophin signaling pathway, hsa04740:Olfactory transduction, hsa04912:GnRH signaling pathway, hsa04916:Melanogenesis, hsa05214:Glioma, |
| ENSG00000058404 | 816 | hsa04012:ErbB signaling pathway, hsa04020:Calcium signaling pathway, hsa04114:Oocyte meiosis, hsa04310:Wnt signaling pathway, hsa04720:Long-term potentiation, |
| | | hsa04722:Neurotrophin signaling pathway, hsa04740:Olfactory transduction, hsa04912:GnRH signaling pathway, hsa04916:Melanogenesis, hsa05214:Glioma, |
| ENSG00000145349 | 817 | hsa04012:ErbB signaling pathway, hsa04020:Calcium signaling pathway, hsa04114:Oocyte meiosis, hsa04310:Wnt signaling pathway, hsa04720:Long-term potentiation, hsa04722:Neurotrophin signaling pathway, hsa04740:Olfactory transduction, hsa04912:GnRH signaling pathway, hsa04916:Melanogenesis, hsa05214:Glioma, |
| ENSG00000148660 | 818 | hsa04012:ErbB signaling pathway, hsa04020:Calcium signaling pathway, hsa04114:Oocyte meiosis, hsa04310:Wnt signaling pathway, hsa04720:Long-term potentiation, hsa04722:Neurotrophin signaling pathway, hsa04740:Olfactory transduction, hsa04912:GnRH signaling pathway, hsa04916:Melanogenesis, hsa05214:Glioma, |
| ENSG00000113712 | 1452 | hsa04310:Wnt signaling pathway, hsa04340:Hedgehog signaling pathway, |
| ENSG00000180138 | 122011 | hsa04310:Wnt signaling pathway, hsa04340:Hedgehog signaling pathway, |
| ENSG00000213923 | 1454 | hsa04310:Wnt signaling pathway, hsa04340:Hedgehog signaling pathway, hsa04710:Circadian rhythm, |
| ENSG00000101266 | 1457, 283106 | hsa04310:Wnt signaling pathway, hsa04520:Adherens junction, hsa04530:Tight junction, |
| ENSG00000070770 | 1459 | hsa04310:Wnt signaling pathway, hsa04520:Adherens junction, hsa04530:Tight junction, |
| ENSG00000168036 | 1499 | hsa04310:Wnt signaling pathway, hsa04510:Focal adhesion, hsa04520:Adherens junction, hsa04530:Tight junction, hsa04670:Leukocyte transendothelial migration, hsa04916:Melanogenesis, hsa05130:Pathogenic Escherichia coli infection, hsa05200:Pathways in cancer, hsa05210:Colorectal cancer, hsa05213 :Endometrial cancer, hsa05215 :Prostate cancer, hsa05216:Thyroid cancer, hsa05217:Basal cell carcinoma, hsa05412:Arrhythmogenic right ventricular cardiomyopathy (ARVC), |
| ENSG00000178585 | 56998 | hsa04310:Wnt signaling pathway, |
| ENSG00000147869 | 9350 | hsa04310:Wnt signaling pathway, |
| ENSG00000100888 | 57680 | hsa04310:Wnt signaling pathway, |
| ENSG00000055130 | 8454 | hsa04110:Cell cycle, hsa04114:Oocyte meiosis, hsa04120:Ubiquitin mediated proteolysis, hsa04310:Wnt signaling pathway, hsa04350:TGF-beta signaling pathway, |
| ENSG00000110092 | 595 | hsa04110:Cell cycle, hsa04115:p53 signaling pathway, hsa04310:Wnt signaling pathway, hsa04510:Focal adhesion, hsa04630:Jak-STAT signaling pathway, hsa05200:Pathways in cancer, hsa05210:Colorectal cancer, hsa05212:Pancreatic cancer, hsa05213:Endometrial cancer, hsa05214:Glioma, hsa05215:Prostate cancer, hsa05216:Thyroid cancer, hsa05218:Melanoma, hsa05219:Bladder cancer, hsa05220:Chronic myeloid leukemia, hsa05221:Acute myeloid leukemia, hsa05222:Small cell lung cancer, hsa05223:Non-small cell lung cancer, hsa05416:Viral myocarditis, |
| ENSG00000118971 | 894 | hsa04110:Cell cycle, hsa04115:p53 signaling pathway, hsa04310:Wnt signaling pathway, hsa04510:Focal adhesion, |
| | | hsa04630:Jak-STAT signaling pathway, |
| ENSG00000112576 | 896 | hsa04110:Cell cycle, hsa04115:p53 signaling pathway, hsa04310:Wnt signaling pathway, hsa04510:Focal adhesion, hsa04630:Jak-STAT signaling pathway, |
| ENSG00000107984 | 22943 | hsa04310:Wnt signaling pathway, |
| ENSG00000155011 | 27123 | hsa04310:Wnt signaling pathway, |
| ENSG00000104371 | 27121 | hsa04310:Wnt signaling pathway, |
| ENSG00000100592 | 23002 | hsa04310:Wnt signaling pathway, |
| ENSG00000146122 | 23500 | hsa04310:Wnt signaling pathway, |
| ENSG00000107404 | 1855, 8215 | hsa04310:Wnt signaling pathway, hsa04330:Notch signaling pathway, hsa04916:Melanogenesis, hsa05200:Pathways in cancer, hsa05210:Colorectal cancer, hsa05217:Basal cell carcinoma, |
| ENSG00000004975 | 1856 | hsa04310:Wnt signaling pathway, hsa04330:Notch signaling pathway, hsa04916:Melanogenesis, hsa05200:Pathways in cancer, hsa05210:Colorectal cancer, hsa05217:Basal cell carcinoma, |
| ENSG00000161202 | 1857 | hsa04310:Wnt signaling pathway, hsa04330:Notch signaling pathway, hsa04916:Melanogenesis, hsa05200:Pathways in cancer, hsa05210:Colorectal cancer, hsa05217:Basal cell carcinoma, |
| ENSG00000165879 | 10023 | hsa04310:Wnt signaling pathway, |
| ENSG00000181274 | 23401 | hsa04310:Wnt signaling pathway, |
| ENSG00000157240 | 8321 | hsa04310:Wnt signaling pathway, hsa04916:Melanogenesis, hsa05200:Pathways in cancer, hsa05210:Colorectal cancer, hsa05217:Basal cell carcinoma, |
| ENSG00000111432 | 11211 | hsa04310:Wnt signaling pathway, hsa04916:Melanogenesis, hsa05200:Pathways in cancer, hsa05210:Colorectal cancer, hsa05217:Basal cell carcinoma, |
| ENSG00000180340 | 2535 | hsa04310:Wnt signaling pathway, hsa04916:Melanogenesis, hsa05200:Pathways in cancer, hsa05210:Colorectal cancer, hsa05217:Basal cell carcinoma, |
| ENSG00000104290 | 7976 | hsa04310:Wnt signaling pathway, hsa04916:Melanogenesis, hsa05200:Pathways in cancer, hsa05210:Colorectal cancer, hsa05217:Basal cell carcinoma, |
| ENSG00000174804 | 8322 | hsa04310:Wnt signaling pathway, hsa04916:Melanogenesis, hsa05200:Pathways in cancer, hsa05210:Colorectal cancer, hsa05217:Basal cell carcinoma, |
| ENSG00000163251 | 7855 | hsa04310:Wnt signaling pathway, hsa04916:Melanogenesis, hsa05200:Pathways in cancer, hsa05210:Colorectal cancer, hsa05217:Basal cell carcinoma, |
| ENSG00000164930 | 8323 | hsa04310:Wnt signaling pathway, hsa04916:Melanogenesis, hsa05200:Pathways in cancer, hsa05210:Colorectal cancer, hsa05217:Basal cell carcinoma, |
| ENSG00000155760 | 8324 | hsa04310:Wnt signaling pathway, hsa04916:Melanogenesis, hsa05200:Pathways in cancer, hsa05210:Colorectal cancer, hsa05217:Basal cell carcinoma, |
| ENSG00000177283 | 8325 | hsa04310:Wnt signaling pathway, hsa04916:Melanogenesis, hsa05200:Pathways in cancer, hsa05210:Colorectal cancer, hsa05217:Basal cell carcinoma, |
| ENSG00000188763 | 8326 | hsa04310:Wnt signaling pathway, hsa04916:Melanogenesis, hsa05200:Pathways in cancer, hsa05210:Colorectal cancer, hsa05217:Basal cell carcinoma, |
| ENSG00000082701 | 2932 | hsa04012:ErbB signaling pathway, hsa04062:Chemokine signaling pathway, hsa04110:Cell cycle, hsa04310:Wnt signaling pathway, hsa04340:Hedgehog signaling pathway, hsa04360:Axon guidance, hsa04510:Focal adhesion, hsa04660:T cell receptor signaling pathway, hsa04662:B cell receptor signaling pathway, hsa04722:Neurotrophin signaling pathway, hsa04910:Insulin signaling pathway, hsa04916:Melanogenesis, hsa05010:Alzheimer's disease, hsa05200:Pathways in cancer, hsa05210:Colorectal cancer, hsa05213:Endometrial cancer, hsa05215:Prostate cancer, hsa05217:Basal cell carcinoma, |
| ENSG00000177606 | 3725 | hsa04010:MAPK signaling pathway, hsa04012:ErbB signaling pathway, hsa04310:Wnt signaling pathway, hsa04510:Focal adhesion, hsa04620:Toll-like receptor signaling pathway, hsa04660:T cell receptor signaling pathway, hsa04662:B cell receptor signaling pathway, hsa04722:Neurotrophin signaling pathway, hsa04912:GnRH signaling pathway, hsa05120:Epithelial cell signaling in Helicobacter pylori infection, hsa05200:Pathways in cancer, hsa05210:Colorectal cancer, hsa05211:Renal cell carcinoma, |
| ENSG00000162337 | 4041 | hsa04310:Wnt signaling pathway, |
| ENSG00000070018 | 4040 | hsa04310:Wnt signaling pathway, |
| ENSG00000204435, ENSG00000206300, ENSG00000206406, | 1460, 58496 | hsa04310:Wnt signaling pathway, hsa04520:Adherens junction, hsa04530:Tight junction, hsa04530:Tight junction, |
| ENSG00000138795 | 51176 | hsa04310:Wnt signaling pathway, hsa04520:Adherens junction, hsa04916:Melanogenesis, hsa05200:Pathways in cancer, hsa05210:Colorectal cancer, hsa05213:Endometrial cancer, hsa05215:Prostate cancer, hsa05216:Thyroid cancer, hsa05217:Basal cell carcinoma, hsa05221:Acute myeloid leukemia, hsa05412:Arrhythmogenic right ventricular cardiomyopathy (ARVC), |
| ENSG00000137673 | 4316 | hsa04310:Wnt signaling pathway, |
| ENSG00000109339 | 5602 | hsa04010:MAPK signaling pathway, hsa04012:ErbB signaling pathway, hsa04310:Wnt signaling pathway, hsa04510:Focal adhesion, hsa04620:Toll-like receptor signaling pathway, hsa04621:NOD-like receptor signaling pathway, hsa04622:RIG-I-like receptor signaling pathway, hsa04664:Fc epsilon RI signaling pathway, hsa04722:Neurotrophin signaling pathway, hsa04910:Insulin signaling pathway, hsa04912:GnRH signaling pathway, hsa04914:Progesterone-mediated oocyte maturation, hsa04920:Adipocytokine signaling pathway, hsa04930:Type II diabetes mellitus, hsa05120:Epithelial cell signaling in Helicobacter pylori infection, hsa05200:Pathways in cancer, hsa05210:Colorectal cancer, hsa05212:Pancreatic cancer, |
| ENSG00000107643 | 5599 | hsa04010:MAPK signaling pathway, hsa04012:ErbB signaling pathway, hsa04310:Wnt signaling pathway, hsa04510:Focal adhesion, hsa04620:Toll-like receptor signaling pathway, hsa04621:NOD-like receptor signaling pathway, hsa04622:RIG-I-like receptor signaling pathway, hsa04664:Fc epsilon RI signaling pathway, hsa04722:Neurotrophin signaling pathway, hsa04910:Insulin signaling pathway, hsa04912:GnRH signaling pathway, hsa04914:Progesterone-mediated oocyte maturation, hsa04920:Adipocytokine signaling pathway, hsa04930:Type II diabetes mellitus, hsa05120:Epithelial cell signaling in Helicobacter pylori infection, hsa05200:Pathways in cancer, hsa05210:Colorectal cancer, hsa05212:Pancreatic cancer, |
| ENSG00000050748 | 5601 | hsa04010:MAPK signaling pathway, hsa04012:ErbB signaling pathway, hsa04310:Wnt signaling pathway, hsa04510:Focal adhesion, hsa04620:Toll-like receptor signaling pathway, hsa04621:NOD-like receptor signaling pathway, hsa04622:RIG-I-like receptor signaling pathway, hsa04660:T cell receptor signaling pathway, hsa04664:Fc epsilon RI signaling pathway, hsa04722:Neurotrophin signaling pathway, hsa04910:Insulin signaling pathway, hsa04912:GnRH signaling pathway, hsa04914:Progesterone-mediated oocyte maturation, hsa04920:Adipocytokine signaling pathway, hsa04930:Type II diabetes mellitus, hsa05120:Epithelial cell signaling in Helicobacter pylori infection, hsa05200:Pathways in cancer, hsa05210:Colorectal cancer, hsa05212:Pancreatic cancer, |
| ENSG00000135341 | 6885 | hsa04010:MAPK signaling pathway, hsa04310:Wnt signaling pathway, hsa04520:Adherens junction, hsa04620:Toll-like receptor signaling pathway, hsa04621:NOD-like receptor signaling pathway, hsa04622:RIG-I-like receptor signaling pathway, hsa04660:T cell receptor signaling pathway, |
| ENSG00000140807 | 85407 | hsa04310:Wnt signaling pathway, |
| ENSG00000145506 | 85409 | hsa04310:Wnt signaling pathway, |
| ENSG00000087095 | 51701 | hsa04010:MAPK signaling pathway, hsa04310:Wnt signaling pathway, hsa04520:Adherens junction, |
| ENSG00000102908 | 10725 | hsa04310:Wnt signaling pathway, hsa04360:Axon guidance, hsa04370:VEGF signaling pathway, hsa04650:Natural killer cell mediated cytotoxicity, hsa04660:T cell receptor signaling pathway, hsa04662:B cell receptor signaling pathway, |
| ENSG00000131196 | 4772 | hsa04310:Wnt signaling pathway, hsa04360:Axon guidance, hsa04370:VEGF signaling pathway, hsa04650:Natural killer cell mediated cytotoxicity, hsa04660:T cell receptor signaling pathway, hsa04662:B cell receptor signaling pathway, |
| ENSG00000101096 | 4773 | hsa04010:MAPK signaling pathway, hsa04310:Wnt signaling pathway, hsa04360:Axon guidance, hsa04370:VEGF signaling pathway, hsa04650:Natural killer cell mediated cytotoxicity, hsa04660:T cell receptor signaling pathway, hsa04662:B cell receptor signaling pathway, |
| ENSG00000072736 | 4775 | hsa04310:Wnt signaling pathway, hsa04360:Axon guidance, hsa04370:VEGF signaling pathway, hsa04650:Natural killer cell mediated cytotoxicity, hsa04660:T cell receptor signaling pathway, hsa04662:B cell receptor signaling pathway, |
| ENSG00000100968 | 4776 | hsa04010:MAPK signaling pathway, hsa04310:Wnt signaling pathway, hsa04360:Axon guidance, hsa04370:VEGF signaling pathway, hsa04650:Natural killer cell mediated cytotoxicity, hsa04660:T cell receptor signaling pathway, hsa04662:B cell receptor signaling pathway, |
| ENSG00000112033 | 5467 | hsa03320:PPAR signaling pathway, hsa04310:Wnt signaling pathway, hsa05200:Pathways in cancer, hsa05221:Acute myeloid leukemia, |
| ENSG00000182621 | 23236 | hsa00562:Inositol phosphate metabolism, hsa04020:Calcium signaling pathway, hsa04062:Chemokine signaling pathway, hsa04070:Phosphatidylinositol signaling system, hsa04270:Vascular smooth muscle contraction, hsa04310:Wnt signaling pathway, hsa04540:Gap junction, hsa04720:Long-term potentiation, hsa04730:Long-term depression, hsa04912:GnRH signaling pathway, hsa04916:Melanogenesis, hsa05010:Alzheimer's disease, hsa05016:Huntington's disease, |
| ENSG00000137841 | 5330 | hsa00562:Inositol phosphate metabolism, hsa04020:Calcium signaling pathway, hsa04062:Chemokine signaling pathway, hsa04070:Phosphatidylinositol signaling system, hsa04270:Vascular smooth muscle contraction, hsa04310:Wnt signaling pathway, hsa04540:Gap junction, hsa04720:Long-term potentiation, hsa04730:Long-term depression, hsa04742:Taste transduction, hsa04912:GnRH signaling pathway, hsa04916:Melanogenesis, hsa05010:Alzheimer's disease, hsa05016:Huntington's disease, |
| ENSG00000149782 | 5331 | hsa00562:Inositol phosphate metabolism, hsa04020:Calcium signaling pathway, hsa04062:Chemokine signaling pathway, hsa04070:Phosphatidylinositol signaling system, hsa04270:Vascular smooth muscle contraction, hsa04310:Wnt signaling pathway, hsa04540:Gap junction, hsa04720:Long-term potentiation, hsa04730:Long-term depression, hsa04912:GnRH signaling pathway, hsa04916:Melanogenesis, hsa05010:Alzheimer's disease, hsa05016:Huntington's disease, |
| ENSG00000101333 | 5332 | hsa00562:Inositol phosphate metabolism, hsa04020:Calcium signaling pathway, hsa04062:Chemokine signaling pathway, hsa04070:Phosphatidylinositol signaling system, hsa04270:Vascular smooth muscle contraction, hsa04310:Wnt signaling pathway, hsa04540:Gap junction, hsa04720:Long-term potentiation, hsa04730:Long-term depression, hsa04912:GnRH signaling pathway, hsa04916:Melanogenesis, hsa05010:Alzheimer's disease, hsa05016:Huntington's disease, |
| ENSG00000102312 | 64840 | hsa04310:Wnt signaling pathway, |
| ENSG00000080815 | 5663 | hsa04310:Wnt signaling pathway, hsa04330:Notch signaling pathway, hsa04722:Neurotrophin signaling pathway, hsa05010:Alzheimer's disease, |
| ENSG00000139174 | 144165 | hsa04310:Wnt signaling pathway, |
| ENSG00000163637 | 166336 | hsa04310:Wnt signaling pathway, |
| ENSG00000154229 | 5578 | hsa04010:MAPK signaling pathway, hsa04012:ErbB signaling pathway, hsa04020:Calcium signaling pathway, hsa04070:Phosphatidylinositol signaling system, hsa04270:Vascular smooth muscle contraction, hsa04310:Wnt signaling pathway, hsa04370:VEGF signaling pathway, hsa04510:Focal adhesion, hsa04530:Tight junction, hsa04540:Gap junction, hsa04650:Natural killer cell mediated cytotoxicity, hsa04664:Fc epsilon RI signaling pathway, hsa04666:Fc gamma R-mediated phagocytosis, hsa04670:Leukocyte transendothelial migration, hsa04720:Long-termpotentiation, hsa04730:Long-term depression, hsa04912:GnRH signaling pathway, hsa04916:Melanogenesis, hsa04960:Aldosterone-regulated sodium reabsorption, hsa05110:Vibrio cholerae infection, hsa05130:Pathogenic Escherichia coli infection, hsa05200:Pathways in cancer, hsa05214:Glioma, hsa05223:Non-small cell lung cancer, |
| ENSG00000166501 | 5579 | hsa04010:MAPK signaling pathway, hsa04012:ErbB signaling pathway, hsa04020:Calcium signaling pathway, hsa04062:Chemokine signaling pathway, hsa04070:Phosphatidylinositol signaling system, hsa04270:Vascular smooth muscle contraction, hsa04310:Wnt signaling pathway, hsa04370:VEGF signaling pathway, hsa04510:Focal adhesion, hsa04530:Tight junction, hsa04540:Gap junction, hsa04650:Natural killer cell mediated cytotoxicity, hsa04662:B cell receptor signaling pathway, hsa04664:Fc epsilon RI signaling pathway, hsa04666:Fc gamma R-mediated phagocytosis, hsa04670:Leukocyte transendothelial migration, hsa04720:Long-term potentiation, hsa04730:Long-term depression, hsa04912:GnRH signaling pathway, hsa04916:Melanogenesis, hsa04960:Aldosterone-regulated sodium reabsorption, hsa05110:Vibrio cholerae infection, hsa05200:Pathways in cancer, hsa05214:Glioma, hsa05223:Non-small cell lung cancer, |
| ENSG00000126583 | 5582 | hsa04010:MAPK signaling pathway, hsa04012:ErbB signaling pathway, hsa04020:Calcium signaling pathway, hsa04070:Phosphatidylinositol signaling system, hsa04270:Vascular smooth muscle contraction, hsa04310:Wnt signaling pathway, hsa04370:VEGF signaling pathway, hsa04510:Focal adhesion, hsa04530:Tight junction, hsa04540:Gap junction, hsa04650:Natural killer cell mediated cytotoxicity, hsa04666:Fc gamma R-mediated phagocytosis, hsa04670:Leukocyte transendothelial migration, hsa04720:Long-term potentiation, hsa04730:Long-term depression, hsa04916:Melanogenesis, hsa04960:Aldosterone-regulated sodium reabsorption, hsa05110:Vibrio cholerae infection, hsa05200:Pathways in cancer, hsa05214:Glioma, hsa05223:Non-small cell lung cancer, |
| ENSG00000183943 | 5613 | hsa04010:MAPK signaling pathway, hsa04020:Calcium signaling pathway, hsa04062:Chemokine signaling pathway, hsa04114:Oocyte meiosis, hsa04210:Apoptosis, hsa04270:Vascular smooth muscle contraction, hsa04310:Wnt signaling pathway, hsa04340:Hedgehog signaling pathway, hsa04540:Gap junction, hsa04720:Long-term potentiation, hsa04740:Olfactory transduction, hsa04742:Taste transduction, hsa04910:Insulin signaling pathway, hsa04912:GnRH signaling pathway, hsa04914:Progesterone-mediated oocyte maturation, |
| | | hsa04916:Melanogenesis, hsa05020:Prion diseases, hsa05110:Vibrio cholerae infection, hsa05414:Dilated cardiomyopathy, |
| | | |
| ENSG00000072062 | 5566 | hsa04010:MAPK signaling pathway, hsa04020:Calcium signaling pathway, hsa04062:Chemokine signaling pathway, hsa04114:0ocyte meiosis, hsa04210:Apoptosis, hsa04270:Vascular smooth muscle contraction, hsa04310:Wnt signaling pathway, hsa04340:Hedgehog signaling pathway, hsa04540:Gap junction, hsa04720:Long-term potentiation, hsa04740:Olfactory transduction, hsa04742:Taste transduction, hsa04910:Insulin signaling pathway, hsa04912:GnRH signaling pathway, hsa04914:Progesterone-mediated oocyte maturation, hsa04916:Melanogenesis, hsa05020:Prion diseases, hsa05110:Vibrio cholerae infection, hsa05414:Dilated cardiomyopathy, |
| ENSG00000142875 | 5567 | hsa04010:MAPK signaling pathway, hsa04020:Calcium signaling pathway, hsa04062:Chemokine signaling pathway, hsa04114:Oocyte meiosis, hsa04210:Apoptosis, hsa04270:Vascular smooth muscle contraction, hsa04310:Wnt signaling pathway, hsa04340:Hedgehog signaling pathway, hsa04540:Gap junction, hsa04720:Long-term potentiation, hsa04740:Olfactory transduction, hsa04742:Taste transduction, hsa04910:Insulin signaling pathway, hsa04912:GnRH signaling pathway, hsa04914:Progesterone-mediated oocyte maturation, hsa04916:Melanogenesis, hsa05020:Prion diseases, hsa05110:Vibrio cholerae infection, hsa05414:Dilated cardiomyopathy, |
| ENSG00000165059 | 5568 | hsa04010:MAPK signaling pathway, hsa04020:Calcium signaling pathway, hsa04062:Chemokine signaling pathway, hsa04114:Oocyte meiosis, hsa04210:Apoptosis, hsa04270:Vascular smooth muscle contraction, hsa04310:Wnt signaling pathway, hsa04340:Hedgehog signaling pathway, hsa04540:Gap junction, hsa04720:Long-term potentiation, hsa04740:Olfactory transduction, hsa04742:Taste transduction, hsa04910:Insulin transduction, hsa04742:Taste transduction, hsa04910:Insulin signaling pathway, hsa04912:GnRH signaling pathway, hsa04914:Progesterone-mediated oocyte maturation, hsa04916:Melanogenesis, hsa05020:Prion diseases, hsa05110:Vibrio cholerae infection, hsa05414:Dilated cardiomyopathy, |
| ENSG00000113575 | 5515 | hsa04114:Oocyte meiosis, hsa04310:Wnt signaling pathway, hsa04350:TGF-beta signaling pathway, hsa04530:Tight junction, hsa04730:Long-term depression, |
| ENSG00000104695 | 5516 | hsa04114:Oocyte meiosis, hsa04310:Wnt signaling pathway, hsa04350:TGF-beta signaling pathway, hsa04530:Tight junction, hsa04730:Long-term depression, |
| ENSG00000105568 | 5518 | hsa04114:Oocyte meiosis, hsa04310:Wnt signaling pathway, hsa04350:TGF-beta signaling pathway, hsa04530:Tight junction, hsa04730:Long-term depression, |
| ENSG00000137713 | 5519 | hsa04114:Oocyte meiosis, hsa04310:Wnt signaling pathway, hsa04350:TGF-beta signaling pathway, hsa04530:Tight junction, hsa04730:Long-term depression, |
| ENSG00000066027 | 5525 | hsa04114:Oocyte meiosis, hsa04310:Wnt signaling pathway, |
| ENSG00000068971 | 5526 | hsa04114:Oocyte meiosis, hsa04310:Wnt signaling pathway, |
| ENSG00000112640 | 5528 | hsa04114:Oocyte meiosis, hsa04310:Wnt signaling pathway, |
| ENSG00000154001 | 5529 | hsa04114:Oocyte meiosis, hsa04310:Wnt signaling pathway, |
| ENSG00000078304 | 5527 | hsa04114:Oocyte meiosis, hsa04310:Wnt signaling pathway, |
| ENSG00000138814 | 5530 | hsa04010:MAPK signaling pathway, hsa04020:Calcium signaling pathway, hsa04114:Oocyte meiosis, hsa04210:Apoptosis, hsa04310:Wnt signaling pathway, hsa04360:Axon guidance, hsa04370:VEGF signaling pathway, hsa04650:Natural killer cell mediated cytotoxicity, hsa04660:T cell receptor signaling pathway, hsa04662:B cell receptor signaling pathway, hsa04720:Long-term potentiation, hsa05010:Alzheimer's disease, hsa05014:Amyotrophic lateral sclerosis (ALS), |
| ENSG00000107758 | 5532 | hsa04010:MAPK signaling pathway, hsa04020:Calcium signaling pathway, hsa04114:0ocyte meiosis, hsa04210:Apoptosis, hsa04310:Wnt signaling pathway, hsa04360:Axon guidance, hsa04370:VEGF signaling pathway, hsa04650:Natural killer cell mediated cytotoxicity, hsa04660:T cell receptor signaling pathway, hsa04662:B cell receptor signaling pathway, hsa04720:Long-term potentiation, hsa05010:Alzheimer's disease, hsa05014:Amyotrophic lateral sclerosis (ALS), |
| ENSG00000120910 | 5533 | hsa04010:MAPK signaling pathway, hsa04020:Calcium signaling pathway, hsa04114:Oocyte meiosis, hsa04210:Apoptosis, hsa04310:Wnt signaling pathway, hsa04360:Axon guidance, hsa04370:VEGF signaling pathway, hsa04650:Natural killer cell mediated cytotoxicity, hsa04660:T cell receptor signaling pathway, hsa04662:B cell receptor signaling pathway, hsa04720:Long-term potentiation, hsa05010:Alzheimer's disease, hsa05014:Amyotrophic lateral sclerosis (ALS), |
| ENSG00000115953 | 5534 | hsa04010:MAPK signaling pathway, hsa04020:Calcium signaling pathway, hsa04114:Oocyte meiosis, hsa04210:Apoptosis, hsa04310:Wnt signaling pathway, hsa04360:Axon guidance, hsa04370:VEGF signaling pathway, hsa04650:Natural killer cell mediated cytotoxicity, hsa04660:T cell receptor signaling pathway, hsa04662:B cell receptor signaling pathway, hsa04720:Long-term potentiation, hsa05010:Alzheimer's disease, hsa05014:Amyotrophic lateral sclerosis (ALS), |
| ENSG00000188386 | 5535 | hsa04010:MAPK signaling pathway, hsa04020:Calcium signaling pathway, hsa04114:Oocyte meiosis, hsa04210:Apoptosis, hsa04310:Wnt signaling pathway, hsa04360:Axon guidance, hsa04370:VEGF signaling pathway, hsa04650:Natural killer cell mediated cytotoxicity, hsa04660:T cell receptor signaling pathway, hsa04662:B cell receptor signaling pathway, hsa04720:Long-term potentiation, hsa05010:Alzheimer's disease, hsa05014:Amyotrophic lateral sclerosis (ALS), |
| ENSG00000067560 | 387 | hsa04062:Chemokine signaling pathway, hsa04270:Vascular smooth muscle contraction, hsa04310:Wnt signaling pathway, hsa04350:TGF-beta signaling pathway, hsa04360:Axon guidance, hsa04510:Focal adhesion, hsa04520:Adherens junction, hsa04530:Tight junction, hsa04660:T cell receptor signaling |
| | | pathway, hsa04670:Leukocyte transendothelial migration, hsa04722:Neurotrophin signaling pathway, hsa04810:Regulation of actin cytoskeleton, hsa05130:Pathogenic Escherichia coli infection, hsa05200:Pathways in cancer, |
| ENSG00000136238 | 5879 | hsa04010:MAPK signaling pathway, hsa04062:Chemokine signaling pathway, hsa04310:Wnt signaling pathway, hsa04360:Axon guidance, hsa04370:VEGF signaling pathway, hsa04510:Focal adhesion, hsa04520:Adherens junction, hsa04620:Toll-like receptor signaling pathway, hsa04650:Natural killer cell mediated cytotoxicity, hsa04662:B cell receptor signaling pathway, hsa04664:Fc epsilon RI signaling pathway, hsa04666:Fc gamma R-mediated phagocytosis, hsa04670:Leukocyte transendothelial migration, hsa04722:Neurotrophin signaling pathway, hsa04810:Regulation of actin cytoskeleton, hsa05014:Amyotrophic lateral sclerosis (ALS), hsa05120:Epithelial cell signaling in Helicobacter pylori infection, hsa05200:Pathways in cancer, hsa05210:Colorectal cancer, hsa05211:Renal cell carcinoma, hsa05212:Pancreatic cancer, hsa05416:Viral myocarditis, |
| ENSG00000128340 | 5880 | hsa04010:MAPK signaling pathway, hsa04062:Chemokine signaling pathway, hsa04310:Wnt signaling pathway, hsa04360:Axon guidance, hsa04370:VEGF signaling pathway, hsa04510:Focal adhesion, hsa04520:Adherens junction, hsa04650:Natural killer cell mediated cytotoxicity, hsa04662:B cell receptor signaling pathway, hsa04664:Fc epsilon RI signaling pathway, hsa04666:Fc gamma R-mediated phagocytosis, hsa04670:Leukocyte transendothelial migration, hsa04810:Regulation of actin cytoskeleton, hsa05200:Pathways in cancer, hsa05210:Colorectal cancer, hsa05212:Pancreatic cancer, hsa05416:Viral myocarditis, |
| ENSG00000169750 | 5881 | hsa04010:MAPK signaling pathway, hsa04310:Wnt signaling pathway, hsa04360:Axon guidance, hsa04370:VEGF signaling pathway, hsa04510:Focal adhesion, hsa04520:Adherens junction, hsa04650:Natural killer cell mediated cytotoxicity, hsa04662:B cell receptor signaling pathway, hsa04664:Fc epsilon RI signaling pathway, hsa04810:Regulation of actin cytoskeleton, hsa05200:Pathways in cancer, hsa05210:Colorectal cancer, hsa05212:Pancreatic cancer, hsa05416:Viral myocarditis, |
| ENSG00000100387 | 9978 | hsa03420:Nucleotide excision repair, hsa04110:Cell cycle, hsa04114:Oocyte meiosis, hsa04120:Ubiquitin mediated proteolysis, hsa04310:Wnt signaling pathway, hsa04350:TGF-beta signaling pathway, hsa05200:Pathways in cancer, hsa05211:Renal cell carcinoma, |
| ENSG00000104332 | 6422 | hsa04310:Wnt signaling pathway, |
| ENSG00000145423 | 6423 | hsa04310:Wnt signaling pathway, |
| ENSG00000106483 | 6424 | hsa04310:Wnt signaling pathway, |
| ENSG00000120057 | 6425 | hsa04310:Wnt signaling pathway, |
| ENSG00000196470 | 6477 | hsa04115:p53 signaling pathway, hsa04120:Ubiquitin mediated proteolysis, hsa04310:Wnt signaling pathway, |
| ENSG00000067900 | 6093, 727758 | hsa04062:Chemokine signaling pathway, hsa04270:Vascular smooth muscle contraction, hsa04310:Wnt signaling pathway, hsa04350:TGF-beta signaling pathway, hsa04360:Axon guidance, hsa04510:Focal adhesion, hsa04670:Leukocyte transendothelial migration, hsa04810:Regulation of actin cytoskeleton, hsa05130:Pathogenic Escherichia coli infection, |
| ENSG00000116161 | 27101, 644877 | hsa04310:Wnt signaling pathway, |
| ENSG00000081059 | 6932 | hsa04310:Wnt signaling pathway, hsa04520:Adherens junction, hsa04916:Melanogenesis, hsa05200:Pathways in cancer, hsa05210:Colorectal cancer, hsa05213:Endometrial cancer, hsa05215:Prostate cancer, hsa05216:Thyroid cancer, hsa05217:Basal cell carcinoma, hsa05221:Acute myeloid leukemia, hsa05412:Arrhythmogenic right ventricular cardiomyopathy (ARVC), |
| ENSG00000148737 | 6934 | hsa04310:Wnt signaling pathway, hsa04520:Adherens junction, hsa04916:Melanogenesis, hsa05200:Pathways in cancer, hsa05210:Colorectal cancer, hsa05213:Endometrial cancer, hsa05215:Prostate cancer, hsa05216:Thyroid cancer, hsa05217:Basal cell carcinoma, hsa05221:Acute myeloid leukemia, hsa05412:Arrhythmogenic right ventricular cardiomyopathy (ARVC), |
| ENSG00000177565 | 79718 | hsa04310:Wnt signaling pathway, |
| ENSG00000101849 | 6907 | hsa04310:Wnt signaling pathway, |
| ENSG00000092377 | 90665 | hsa04310:Wnt signaling pathway, |
| ENSG00000141510 | 7157 | hsa04010:MAPK signaling pathway, hsa04110:Cell cycle, hsa04115:p53 signaling pathway, hsa04210:Apoptosis, hsa04310:Wnt signaling pathway, hsa04722:Neurotrophin signaling pathway, hsa05014:Amyotrophic lateral sclerosis (ALS), hsa05016:Huntington's disease, hsa05200:Pathways in cancer, hsa05210:Colorectal cancer, hsa05212:Pancreatic cancer, hsa05213:Endometrial cancer, hsa05214:Glioma, hsa05215:Prostate cancer, hsa05216:Thyroid cancer, hsa05217:Basal cell carcinoma, hsa05218:Melanoma, hsa05219:Bladder cancer, hsa05220:Chronic myeloid leukemia, hsa05222:Small cell lung cancer, hsa05223:Non-small cell lung cancer, |
| ENSG00000136997 | 4609 | hsa04010:MAPK signaling pathway, hsa04012:ErbB signaling pathway, hsa04110:Cell cycle, hsa04310:Wnt signaling pathway, hsa04350:TGF-beta signaling pathway, hsa04630:Jak-STAT signaling pathway, hsa05200:Pathways in cancer, hsa05210:Colorectal cancer, hsa05213:Endometrial cancer, hsa05216:Thyroid cancer, hsa05219:Bladder cancer, hsa05220:Chronic myeloid leukemia, hsa05221:Acute myeloid leukemia, hsa05222:Small cell lung cancer, |
| ENSG00000173218 | 81839 | hsa04310:Wnt signaling pathway, |
| ENSG00000162738 | 57216 | hsa04310:Wnt signaling pathway, |
| ENSG00000105989 | 7472 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000125084 | 7471 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000135925 | 80326 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000169884 | 7480 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000085741 | 7481 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000002745 | 51384 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000134245 | 7482 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000108379 | 7473 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000154342 | 89780 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000162552 | 54361 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000114251 | 7474 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000111186 | 81029 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000115596 | 7475 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000154764 | 7476 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000188064 | 7477 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000061492 | 7478 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000075290 | 7479 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000143816 | 7483 | hsa04310:Wnt signaling pathway, hsa04340:Hedgehog signaling pathway, hsa04916:Melanogenesis, hsa05200:Pathways in cancer, hsa05217:Basal cell carcinoma, |
| ENSG00000158955 | 7484 | hsa04310:Wnt signaling pathway, hsa04340:Hedgehog signaling pathway, hsa04916:Melanogenesis, hsa05200:Pathways in cancer, hsa05217:Basal cell carcinoma, |

## Claims

1. A method for identifying a compound modulating Wnt/β-catenin signaling comprising the steps of:
a) determining the amounts of at least the biomarkers NKD1 and C10orf54 in a sample comprising cells, wherein said cells have been brought into contact with a compound suspected to be a modulator of Wnt/β-catenin signaling; and
b) comparing the amount of the biomarker NKD1 determined in step a) to a reference for NKD1 and comparing the amount of the biomarker C10orf54 determined in step a) to a reference for C10orf54, whereby a compound modulating Wnt/β-catenin signaling is identified.

2. The method of claim 1, wherein said reference is obtained from a sample comprising cells corresponding to the cells of step a) and wherein said cells have not been brought into contact with a compound suspected to modulate Wnt/β-catenin signaling.

3. The method of claim 2, wherein amounts of said biomarkers determined in step a) differing from the corresponding references are indicative for a compound modulating Wnt/β-catenin signaling.

4. The method of claim 1, wherein said reference is obtained from a sample of cells corresponding to the cells of step a) and wherein said cells have been brought into contact with a compound known to modulate Wnt/β-catenin signaling.

5. The method of claim 4, wherein amounts of said biomarkers determined in step a) which are essentially identical to the corresponding reference are indicative for a compound modulating Wnt/β-catenin signaling.

6. The method of any one of claims 1 to 5, wherein said compound modulating Wnt/β-catenin signaling is selected from the group consisting of: antisense RNA, siRNA, micro RNA, ribozymes, oligonucleotides, peptides, PNAs, proteins, antibodies, aptamers, and organic small molecule compounds.

7. The method of any one of claims 1 to 6, wherein said cells are human or animal cells.

8. The method of any one of claims 1 to 7, wherein said compound modulating Wnt/β-catenin signaling is an inhibitor of said Wnt/β-catenin signaling.

9. A method for identifying a drug comprising the steps of the method for identifying a compound modulating Wnt/β-catenin signaling of any one of claims 1 to 8 and, further, the step of identifying a compound modulating Wnt/β-catenin signaling as a drug.

10. The method of claim 9, wherein said drug is a drug against cancer.

11. The method of claim 10, wherein said cancer is selected from the group consisting of: colorectal cancer, breast cancer, esophageal cancer, melanoma, hepatocellular carcinoma, desmoid tumor, pancreatic cancer, gastric cancer, ovarian cancer, and prostate cancer.

12. Use of at least the biomarkers NKD1 and C10orf54 in a sample comprising cells for determining whether said cells have been brought into contact with a compound modulating Wnt/β-catenin signaling.

13. The use of claim 12, wherein said compound modulating Wnt/β-catenin signaling is an inhibitor of Wnt/β-catenin signaling.

14. The use of claim 12 or 13, wherein said compound modulating Wnt/β-catenin signaling is a drug or suspected to be a drug against cancer.

15. The use of claim 14, wherein said cancer is selected from the group consisting of: colorectal cancer, breast cancer, esophageal cancer, melanoma, hepatocellular carcinoma, desmoid tumor, pancreatic cancer, gastric cancer, ovarian cancer, and prostate cancer.
